# EUROPEAN PATENT APPLICATION

(11) **EP 1 908 752 A1**
(43) Date of publication of application: **09.04.2008**
(21) Application number: 06756992.1
(22) Date of filing: 05.06.2006
(51) Int. Cl.: C07D 215/22, A61K 31/4704, A61K 31/4709, A61K 31/473, A61K 31/4741, A61K 31/5377, A61P 25/00, A61P 25/02, A61P 25/04, C07D 215/227, C07D 221/16, C07D 401/06, C07D 401/12, C07D 401/14, C07D 405/12, C07D 413/14, C07D 417/14, C07D 491/056

(54) **NOVEL 2-QUINOLONE DERIVATIVE**

(30) Priority: 07.06.2005 JP 2005167479
(71) Applicant: Dainippon Sumitomo Pharma Co., Ltd., Osaka 541-8524 (JP)
(72) Inventor: IKEDA, Kazuhito, Dainippon Sumitomo Pharma Co. Ltd, Suita-shi, Osaka 5640053 (JP); KUMAGAI, Kazuo, Dainippon Sumitomo Pharma Co. Ltd., Takarazuka-shi, Hyogo 66510055 (JP); TSUBOI, K., Dainippon Sumitomo Pharma Co. Ltd., Suita-shi, Osaka 5640053 (JP); FUKUDA, Nobuhisa, Tokyo 1120002 (JP)
(74) Representative: Duckworth, Timothy John
(86) International application number: PCT/JP2006/311246
(87) International publication number: WO 2006/132192

(57) **Abstract**

A compound represented by the formula (1) or a pharmaceutically acceptable salt thereof which has a therapeutic or prophylactic effect on an SNS-related disease such as neuropathic pain. (1) wherein R¹ and R² independently represent an alkyl group having 1 to 4 carbon atoms or the like, or R¹ and R² may together form a 5- to 7-membered ring; n represents a numerical number of 1 to 3; A represents a substituted or unsubstituted aryl group or the like or a formula: -N(R⁵)R⁶ (wherein R⁵ and R⁶ independently represent a substituted or unsubstituted alkyl group or the like); and R³ and R⁴ independently represent a sustituted or unsubstituted alkyl group or the like, or R³ and R⁴ may together form a substituted or unsubstituted, saturated or unsubstituted nitrogenated heterocyclic ring, provided that both of R¹ and R² are not a hydrogen atom when R³ and R⁴ together form a piperidine ring.

## Description

### TECHNICAL FIELD

The present invention relates to a pharmaceutical composition for treatment or prophylaxis of all pathoses attributable to SNS (sensory neuron specific sodium channel) which contains a compound having a 2-quinolone skeleton or a pharmaceutically acceptable salt thereof as an active ingredient. Specifically, it relates to a pharmaceutical composition for treatment or prophylaxis of a disease such as neuropathic pain, nociceptive pain, urinary disturbance or multiple sclerosis.

### BACKGROUND ART

Hodgkin and Huxley proved in 1953 that the true character of neural action is the Na channel. Thereafter, Na channel inhibitors have been continuously developed as antiarrhythmic agents or local anesthetics. In 1961, lidocaine, one of the Na channel inhibitors was found to have analgesic effect and was begun to be clinically used as an analgesic. However, since the Na channel is present also in nonnervous tissues such as muscles and heart, the side effects of lidocaine caused by its general administration remain an unsolved problem.
On the other hand, with the advance of molecular biology, subtypes of the Na channel were revealed one after another, and it is known at present that there are 10 kinds of α subunits which form the pores of the Na channel. The sensory neuron specific sodium channel, i.e., SNS is one of such Na channel α subunits, is a tetrodotoxin (TTX)-resistant Na channel localized in the small-diameter cells (C fiber) of dorsal root ganglion concerned in neural perception, and is referred to also as SCN10A, PN3 or NaV1.8 (non-patent documents 1 and 2). It has been reported that an SNS knockout mouse is insensible to a mechanical stimulus and that hyperesthesia and paresthesia are alleviated in a neuropathic pain model or an inflammatory pain model by administration of an antisence against SNS.
Therefore, an SNS inhibitor was expected to be usable as an agent having analgesic effect on diseases such as neuropathic pain and nociceptive pain which are accompanied by a pain, numbness, urtication, a dull pain and the like, for which C fiber is responsible. Moreover, since SNS does not appear in nonnervous tissues and central nervous system, an agent capable of inhibiting SNS selectively was expected to be usable as an agent having no side effect due to the nonnervous tissues or central nervous system.

In the case of urinary disturbance, the following is being proved: urinary frequency, the main symptom of urinary disturbance is caused by the excessive action of C fiber, that is, the dysfunction of centripetal sensory nerve tract from lower urinary tract are responsible for excessively active bladder and cystalgia, so that the depression of C fiber sensory nerve from bladder is effective in treating urinary disturbance (non-patent document 3).
Therefore, an agent capable of inhibiting SNS, the true character of the neural action of C fiber is expected to be usable as a therapeutic or prophylactic agent for urinary disturbance which has a novel site of action.
On the other hand, it has recently been reported that SNS found only in C fiber appears aberrantly in the cerebellum Purkinje cells of a patient with multiple sclerosis and participates in the occurrence of the abnormal discharge pattern of the cerebellum (non-patent document 4). Therefore, an SNS inhibitor is expected to be usable as a novel therapeutic or prophylactic agent for the induction of symptoms such as ataxia by the abnormal discharge of cerebellar nerve caused by the appearance of SNS.

The present state of the treatment of the above-mentioned diseases in clinical sites is described below.

### (1) Neuropathic pain

Neuropathic pain refers to a spontaneous or chronic pain caused by an injury or stimulus to nerve without an external wound or without the participation of tissue inflammation that has been completely healed. The neuropathic pain includes, for example, lumbar postoperative neuralgia, diabetic neurosis, postherpetic neuralgia, reflex sympathetic pain, phantom limb pain, the pain of spinal cord damage, the pain of full-blown cancer, and prolonged postoperative pain. Against the neuropathic pain, NSAIDS (non-steroidal anti-inflammatory drugs) such as aspirin are completely ineffective, and opioids such as morphine involve problems such as drug resistance and the induction of mental symptoms.
At present, a drug put in the market as a drug effective in relieving the neuropathic pain is only mexiletine efficacious for diabetic neurosis. It has been reported that although mexiletine has analgesic effect, it is liable to have side effect because of its non-selectivity for the Na channel and hence cannot be taken in a large dose. Besides mexiletine, several agents are clinically used as auxiliary drugs in analgesic. These agents include, for example, antidepressants (sulpiride, trazodone, fluvoxatine and milnacipran), adrenergic drugs (clonidine and dexmedetomidine), NMDA receptor antagonists (ketamine hydrochloride and dextromethorphan), antianxiety agents (diazepam, lorazepam, etizolam and hydroxyzine hydrochloride), anticonvulsants (carbamazepine, phenytoin, sodium valproate and zonisamide), and calcium antagonists (nifedipine, verapamil hydrochloride and lomerizine hydrochloride). All of them are used as auxiliary drugs in analgesic. For the above reason, there is no decisive therapeutic agent having no side effect due to nonnervous tissues and central nervous system and having a specific analgesic effect on a pain.

### (2) Nociceptive pain

Nociceptive pain refers to a pain caused by the activation of a nociceptor (A δ, C fiber) by a mechanical, thermal or chemical noxious stimulus given, for example, by damage to a tissue. The nociceptor is sensitized by an endogenous chemical stimulus (a trigger substance) such as serotonin, substance P, bradykinin, prostaglandin or histamine. As the nociceptive pain, there are exemplified lumbago, bellyache, and pains due to rheumatoid arthritis and arthritis deformans. There are clinically used NSAIDS (acetylsalicylic acid, acetaminophenone, diclofenac sodium, indomethacin, mofezolac, flurbiprofen, loxoprofen sodium and ampiroxicam), steroid drugs (prednisolone, methylprednisolone, dexamethasone and betamethasone), PGE1 (prostaglandin E1) (alprostadil, lipo-alprostadil and limaprost alprostadil) and PGI2 (beraprost sodium).

### (3) Urinary disturbance

Urinary disturbance is a disease the main symptoms of which are urinary frequency, urorrhea, a feeling of residual urine and urodynia. At present, a muscarinic receptor inhibitor capable of depressing bladder parasympathetic nervous tract is mainly used in pharmacotherapy for excessively active bladder but the limits of its effect have been clarified. It has been reported that vanilloid receptor stimulators such as capsaicin and resiniferatoxin act specifically on C fiber to suppress its function, but no agent has been found which acts on SNS localized in C fiber.

### (4) Multiple sclerosis

Multiple sclerosis is a kind of demyelinating disease. In multiple sclerosis, focuses of demyelination are scattered in the white matter of central nervous system and are various focuses including new ones and old ones. The focuses tend to appear in the white matters of the periphery of lateral ventricle, optic nerve, brain stem, spinal cord and the like. Histologically, the marrow sheath is destroyed but the axon and nerve cells are not invaded. As clinical symptoms of multiple sclerosis, symptoms such as optic neuritis, oculogyration troubles (e.g. diplopia and nystagmus), spastic paralysis, painful tonic spasm, Lhermitte's syndrome, ataxia, lalopathy, vesicorectal disorder and the like appear in various combinations. Although the cause of multiple sclerosis is unknown, an autoimmune disease theory, an infection theory and the like have been proposed. At present, there is neither a prophylactic agent nor a therapeutic agent which is effective against multiple sclerosis.

As agents having inhibitory effect on SNS, pyrazole derivatives (e.g. pyrazole amide compounds and pyrazole sulfonamide compounds), piperidine derivatives and pyrazolopyrimidine derivatives have been disclosed (patent documents 1, 2 and 3).
Patent document 1: International Patent Laid-Open No. WO03/037274 pamphlet
Patent document 2: International Patent Laid-Open No. WO03/037890 pamphlet
Patent document 3: International Patent Laid-Open No. WO03/037900 pamphlet
Non-patent document 1: Nature 379: 257, 1996
Non-patent document 2: Pain 78: 107, 1998
Non-patent document 3: Urology 57: 116, 2001
Non-patent document 4: Brain Research 959: 235, 2003

### DISCLOSURE OF THE INVENTION

### Problem to be Solved by the Invention

A problem for the present invention is to provide a pharmaceutical composition for treatment or prophylaxis of all pathoses attributable to SNS, specifically, a disease such as neuropathic pain, nociceptive pain, urinary disturbance or multiple sclerosis.

### Means for Solving the Problem

The present inventors found that a compound having a 2-quinolone skeleton or a pharmaceutically acceptable salt thereof inhibits TTX-resistant Na channel in human-SNS-gene expression cells, namely, the compound or salt thereof has inhibitory activity against SNS. The human-SNS-gene expression cells were obtained by introducing the human SNS gene into Chinese hamster ovary cells (CHO-K1) and expressing it stably. Since the CHO-K1 cells have no TTX-resistant Na channel component in themselves, the TTX-resistant Na channel component in the human-SNS-gene expression cells is SNS. Therefore, it is conjectured that such a compound is an SNS inhibitor.
That is, the present invention relates to the following:
[1] A compound represented by the formula (1):

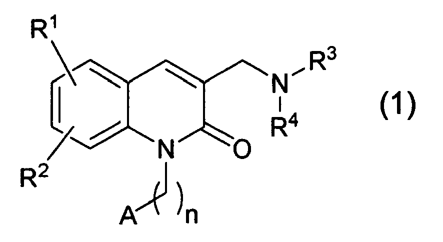

wherein R¹ and R² are independently a hydrogen atom, a halogen atom, a cyano group, a nitro group, a carboxyl group, an alkyl group of 1 to 4 carbon atoms, a haloalkyl group of 1 to 4 carbon atoms, an alkoxy group of 1 to 4 carbon atoms, a haloalkoxy group of 1 to 4 carbon atoms, an alkylthio group of 1 to 4 carbon atoms, an alkoxycarbonyl group of 2 to 4 carbon atoms, an alkylcarbonyl group of 2 to 4 carbon atoms, or a group represented by the formula: -L-Ar wherein L is a single bond, -O-, -OCH₂- or -CH₂-, and Ar is a substituted or unsubstituted phenyl group or a substituted or unsubstituted pyridyl group, or R¹ and R², when taken together, may form a 5- to 7-membered ring,
n is 1 to 3,
A is a substituted or unsubstituted aryl group, a substituted or unsubstituted heteroaromatic group, or a group represented by the formula: -N(R⁵)R⁶ wherein R⁵ and R⁶ are independently a hydrogen atom, a substituted or unsubstituted alkyl group or a substituted or unsubstituted cycloalkyl group, or R⁵ and R⁶, when taken together with the nitrogen atom to which they are bonded, may form a substituted or unsubstituted 5- to 8-membered saturated or unsaturated nitrogen-containing heterocyclic ring, said nitrogen-containing heterocyclic ring containing 0 or 1 oxygen atom, 0 or 1 sulfur atom and 1 or 2 nitrogen atoms, and
R³ and R⁴ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted and saturated or unsaturated aliphatic heterocyclic group, a substituted or unsubstituted aryl group or a substituted or unsubstituted heteroaromatic group, or R³ and R⁴, when taken together with the nitrogen atom to which they are bonded, may form a substituted or unsubstituted 5- to 10-membered saturated or unsaturated nitrogen-containing heterocyclic ring, said nitrogen-containing heterocyclic ring containing 0 to 2 oxygen atoms, 0 to 2 sulfur atoms and 1 to 3 nitrogen atoms, provided that when R³ and R⁴ are taken together with the nitrogen atom to which they are bonded, to form a piperidine ring, R¹ and R² are not hydrogen atoms at the same time, or a pharmaceutically acceptable salt thereof.
[2] A compound or a pharmaceutically acceptable salt thereof according to [1], wherein R³ and R⁴ are independently a hydrogen atom or a substituted or unsubstituted alkyl group, or R³ and R⁴, when taken together with the nitrogen atom to which they are bonded, may form a substituted or unsubstituted 5- to 10-membered saturated or unsaturated nitrogen-containing heterocyclic ring, said nitrogen-containing heterocyclic ring containing 0 to 2 oxygen atoms, 0 to 2 sulfur atoms and 1 to 3 nitrogen atoms.
[3] A compound or a pharmaceutically acceptable salt thereof according to [1] or [2], wherein A is a group represented by the formula: -N(R⁵)R⁶ wherein R⁵ and R⁶ are independently a hydrogen atom, a substituted or unsubstituted alkyl group or a substituted or unsubstituted cycloalkyl group, or R⁵ and R⁶, when taken together with the nitrogen atom to which they are bonded, may form a substituted or unsubstituted 5- to 8-membered saturated or unsaturated nitrogen-containing heterocyclic ring, said nitrogen-containing heterocyclic ring containing 0 or 1 oxygen atom, 0 or 1 sulfur atom and 1 or 2 nitrogen atoms.
[4] A compound or a pharmaceutically acceptable salt thereof according to [1] or [2], wherein A is a substituted or unsubstituted aryl group.
[5] A compound or a pharmaceutically acceptable salt thereof according to [1] or [2], wherein A is a substituted or unsubstituted heteroaromatic group.
[6] A compound or a pharmaceutically acceptable salt thereof according to [5], wherein the heteroaromatic group is a substituted or unsubstituted pyridyl group.
[7] A compound according to any one of [1] to [6], which is represented by the formula (2):

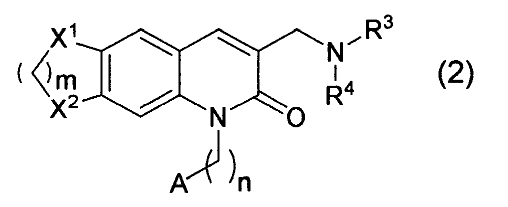

wherein A, n, R³ and R⁴ are as defined above; m is 1 to 3; and X¹ and X² are independently methylene or an oxygen atom, or a pharmaceutically acceptable salt thereof.
[8] A compound according to any one of [1] to [6], which is represented by the formula (3):

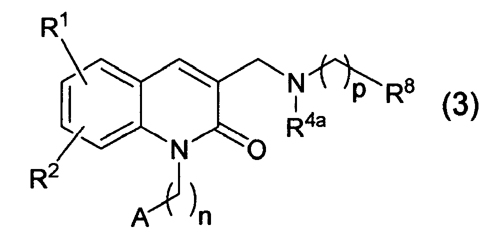

wherein A, n, R¹ and R² are as defined above; p is 1 to 4; R^{4a} is a hydrogen atom or an alkyl group; and R⁸ is a substituted or unsubstituted aryl group or a substituted or unsubstituted heteroaromatic group, or a pharmaceutically acceptable salt thereof.
[9] A compound according to any one of [1] to [6], which is represented by the formula (3a):

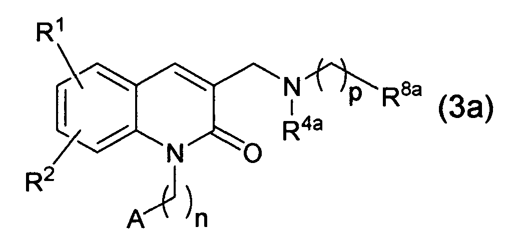

wherein A, n, R¹, R², p and R^{4a} are as defined above, and R^{8a} is a substituted or unsubstituted amino group or a substituted or unsubstituted carbamoyl group, or a pharmaceutically acceptable salt thereof.
[10] A compound or a pharmaceutically acceptable salt thereof according to [8] or [9], wherein A is a group represented by the formula: -N(R⁵)R⁶ wherein R⁵ and R⁶ are independently a hydrogen atom, a substituted or unsubstituted alkyl group or a substituted or unsubstituted cycloalkyl group, or R⁵ and R⁶, when taken together with the nitrogen atom to which they are bonded, may form a substituted or unsubstituted 5- to 8-membered saturated or unsaturated nitrogen-containing heterocyclic ring, said nitrogen-containing heterocyclic ring containing 0 or 1 oxygen atom, 0 or 1 sulfur atom and 1 or 2 nitrogen atoms.
[11] An SNS inhibitor comprising a compound or a pharmaceutically acceptable salt thereof according to any one of [1] to [10] as an active ingredient.
[12] A pharmaceutical composition for treatment or prophylaxis of neuropathic pain, nociceptive pain, urinary disturbance or multiple sclerosis comprising a compound or a pharmaceutically acceptable salt thereof according to any one of [1] to [10] as an active ingredient.

### Advantages of the Invention

By the present invention, an SNS inhibitor is provided which contains a compound having a 2-quinolone skeleton or a pharmaceutically acceptable salt thereof. The SNS inhibitor of the present invention is useful as a pharmaceutical composition for treatment or prophylaxis of all pathoses attributable to SNS. Specifically, the SNS inhibitor may be administered to a patient with neuropathic pain, nociceptive pain, urinary disturbance, multiple sclerosis or the like.

### BEST MODE FOR CARRYING OUT THE INVENTION

In the present description, the halogen atom includes fluorine atom, chlorine atom, bromine atom and iodine atom.

In the present description, the alkyl group refers to a linear or branched alkyl group of 1 to 6 carbon atoms. Specific examples thereof are methyl group, ethyl group, propyl group (1-propyl group), isopropyl group (2-propyl group), butyl group (1-butyl group), sec-butyl group (2-butyl group), isobutyl group (2-methyl-1-propyl group), t-butyl group (2-methyl-2-propyl group), pentyl group (1-pentyl group), hexyl group (1-hexyl group), etc. Preferable examples thereof are alkyl groups of 1 to 4 carbon atoms.
In the present description, the alkenyl group refers to a linear or branched alkenyl group of 2 to 6 carbon atoms. Specific examples thereof are vinyl group, 1-propenyl group, 2-propenyl group, 1-methylvinyl group, 1-butenyl group, 1-ethylvinyl group, 1-methyl-2-propenyl group, 2-butenyl group, 3-butenyl group, 2-methyl-1-propenyl group, 2-methyl-2-propenyl group, 1-pentenyl group, 1-hexenyl group, etc. Preferable examples thereof are alkenyl groups of 2 to 4 carbon atoms.
In the present description, the alkynyl group refers to a linear or branched alkynyl group of 2 to 6 carbon atoms. Specific examples thereof are ethynyl group, 1-propynyl group, 2-propynyl group, 1-butynyl group, 1-methyl-2-propynyl group, 3-butynyl group, 1-pentynyl group, 1-hexynyl group, etc. Preferable examples thereof are alkynyl groups of 2 to 4 carbon atoms.

In the present description, the alkoxy group refers to a linear or branched alkoxy group of 1 to 6 carbon atoms. Specific examples thereof are methoxy group, ethoxy group, propoxy group, 1-methylethoxy group, butoxy group, 1-methylpropoxy group, 2-methylpropoxy group, 1,1-dimethylethoxy group, pentyloxy group, hexyloxy group, etc. Preferable examples thereof are alkoxy groups of 1 to 4 carbon atoms.
In the present description, the alkylthio group refers to a linear or branched alkylthio group of 1 to 6 carbon atoms. Specific examples thereof are methylthio group, ethylthio group, propylthio group, 1-methylethylthio group, butylthio group, 1-methylpropylthio group, 2-methylpropylthio group, 1,1-dimethylethylthio group, pentylthio group, hexylthio group, etc. Preferable examples thereof are alkylthio groups of 1 to 4 carbon atoms.

In the present description, as the alkyl portion of the alkylcarbonyl group, the same groups as those exemplified above as the alkyl group are exemplified. Preferable examples of the alkylcarbonyl group are acetyl group, propionyl group, butyryl group, etc.
In the present description, the term "alkylcarbonyloxy group" means a group formed by bonding of an oxygen atom to the above-mentioned alkylcarbonyl group.
In the present description, as the alkoxy portion of the alkoxycarbonyl group, the same groups as those exemplified above as the alkoxy group are exemplified. Preferable examples of the alkoxycarbonyl group are methoxycarbonyl group, ethoxycarbonyl group, propoxycarbonyl group, butoxycarbonyl group, etc.
In the present description, the haloalkyl group includes linear or branched alkyl groups of 1 to 6 carbon atoms substituted by 1 to 5 halogen atoms which may be the same or different. Specific examples thereof are trifluoromethyl group, 2,2-difluoroethyl group, 2,2,2-trifluoroethyl group, 2-chloroethyl group, pentafluoroethyl group, 3,3,3-trifluoropropyl group, etc. Preferable examples thereof are haloalkyl groups of 1 to 4 carbon atoms.
In the present description, the haloalkoxy group includes linear or branched alkoxy groups of 1 to 6 carbon atoms substituted by 1 to 5 halogen atoms which may be the same or different. Specific examples thereof are trifluoromethoxy group, 2,2-difluoroethoxy group, 2,2,2-trifluoroethoxy group, 2-chloroethoxy group, pentafluoroethoxy group, 3,3,3-trifluoropropoxy group, etc. Preferable examples thereof are haloalkoxy groups of 1 to 4 carbon atoms.

In the present description, the cycloalkyl group refers to a 3- to 8-membered cycloalkyl group. Specific examples thereof are cyclopropyl group, cyclobutyl group, cyclopentyl group, cyclohexyl group, cycloheptyl group, cyclooctyl group, etc. Preferable examples thereof are 4- to 6-membered cycloalkyl groups.
In the present description, the cycloalkenyl group refers to a 4- to 8-membered cycloalkenyl group. Specific examples thereof are cyclobutenyl group, cyclopentenyl group, cyclohexenyl group, cycloheptenyl group, cyclooctenyl group, etc. The bonding position of the cycloalkenyl group is not particularly limited. Preferable examples of the cycloalkenyl group are 5- or 6-membered cycloalkenyl groups.

In the present description, the aryl group refers to a 6- to 10-membered aryl group. Specific examples thereof are phenyl group, 1-naphthyl group, 2-naphthyl group, etc.
In the present description, the term "aryl" in the arylcarbonyl group or the arylsulfonyl group has the same meaning as defined above.
In the present description, the saturated aliphatic heterocyclic group refers to a 4- to 8-membered saturated aliphatic heterocyclic group containing 1 to 3 heteroatoms selected from 0 to 3 nitrogen atoms, 0 to 2 oxygen atoms and 0 to 2 sulfur atoms. The bonding position of the saturated aliphatic heterocyclic group is not particularly limited as long as the bonding is chemically stable. Specific examples of the saturated aliphatic heterocyclic group are azetidinyl group, pyrrolidinyl group, piperidyl group, piperidino group, piperazinyl group, azepanyl group, azocanyl group, tetrahydrofuryl group, tetrahydrothienyl group, tetrahydropyranyl group, morpholinyl group, morpholino group, thiomorpholinyl group, 1,4-dioxanyl group, etc.

In the present description, the unsaturated aliphatic heterocyclic group refers to a 5- to 10-membered monocyclic or bicyclic unsaturated aliphatic heterocyclic group which contains 1 to 3 heteroatoms selected from 0 to 3 nitrogen atoms, 0 to 2 oxygen atoms and 0 to 2 sulfur atoms and contains one or three double bonds. The bonding position of the unsaturated aliphatic heterocyclic group is not particularly limited as long as the bonding is chemically stable. Specific examples of the unsaturated aliphatic heterocyclic group are 2-pyrrolinyl group, 2-imidazolinyl group, tetrahydroisoquinoline group, etc.

In the present description, the heteroaromatic group refers to a 5- to 10-membered monocyclic or bicyclic heteroaromatic group containing 1 to 4 heteroatoms selected from 0 to 4 nitrogen atoms, 0 to 2 oxygen atoms and 0 to 2 sulfur atoms. The bonding position of the heteroaromatic group is not particularly limited as long as the bonding is chemically stable. Specific examples of the heteroaromatic group are furyl group, thienyl group, pyrrolyl group, thiazolyl group, imidazolyl group, pyrazolyl group, furazanyl group, triazolyl group, pyridyl group, pyrimidinyl group, pyrazinyl group, indolyl group, quinolyl group, isoquinolyl group, quinazolinyl group, imidazo[2,1-b][1,3]thiazolyl group, etc.
In the present description, the term "heteroaromatic" in the heteroaromatic carbonyl group or the heteroaromatic sulfonyl group has the same meaning as defined above.
When L is -OCH₂- in the group represented by the formula: -L-Ar wherein L is a single bond, -O-, -OCH₂- or -CH₂-, and Ar is a substituted or unsubstituted phenyl group or a substituted or unsubstituted pyridyl group, the formula indicates that a 2-quinolone skeleton is bonded to the left of -OCH₂- and Ar to the right. As the pyridyl group for Ar, 2-pyridyl group, 3-pyridyl group and 4-pyridyl group may be exemplified. When the phenyl or pyridyl group for Ar is substituted, the substituent includes halogen atoms, alkyl groups, haloalkyl groups, alkoxy groups and haloalkoxy groups. Preferable examples of the group represented by the formula: -L-Ar are substituted or unsubstituted phenyl group, substituted or unsubstituted phenyloxy group, and substituted or unsubstituted benzyloxy group. Of these, phenyloxy group is especially preferable.
In the formula (1), preferable examples of R¹ or R² are hydrogen atom, fluorine atom, chlorine atom, methyl group, ethyl group, propyl group, trifluoromethyl group, methoxy group, trifluoromethoxy group, methylthio group, substituted or unsubstituted phenyl group, substituted or unsubstituted phenyloxy group, and substituted or unsubstituted benzyloxy group. Of these, fluorine atom, chlorine atom, methyl group, methoxy group, trifluoromethoxy group, phenyloxy group and benzyloxy group are especially preferable.
The 5- to 7-membered ring which R¹ and R² form when taken together refers to a 5- to 7-membered ring formed when R¹ and R² are bonded to adjacent carbon atoms, respectively, and taken together to represent a trimethylene group, a tetramethylene group, a pentamethylene group, a methylenedioxy group, an ethylenedioxy group, a trimethylenedioxy group or the like. Preferably, the 5- to 7-membered ring refers to a 5- to 7-membered ring formed when R¹ bonded to the carbon atom in the 6-position of the 2-quinolone skeleton and R² bonded to the carbon atom in the 7-position are taken together to represent a trimethylene group, a tetramethylene group, a pentamethylene group, a methylenedioxy group, an ethylenedioxy group or a trimethylenedioxy group. Specific examples of the 2-quinolone skeleton in the case of the formation of said 5- to 7-membered ring are those represented by the formulas (5) to (10):

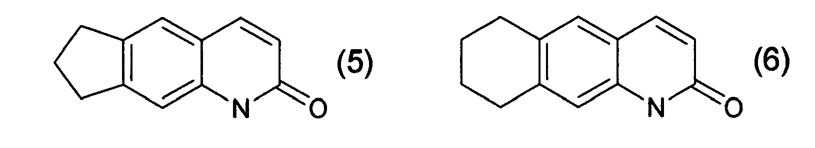

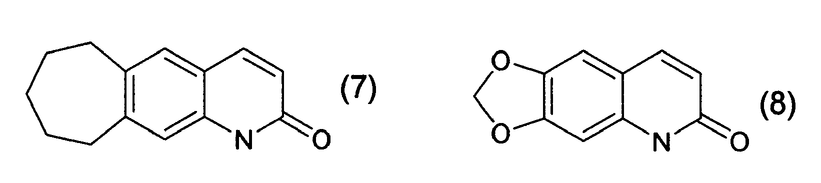

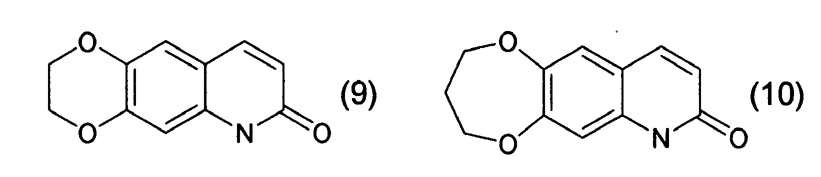

More preferable examples of the 5- to 7-membered ring which R¹ and R² form when taken together are 5-membered rings formed when R¹ bonded to the carbon atom in the 6-position of the 2-quinolone skeleton and R² bonded to the carbon atom in the 7-position are taken together to represent a trimethylene group or a methylenedioxy group, namely, the above formula (5) and formula (8).
The substituent(s) of the aryl group or heteroaromatic group for A includes halogen atoms, alkyl groups, haloalkyl groups, alkoxy groups, haloalkoxy groups, carboxyl group, alkoxycarbonyl groups, etc. The aryl group or the heteroaromatic group may be substituted by 1 to 3 substituents which may be the same or different. Specific examples of the substituent(s) are fluorine atom, chlorine atom, methyl group, trifluoromethyl group, methoxy group, trifluoromethoxy group, carboxyl group, methoxycarbonyl group, etc. A preferable example of the aryl group for A is phenyl group. A preferable example of the heteroaromatic group for A is pyridyl group, and the bonding position of the pyridyl group is on any carbon atom.
In the group represented by the formula: - N(R⁵)R⁶ for A, the alkyl group for each of R⁵ and R⁶ includes methyl group, ethyl group and propyl group. The cycloalkyl group for each of R⁵ and R⁶ includes cyclopentyl group and cyclohexyl group. The substituent(s) of the alkyl group or the cycloalkyl group includes halogen atoms, hydroxyl group, carboxyl group, alkoxy groups and alkoxycarbonyl groups. An especially preferable example of the alkyl group for each of R⁵ and R⁶ is methyl group. Preferable examples of the nitrogen-containing heterocyclic ring which R⁵ and R⁶ form when taken together are pyrrolidine, piperidine, piperazine, azepane, azocane, morpholine, etc. Of these, pyrrolidine, piperidine and morpholine are especially preferable.

When the alkyl group, alkenyl group, alkynyl group, cycloalkyl group, cycloalkenyl group, saturated or unsaturated aliphatic heterocyclic group, aryl group or heteroaromatic group for R³ or R⁴ is substituted, the substituent(s) is selected from the following groups a) to e) and such a group for R³ or R⁴ may be substituted by 1 to 5 substituents which may be the same or different:
a) halogen atoms, hydroxyl group, carboxyl group and cyano group;
b) substituted or unsubstituted amino groups, substituted or unsubstituted carbamoyl groups and substituted or unsubstituted sulfamoyl groups; [the substituents of the substituted amino groups, the substituted carbamoyl groups and the substituted sulfamoyl groups include the following (1) and (2):
   (1) alkyl groups, alkylcarbonyl groups and alkylsulfonyl groups
      [the groups in this group may be substituted by one or more substituents selected from halogen atoms, hydroxyl group, carboxyl group, alkoxy groups, haloalkoxy groups, cycloalkyl groups, saturated or unsaturated aliphatic heterocyclic groups, substituted or unsubstituted aryl groups and substituted or unsubstituted heteroaromatic groups (the substituents of said substituted aryl groups and said substituted heteroaromatic groups include halogen atoms, alkyl groups, alkoxy groups, haloalkyl groups and haloalkoxy groups).], and
   (2) aryl groups, heteroaromatic groups, arylcarbonyl groups, heteroaromatic carbonyl groups, arylsulfonyl groups and heteroaromatic sulfonyl groups
      [the groups in this group may be substituted by one or more substituents selected from halogen atoms, alkyl groups, alkoxy groups, haloalkyl groups and haloalkoxy groups.]. Alternatively, two substituents may bind to each other to form a nitrogen-containing heterocyclic ring together with the adjacent nitrogen atom [said nitrogen-containing heterocyclic ring includes pyrrolidine, piperidine, piperazine, azepane, azocane, morpholine, etc.].];
c) alkylcarbonyl groups, alkoxy groups, alkoxycarbonyl groups, alkylcarbonyloxy groups and alkylthio groups [these groups may be substituted by one or more substituents selected from halogen atoms, hydroxyl group, amino group optionally substituted by one or two alkyl groups which may be the same or different, carboxyl group, alkoxy groups, alkoxycarbonyl groups, optionally substituted aryl groups and optionally substituted heteroaromatic groups, and the substituents of said aryl groups and said heteroaromatic groups include halogen atoms, alkyl groups, alkoxy groups, haloalkyl groups and haloalkoxy groups.];
d) cycloalkyl groups, cycloalkenyl groups and saturated or unsaturated aliphatic heterocyclic groups [these groups may be substituted by one or more substituents selected from halogen atoms, hydroxyl group, amino group optionally substituted by one or two alkyl groups which may be the same or different, carboxyl group, alkoxycarbonyl groups, alkoxy groups, optionally substituted alkyl groups and optionally substituted aryl groups, the substituents of said alkyl groups include halogen atoms, hydroxyl group, carboxyl group and alkoxy groups, and the substituents of said aryl groups include halogen atoms, alkyl groups, hydroxyl group, carboxyl group and alkoxy groups.]; and
e) aryl groups, heteroaromatic groups, arylcarbonyl groups and heteroaromatic carbonyl groups [these groups may be substituted by one or more substituents selected from the following (3) to (6):
   (3) halogen atoms, hydroxyl group, carboxyl group, alkoxycarbonyl groups, alkoxy groups, haloalkoxy groups and methylenedioxy group,
   (4) substituted or unsubstituted amino groups, substituted or unsubstituted carbamoyl groups and substituted or unsubstituted sulfamoyl groups
      [the substituents of the substituted amino groups, substituted carbamoyl groups and substituted sulfamoyl groups include the following (i) and (ii):
      (i) alkyl groups, alkylcarbonyl groups and alkylsulfonyl groups
         [the groups in this group may be substituted by one or more substituents selected from halogen atoms, hydroxyl group, carboxyl group, alkoxy groups, haloalkoxy groups, cycloalkyl groups, saturated or unsaturated aliphatic heterocyclic groups, substituted or unsubstituted aryl groups and substituted or unsubstituted heteroaromatic groups (the substituents of said substituted aryl groups and said substituted heteroaromatic groups include halogen atoms, alkyl groups, alkoxy groups, haloalkyl groups and haloalkoxy groups).], and
      (ii) aryl groups, heteroaromatic groups, arylcarbonyl groups, heteroaromatic carbonyl groups, arylsulfonyl groups and heteroaromatic sulfonyl groups
         [the groups in this group may be substituted by one or more substituents selected from halogen atoms, alkyl groups, alkoxy groups, haloalkyl groups and haloalkoxy groups]. Alternatively, two substituents may bind to each other to form a nitrogen-containing heterocyclic ring together with the adjacent nitrogen atom (said nitrogen-containing heterocyclic ring includes pyrrolidine, piperidine, piperazine, azepane, azocane, morpholine, etc.).],
   (5) optionally substituted alkyl groups
      [the substituents of said alkyl groups include halogen atoms, hydroxyl group, carboxyl group and alkoxy groups.], and
   (6) optionally substituted aryl groups
      [the substituents of said aryl groups include halogen atoms, alkyl groups, hydroxyl group, carboxyl group and alkoxy groups.]].
Preferable examples of R⁴ are hydrogen atom and unsubstituted alkyl groups. Of these, hydrogen atom and methyl group are especially preferable. Preferable examples of R³ are substituted alkyl groups. Preferable examples of the substituents of said alkyl groups are the substituted or unsubstituted amino groups, substituted or unsubstituted carbamoyl groups and substituted or unsubstituted sulfamoyl groups described in the above item b) and the aryl groups and heteroaromatic groups described in the above item e). Specific examples of said aryl groups and said heteroaromatic groups are phenyl group, furyl group, thiazolyl group, imidazolyl group, pyrazolyl group, furazanyl group, indolyl group, etc. These groups may be substituted by the same substituents as those described in the above items (3) to (6).

Specific examples of the 5- to 10-membered saturated or unsaturated monocyclic or bicyclic nitrogen-containing heterocyclic ring which R³ and R⁴ form when taken together are pyrrolidine, piperidine, azepane, azocane, piperazine, morpholine, thiomorpholine, tetrahydroisoquinoline, etc.
The substituent(s) of the substituted nitrogen-containing heterocyclic ring which R⁵ and R⁶ in A form when taken together and the substituent(s) of the substituted 5- to 10-membered saturated or unsaturated nitrogen-containing heterocyclic ring which R³ and R⁴ form when taken together are selected from the following groups f) to j) and each of these nitrogen-containing heterocyclic rings may be substituted by 1 to 5 substituents which may be the same or different:
f) halogen atoms, hydroxyl group, carboxyl group and cyano group;
g) substituted or unsubstituted amino groups [the substituents of the substituted amino groups include the following (7) to (9):
   (7) alkyl groups, alkylcarbonyl groups and alkylsulfonyl groups
      [the groups in this group may be substituted by any of halogen atoms, hydroxyl group, carboxyl group, alkoxy groups, haloalkoxy groups, cycloalkyl groups, saturated or unsaturated aliphatic heterocyclic groups, substituted or unsubstituted aryl groups and substituted or unsubstituted heteroaromatic groups (the substituents of said substituted aryl groups and said substituted heteroaromatic groups include halogen atoms, alkyl groups, alkoxy groups, haloalkyl groups and haloalkoxy groups).],
   (8) carbamoyl group
      [the group in this group may be substituted by any of substituted or unsubstituted alkyl groups and substituted or unsubstituted cycloalkyl groups (the substituents of said substituted alkyl groups and said substituted cycloalkyl groups include halogen atoms, hydroxyl group, carboxyl group and alkoxy groups).], and
   (9) aryl groups, heteroaromatic groups, arylcarbonyl groups, heteroaromatic carbonyl groups, arylsulfonyl groups and heteroaromatic sulfonyl groups
      [the groups in this group may be substituted by any of halogen atoms, alkyl groups, alkoxy groups, haloalkyl groups and haloalkoxy groups.]. Alternatively, two substituents may bind to each other to form a nitrogen-containing heterocyclic ring together with the adjacent nitrogen atom [said nitrogen-containing heterocyclic ring includes pyrrolidine, piperidine, piperazine, azepane, azocane, morpholine, etc.].];
h) substituted or unsubstituted carbamoyl groups and substituted or unsubstituted sulfamoyl groups [the substituents of the substituted carbamoyl groups and the substituted sulfamoyl groups include the following (10) and (11):
   (10) alkyl groups, alkylcarbonyl groups and alkylsulfonyl groups
      [the groups in this group may be substituted by any of halogen atoms, hydroxyl group, carboxyl group, alkoxy groups, haloalkoxy groups, cycloalkyl groups, saturated or unsaturated aliphatic heterocyclic groups, substituted or unsubstituted aryl groups and substituted or unsubstituted heteroaromatic groups (the substituents of said substituted aryl groups and said substituted heteroaromatic groups include halogen atoms, alkyl groups, alkoxy groups, haloalkyl groups and haloalkoxy groups).], and
   (11) aryl groups, heteroaromatic groups, arylcarbonyl groups, heteroaromatic carbonyl groups, arylsulfonyl groups and heteroaromatic sulfonyl groups
      [the groups in this group may be substituted by any of halogen atoms, alkyl groups, alkoxy groups, haloalkyl groups and haloalkoxy groups.]. Alternatively, two substituents may bind to each other to form a nitrogen-containing heterocyclic ring together with the adjacent nitrogen atom [said nitrogen-containing heterocyclic ring includes pyrrolidine, piperidine, piperazine, azepane, azocane, morpholine, etc.].];
      i) alkyl groups, alkylcarbonyl groups, alkoxy groups, alkoxycarbonyl groups, alkylcarbonyloxy groups, alkylthio groups and alkylsulfonyl groups
         [these groups may be substituted by a substituent(s) selected from halogen atoms, hydroxyl group, amino group optionally substituted by one or two alkyl groups which may be the same or different, carboxyl group, alkoxy groups, alkoxycarbonyl groups, optionally substituted aryl groups and optionally substituted heteroaromatic groups, and the substituents of said aryl groups and said heteroaromatic groups include halogen atoms, alkyl groups, alkoxy groups, haloalkyl groups and haloalkoxy groups.]; and
      j) aryl groups, heteroaromatic groups, arylcarbonyl groups, heteroaromatic carbonyl groups, arylsulfonyl groups and heteroaromatic sulfonyl groups
         [these groups may be substituted by a substituent(s) selected from halogen atoms, hydroxyl group, amino group optionally substituted by one or two alkyl groups which may be the same or different, carboxyl group, alkoxycarbonyl groups, alkoxy groups and optionally substituted alkyl groups, and the substituents of said alkyl groups include halogen atoms, hydroxyl group, carboxyl group and alkoxy groups.].

In the formula (2), both X¹ and X² are preferably methylene or oxygen atoms, and m is preferably 1.
In the formula (3), preferable examples of R^{4a} are hydrogen atom and unsubstituted alkyl groups. Of these, hydrogen atom and methyl group are especially preferable. Preferable examples of R⁸ are the aryl groups and heteroaromatic groups described in the above item e). Specific examples of R⁸ are phenyl group, furyl group, thiazolyl group, imidazolyl group, pyrazolyl group, furazanyl group, indolyl group, etc. These groups may be substituted by the same substituents as those described in the above items (3) to (6).
In the formula (3a), preferable examples of R^{8a} are substituted or unsubstituted carbamoyl groups. As the substituents of the substituted carbamoyl groups, there are exemplified the same substituents as those described in the above items (1) and (2).

The compound represented by the general formula (1) of the present invention may be prepared, for example, by the following process:

### Reaction Scheme-1

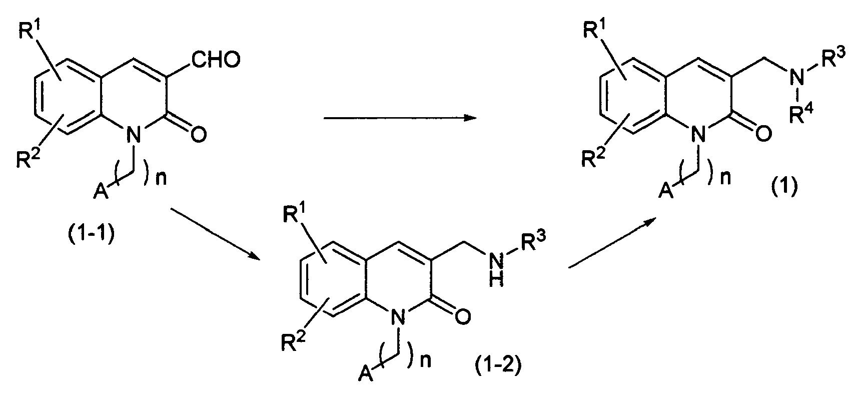

wherein R¹, R², R³, R⁴, n and A are as defined above.
The compound represented by the formula (1) may be prepared by reductive amination reaction of a compound (1-1) with a desirable secondary amine compound. The compound represented by the formula (1) may be prepared also by obtaining a compound (1-2) by reductive amination of a compound (1-1) with a desirable primary amine compound and then subjecting the compound (1-2) to reductive amination with a desirable aldehyde. As a solvent, there may be used ether solvents such as tetrahydrofuran, 1,4-dioxane, etc.; halogenated solvents such as dichloromethane, chloroform, 1,2-dichloroethane, etc.; ethyl acetate; N,N'-dimethylformamide; acetonitrile; and the like. As a reducing agent, there may be used sodium borohydride, sodium triacetoxyborohydride, sodium cyanoborohydride, etc. The reaction temperature is -20°C to the reflux temperature of the solvent for reaction and is preferably, in particular, 0°C to the vicinity of room temperature.
The compound of the formula (1) may be prepared from a compound (1-2) also by the process shown in the following reaction scheme-2:

### Reaction Scheme-2

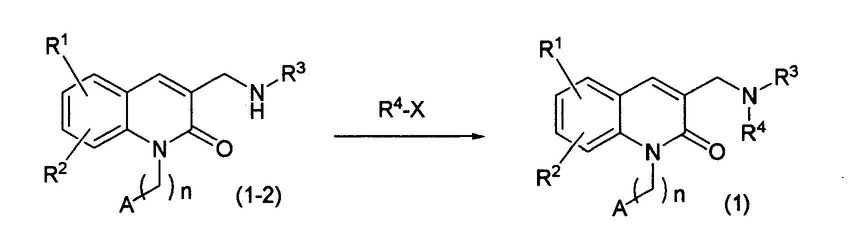

wherein R¹, R², R³, R⁴, n and A are as defined above, and X is a substituent having leaving capability, such as a halogen atom, mesyl group, tosyl group or the like.
The compound of the formula (1) may be prepared by reacting the compound (1-2) with a compound represented by the formula: R⁴-X in the presence of a base in a solvent such as an ether solvent (e.g. tetrahydrofuran or 1,4-dioxane), a halogenated solvent (e.g. dichloromethane, chloroform or 1,2-dichloroethane), ethyl acetate, N,N'-dimethylformamide or acetonitrile at 0°C to the reflux temperature of the solvent for reaction. As the base, there may be used potassium carbonate, cesium carbonate, sodium hydroxide, sodium hydride, potassium hydride, potassium t-butoxide, etc. The base is not particularly limited.
Furthermore, the compound represented by the formula (1) may be prepared also by the process shown in reaction scheme-3:

### Reaction Scheme-3

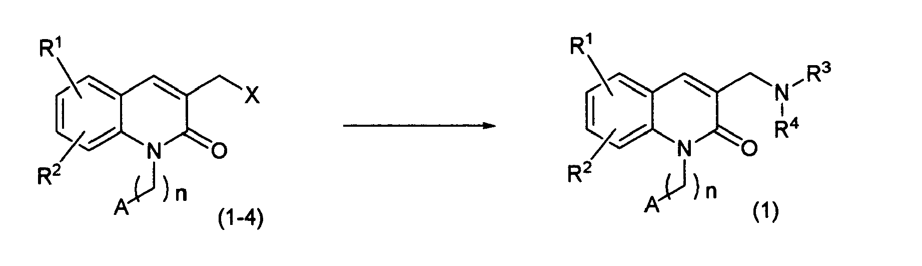

wherein R¹, R², R³, R⁴, n, A and X are as defined above.
The compound represented by the formula (1) may be prepared by reacting a compound (1-4) with a desirable amine compound in the presence of a base in a solvent such as an ether solvent (e.g. tetrahydrofuran or 1,4-dioxane), a halogenated solvent (e.g. dichloromethane, chloroform or 1,2-dichloroethane), ethyl acetate, N,N'-dimethylformamide or acetonitrile at 0°C to the reflux temperature of the solvent for reaction. As the base, there may be used potassium carbonate, cesium carbonate, sodium hydroxide, sodium hydride, potassium hydride, potassium t-butoxide, etc.
The base is not particularly limited.
The above-mentioned compound (1-1) may be prepared, for example, by the process shown in the following reaction scheme-4:

### Reaction Scheme-4

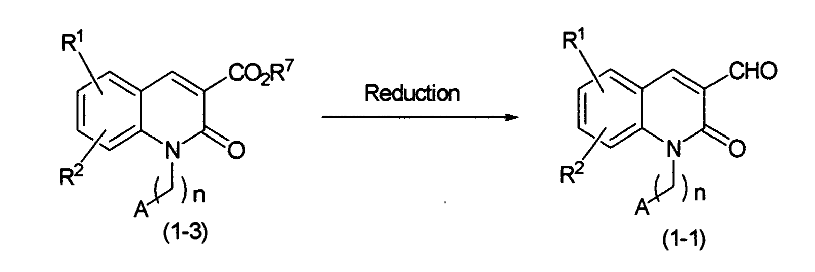

wherein R¹, R², n and A are as defined above, and R⁷ is an alkyl group of 1 to 4 carbon atoms.
The compound (1-1) may be prepared by reducing a compound (1-3). The reduction is carried out at -78°C to 0°C by using a solvent such as an ether solvent (e.g. tetrahydrofuran or diethyl ether) or a halogenated solvent (e.g. dichloromethane or 1,2-dichloroethane). As a reducing agent, diisobutylaluminum hydride (DIBAL) is especially preferable. The compound (1-1) may be prepared also by reducing a compound (1-3) to an alcohol with a reducing agent such as DIBAL or aluminum lithium hydride, followed by oxidation with an oxidizing agent such as manganese dioxide.
The above-mentioned compound (1-4) may be prepared, for example, by the process shown in the following reaction scheme-5:

### Reaction Scheme-5

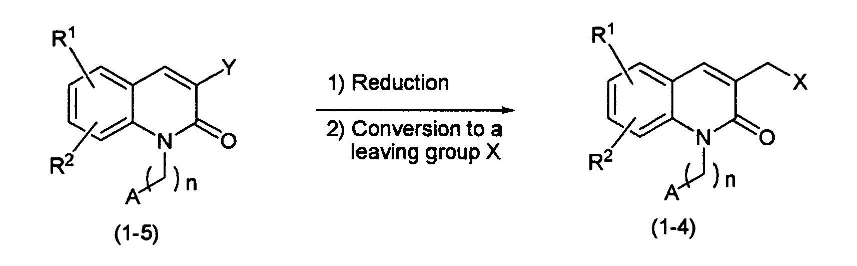

wherein R¹, R², n, A and X are as defined above, and Y is -CO₂R⁷ (wherein R⁷ is as defined above) or CHO.
The compound (1-4) may be prepared by converting a compound (1-5) to an alcohol by reduction and then converting the hydroxyl group to a leaving group X. The reduction step is carried out at -78°C to 0°C by using a solvent such as an ether solvent (e.g. tetrahydrofuran or diethyl ether), a halogenated solvent (e.g. dichloromethane or 1,2-dichloroethane) or an alcohol solvent (e.g. methanol or ethanol) and using a reducing agent such as sodium borohydride, aluminum lithium hydride or diisobutylaluminum hydride. In the step of the conversion to the leaving group, when the leaving group X is a mesyl group or a tosyl group, a mesyl compound or a tosyl compound, respectively, may be obtained by reacting a corresponding chloride in the presence of a base such as triethylamine or pyridine. When the leaving group X is a halogen atom, the conversion may be carried out according to the method described in R.C. Larock, Comprehensive Organic Transformation, VCH Publishers Inc. (1989), Jikken Kagaku Koza (Experimental Chemistry), Fourth Edition, Maruzen Co., Ltd., Shin Jikken Kagaku Koza (New Experimental Chemistry), Maruzen Co., Ltd., or the like. For example, a bromide may be obtained by adding triphenylphosphine and carbon tetrabromide in dichloromethane.
The above-mentioned compound (1-5) may be prepared, for example, by the following process:

### Reaction Scheme-6

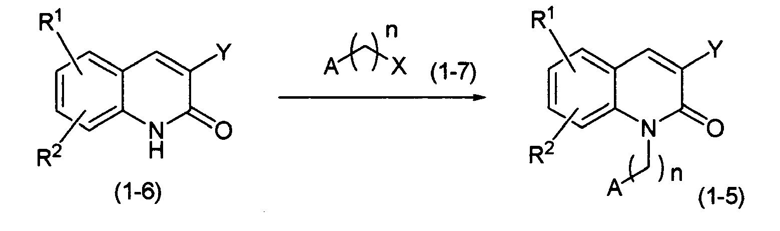

wherein R¹, R², n, A, X and Y are as defined above.
The compound (1-5) may be prepared by reacting a compound (1-6) with a compound (1-7) in the presence of a base in a solvent such as an ether solvent (e.g. tetrahydrofuran or 1,4-dioxane), a halogenated solvent (e.g. dichloromethane, chloroform or 1,2-dichloroethane), ethyl acetate, N,N'-dimethylformamide or acetonitrile at 0°C to the reflux temperature of the solvent for reaction. As the base, there may be used potassium carbonate, cesium carbonate, sodium hydroxide, sodium hydride, potassium hydride, potassium t-butoxide, etc. The base is not particularly limited.
As the above-mentioned compound (1-6), a compound (1-8) in which Y is -CO₂R⁷ (wherein R⁷ is as defined above) may be prepared, for example, by the following process:

### Reaction Scheme-7

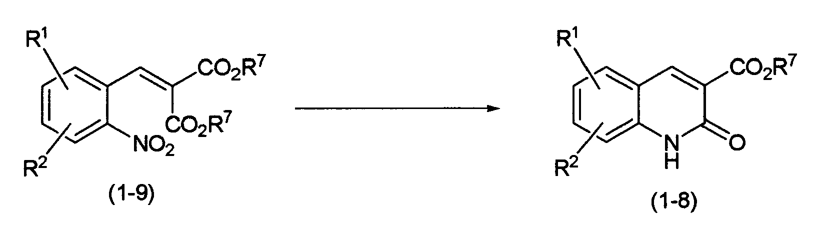

wherein R¹, R² and R⁷ are as defined above.
The compound (1-8) may be prepared by reducing the nitro group of a compound (1-9). As a reducing agent, there are preferably used metal reducing agents such as iron, tin chloride, titanium trichloride, etc. A solvent for reaction is not particularly limited as long as it does not inhibit the reaction. As the solvent, there may be used ether solvents such as tetrahydrofuran, 1,4-dioxane, etc.; halogenated solvents such as dichloromethane, chloroform, 1,2-dichloroethane, etc.; alcohol solvents such as methanol, ethanol, etc.; acetic acid; ethyl acetate; N,N'-dimethylformamide; acetonitrile; mixed solvents thereof; and the like. It is also possible to add ammonium chloride as an additive. The reaction temperature ranges from room temperature to the reflux temperature of the solvent.
The above-mentioned compound (1-9) may be prepared, for example, by the following process:

### Reaction Scheme-8

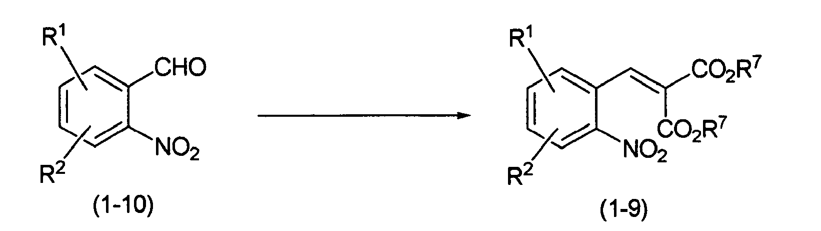

wherein R¹, R² and R⁷ are as defined above.
The compound (1-9) may be prepared by heating a commercial or well-known compound (1-10) and a dialkyl malonate in the presence of a base. A solvent for reaction is not particularly limited as long as it does not inhibit the reaction. As the solvent, there may be used ether solvents such as tetrahydrofuran, 1,4-dioxane, etc.; halogenated solvents such as dichloromethane, chloroform, 1,2-dichloroethane, etc.; alcohol solvents such as methanol, ethanol, etc.; acetic acid; ethyl acetate; N,N'-dimethylformamide; acetonitrile; mixed solvents thereof; and the like. As the base, there may be used organic bases such as piperidine, pyrrolidine, etc.
As the above-mentioned compound (1-6), for example, a compound (1-11) in which Y is -CHO may be prepared by the following process:

### Reaction Scheme-9

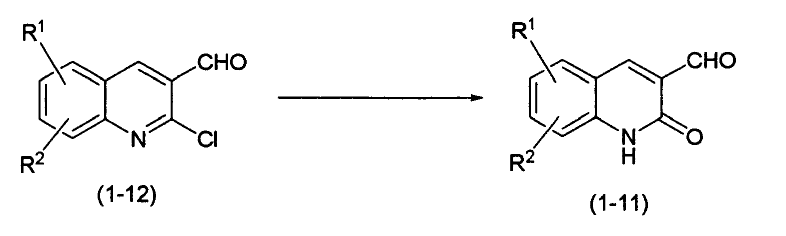

wherein R¹ and R² are as defined above.
The compound (1-11) may be prepared by hydrolyzing a compound (1-12). Although the reaction can be carried out by heating under acidic or basic conditions, it is preferably carried out under acidic conditions. As an acid, there are used acetic acid, hydrochloric acid, sulfuric acid, trifluoroacetic acid, etc. As a solvent, there are used ether solvents such as tetrahydrofuran, 1,4-dioxane, etc.; alcohol solvents such as methanol, ethanol, etc.; acetic acid; N,N'-dimethylformamide; acetonitrile; water; and mixed solvents thereof. The reaction temperature ranges from room temperature to the reflux temperature of the solvent.
The above-mentioned compound (1-12) may be prepared by the following process based on, for example, the process described in literature (J. Chem. Soc. Perkin I, 1981, 1520.):

### Reaction Scheme-10

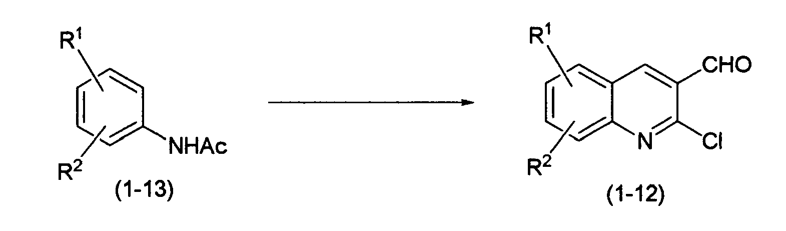

wherein R¹ and R² are as defined above.
Specifically, the compound (1-12) may be prepared by mixing a compound (1-13) with 3 to 10 equivalents of phosphorus oxychloride and 2 to 4 equivalents of N,N'-dimethylformamide and heating the resulting mixture. It is preferable to use 7 equivalents of phosphorus oxychloride and 3 equivalents of N,N'-dimethylformamide.
The above-mentioned compound (1-13) may be prepared by the following process:

### Reaction Scheme-11

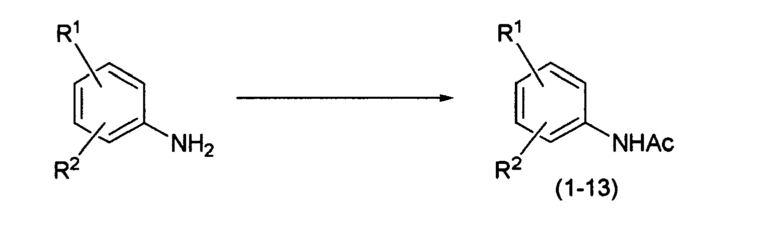

wherein R¹ and R² are as defined above.
The compound (1-13) may be prepared by acetylating a commercial or well-known substituted aniline with acetic anhydride or acetyl chloride in the presence of a base at 0°C to room temperature in a solvent such as an ether solvent (e.g. tetrahydrofuran or 1,4-dioxane), a halogenated solvent (e.g. dichloromethane or chloroform), ethyl acetate, N,N'-dimethylformamide or acetonitrile. As the base, pyridine, triethylamine, dimethylaminopyridine and the like may be used. The base is not particularly limited.
A compound of the present invention represented by the general formula (1) in which n is 2 or 3 and A is the formula: -N(R⁵)R⁶, namely, a compound of the present invention represented by the general formula (11) may be prepared, for example, by the following process:

### Reaction Scheme-12

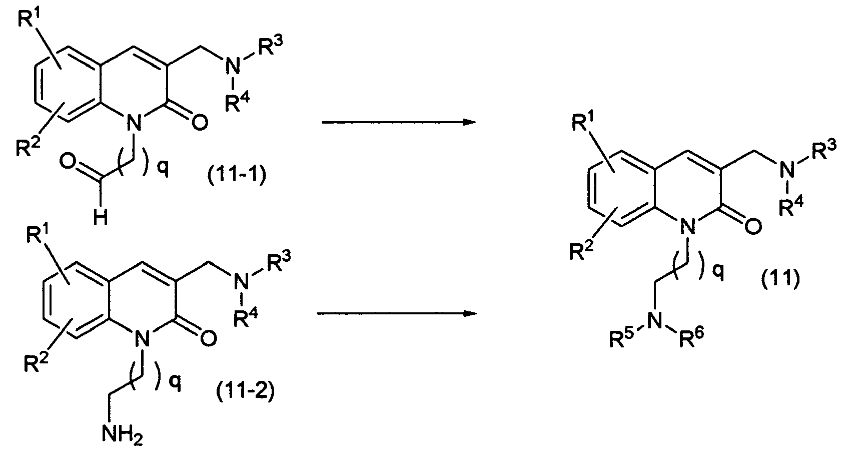

wherein R¹, R², R³, R⁴, R⁵ and R⁶ are as defined above, and q is 1 or 2.
The compound represented by the formula (11) may be prepared by reductive amination reaction of a compound (11-1) with a desirable amine compound or reductive amination reaction of a compound (11-2) with a desirable carbonyl compound. This preparation process is the same as in the above reaction scheme-1.
The above-mentioned compound of the formula (11-1) may be prepared, for example, from a compound (11-3) by the process shown in the following reaction scheme-13.

### Reaction Scheme-13

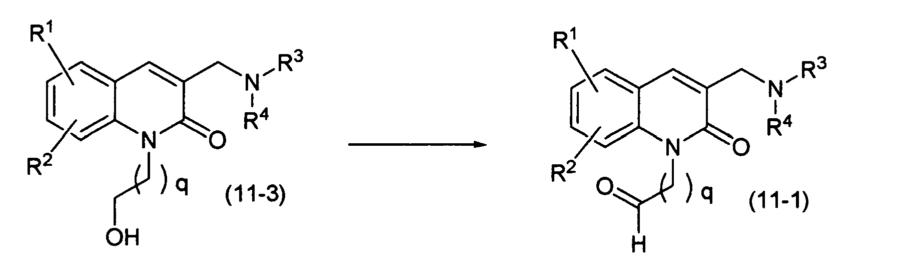

wherein R¹, R², R³, R⁴ and q are as defined above.
Oxidation of the compound (11-3) into the compound (11-1) may be carried out according to the method described in R.C. Larock, Comprehensive Organic Transformation, VCH Publishers Inc. (1989), Jikken Kagaku Koza (Experimental Chemistry), Fourth Edition, Maruzen Co., Ltd., Shin Jikken Kagaku Koza (New Experimental Chemistry), Maruzen Co., Ltd., or the like. The compound (11-1) may be obtained, for example, by the Swern oxidation described in the reference example given hereinafter.
The above-mentioned compound represented by the formula (11-2) may be prepared, for example, from a compound represented by the formula (11-3) by the process shown in reaction scheme-14.

### Reaction Scheme-14

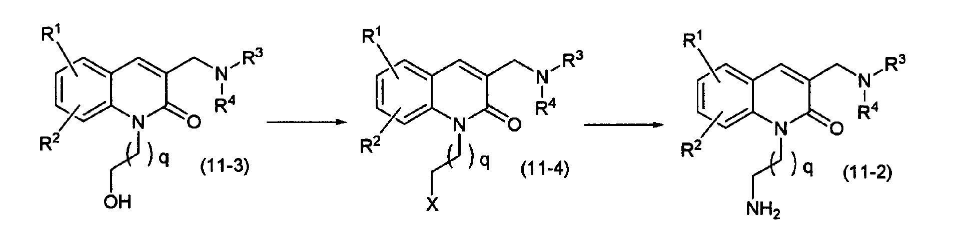

wherein R¹, R², R³, R⁴, X and q are as defined above.
A compound (11-4) may be prepared from the compound (11-3) by the same process as in the above reaction scheme-5. The compound (11-4) may be converted to the compound (11-2) according to the method described in R.C. Larock, Comprehensive Organic Transformation, VCH Publishers Inc. (1989), Jikken Kagaku Koza (Experimental Chemistry), Fourth Edition, Maruzen Co., Ltd., Shin Jikken Kagaku Koza (New Experimental Chemistry), Maruzen Co., Ltd., or the like. The compound (11-2) may be obtained, for example, by reacting potassium phthalimide with the compound (11-4), followed by hydrolysis with an acid, a base, hydrazine or the like.
The above-mentioned compound represented by the formula (11-3) may be prepared, for example, from a compound represented by the formula (1-11) by the process shown in reaction scheme-15.

### Reaction Scheme-15

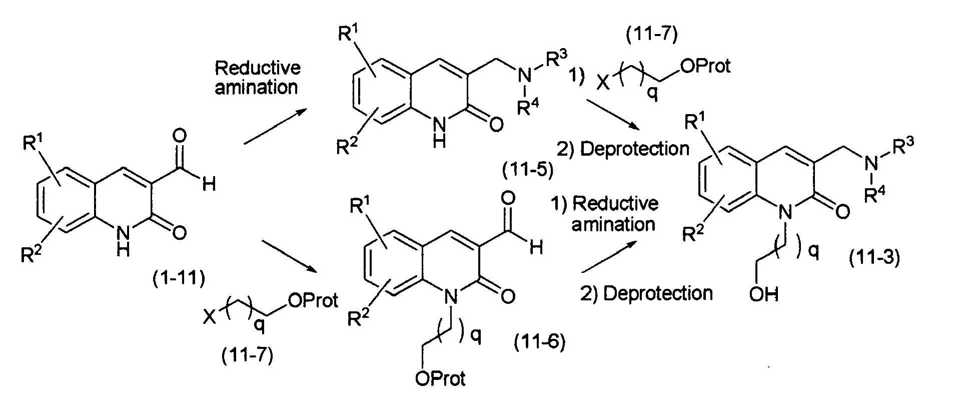

wherein R¹, R², R³, R⁴, X and q are as defined above, and Prot is a protective group.
The compound (11-3) may be prepared by reacting a compound (11-5) obtained from the compound (1-11) by the same process as in the above reaction scheme-1 with a compound (11-7) by the same method as in the above reaction scheme-6, followed by deprotection. As to the kind of Prot and the deprotection, a protective group and a deprotection method may be employed which are well known to those skilled in the art. For example, "T. W. Green et al., Protective Groups in Organic Synthesis, John Wiley & Sons, Inc., 1991" or the like may be consulted. It is preferable to use a t-butyldimethylsilyl group and carry out the deprotection with tetrabutylammonium fluoride. The compound (11-3) may be obtained also by reacting the compound (1-11) with a compound (11-7) to obtain a compound (11-6) previously, followed by reductive amination and deprotection.
The above-mentioned compound represented by the formula (11-3) may be prepared, for example, from a compound represented by the formula (1-11) also by the process shown in reaction scheme-16.

### Reaction Scheme-16

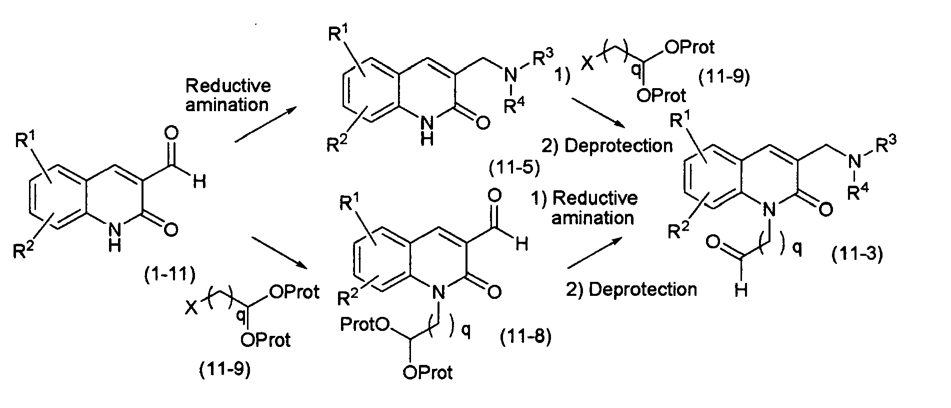

wherein R¹, R², R³, R⁴, X, q and Prot are as defined above.
The compound (11-3) may be prepared by reacting a compound (11-5) obtained from the compound (1-11) by the same process as in the above reaction scheme-1 with a compound (11-9) by the same method as in the above reaction scheme-6, followed by deprotection. As to the kind of Prot and the deprotection, a protective group and a deprotection method may be employed which are well known to those skilled in the art. For example, "T. W. Green et al., Protective Groups in Organic Synthesis, John Wiley & Sons, Inc., 1991" or the like may be consulted. It is preferable to use 2-bromomethyl-1,3-dioxolane and carry out the deprotection by heating in a mixture of 1,4-dioxane, water and sulfuric acid. The compound (11-3) may be obtained also by reacting the compound (1-11) with a compound (11-9) to obtain a compound (11-8) previously, followed by reductive amination and deprotection.
The reactions described above may be carried out according to the methods described in the working examples in the present description and R.C. Larock, Comprehensive Organic Transformation, VCH Publishers Inc. (1989), Jikken Kagaku Koza (Experimental Chemistry), Fourth Edition, Maruzen Co., Ltd., Shin Jikken Kagaku Koza (New Experimental Chemistry), Maruzen Co., Ltd., and the like.
As the starting compound used in each of the production processes described above, a commercial compound may be used, or the starting compound may be properly prepared by a process well known to those skilled in the art.
When the compound or pharmaceutically acceptable salt thereof of the present invention is prepared, a functional group such as a hydroxyl group, carboxyl group or amino group may be protected or deprotected in any step according to need. A protective group and a protection or deprotection method may be employed which are well known to those skilled in the art. For example, "T. W. Green et al., Protective Groups in Organic Synthesis, John Wiley & Sons, Inc., 1991" or the like may be consulted.

If necessary, the compound represented by the general formula (1) may be converted to a pharmaceutically acceptable addition salt with an inorganic acid or an organic acid or a pharmaceutically acceptable alkali addition salt. Such an acid addition salt includes, for example, inorganic acid salts such as hydrochloride, hydrobromide, sulfate, phosphate, etc.; salts with organic carboxylic acids, such as formic acid salt, acetic acid salt, fumaric acid salt, maleic acid salt, oxalic acid salt, citric acid salt, malic acid salt, tartaric acid salt, aspartic acid salt, glutamic acid salt, etc.; and salts with sulfonic acids, such as methanesulfonic acid salt, benzenesulfonic acid salt, p-toluenesulfonic acid salt, hydroxybenzenesulfonic acid salt, dihydroxybenzenesulfonic acid salt, etc. The pharmacologically acceptable alkali addition salt includes ammonium salt, lithium salt, sodium salt, potassium salt, calcium salt, magnesium salt, etc.
The present invention also includes hydrates and solvates (e.g. ethanol solvate) of the compound represented by the general formula (1) or the pharmaceutically acceptable salt thereof. In addition, the present invention includes all of the tautomers and stereoisomers (e.g. optical isomers) of the compound represented by the general formula (1) and all crystal forms of said compound. They may be properly purified by a method such as silica gel column chromatography, HPLC, ion-exchange chromatography, recrystallization or the like, which are well known to those skilled in the art.
In order to obtain the above-mentioned optical isomer in a pure form, an optical resolution method well known to those skilled in the art may be adopted. Specifically, when the compound of the present invention or an intermediate thereof has a basic functional group, it is possible to form a salt of the compound or intermediate with an optically active acid [for example, a monocarboxylic acid (e.g. mandelic acid, N-benzyloxyalanine or lactic acid), a dicarboxylic acid (e.g. tartaric acid, o-diisopropylidenetartaric acid or malic acid) or a sulfonic acid (e.g. camphorsulfonic acid or bromocamphorsulfonic acid)] in an inert solvent. When the compound of the present invention or an intermediate thereof has an acidic substituent, it is possible to form a salt of the compound or intermediate with an optically active amine (for example, an organic amine such as α-phenethylamine, quinine, quinidine, cinchonidine, cinchonine or strychnine). The temperature at the formation of the salt ranges from room temperature to the boiling point of a solvent.

The compound having a 2-quinolone skeleton or pharmaceutically acceptable salt thereof of the present invention has inhibitory activity against SNS and is usable as a therapeutic or prophylactic agent for neuropathic pain and nociceptive pain. As the neuropathic pain referred to here, there are exemplified lumbar postoperative neuralgia, diabetic neurosis, postherpetic neuralgia, reflex sympathetic pain, phantom limb pain, the pain of spinal cord damage, the pain of full-blown cancer, and prolonged postoperative pain. As the nociceptive pain, there are exemplified lumbago, bellyache, and pains due to rheumatoid arthritis and arthritis deformans. The compound of the present invention or the pharmaceutically acceptable salt thereof is usable also as a therapeutic or prophylactic agent for urinary disturbance. As the urinary disturbance referred to here, there are exemplified urinary frequency and cystalgia due to benign prostatic hypertrophy. Furthermore, said compound or pharmaceutically acceptable salt thereof is usable also as a therapeutic or prophylactic agent for inhibiting the abnormal nerve discharge of cerebellum in multiple sclerosis. As agents having no side effect due to nonnervous tissues and central nervous system, compounds having selective inhibitory activity against SNS are more preferable.

The pharmaceutical composition for treatment or prophylaxis of neuropathic pain, nociceptive pain, urinary disturbance or multiple sclerosis of the present invention may be incorporated with various ingredients for compounding, such as a carrier, binder, stabilizer, excipient, diluent, pH buffer, disintegrating agent, solubilizer, dissolution aidding agent, tonicity agent and the like, which are pharmaceutically acceptable and ordinary. The pharmaceutical composition for the treatment or prophylaxis may be orally or parenterally administered. That is, it may be orally administered in a usual dosage form such as tablets, pills, powder, granules, capsules, syrup, emulsion, suspension or the like. When parenterally administered, the pharmaceutical composition may be prepared in the form of an intravenous injection (a drip), intramuscular injection, subcutaneous injection, liniment, ophthalmic solution, ophthalmic ointment or the like.
A solid pharmaceutical composition such as tablets is prepared by blending the active ingredient with, for example, the following ordinary pharmacologically acceptable additives: a carrier or excipient, such as lactose, sucrose, corn starch or the like; a binder such as hydroxypropyl cellulose, poly(vinylpyrrolidone), hydroxypropylmethyl cellulose or the like; a disintegrating agent such as sodium carboxymethyl cellulose, starch sodium glycolate or the like; a lubricant such as stearic acid, magnesium stearate or the like; and a preservative.
In the case of the parenteral administration, the active ingredient is dissolved or suspended in a physiologically acceptable carrier such as water, physiological saline, oil, an aqueous glucose solution or the like, and the solution or suspension may contain an emulsifier, a stabilizer, a salt for adjusting osmotic pressure, or a buffer as a coadjuvant according to need.

Although the dose and the number of administrations are varied depending on administration route, the age, body weight and condition of a patient, and the like, a method is preferable in which the pharmaceutical composition is locally administered to the diseased part. The composition is preferably administered once or more a day. When administered twice or more, the composition is preferably repeatedly administered every day or at proper intervals.
As to the dose, the pharmaceutical composition may be administered to an adult patient in a dose of tens micrograms to 2 g, preferably 1 to hundreds milligrams, more preferably tens milligrams or less, in terms of the active ingredient, per administration and may be administered in one portion or several portions a day. When parenterally administered, the pharmaceutical composition is administered to an adult patient in a dose of, for example, 0.1 to 100 mg/day, preferably 0.3 to 50 mg/day, and may be administered in one portion or several portions a day. A sustained-release preparation may be used in order to reduce the number of administrations.
The pharmaceutical composition for treatment or prophylaxis of neuropathic pain, nociceptive pain, urinary disturbance or multiple sclerosis of the present invention may be utilized as a drug for animal.

### EXAMPLES

The present invention is illustrated below in further detail with working examples and a test example, but the working examples should not be construed as limiting the technical scope of the invention.

### Reference Example 1

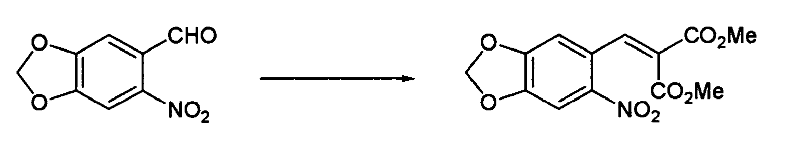

Under a nitrogen atmosphere, piperidine (0.77 ml, 7.76 mmol) and acetic acid (0.22 ml, 3.88 mmol) were added to a solution of 6-nitropiperonal (2 g, 6.47 mmol) and dimethyl malonate (1.1 g, 7.76 mmol) in tetrahydrofuran (30 ml) at room temperature and then stirred with heating at 80°C. After 24 hours, the reaction solution was allowed to cool. The reaction solution was poured into a saturated aqueous sodium chloride solution (100 ml) and extracted with ethyl acetate (50 ml). The organic layer was washed with a saturated aqueous sodium chloride solution (50 ml), dried over sodium sulfate and then concentrated under reduced pressure. The residue was purified by a column chromatography (hexane : ethyl acetate = 2 : 1) to obtain the desired compound (1.26 g, 63%).
¹H-NMR(CDCl₃) δ 8.13(d, 1H), 7.69(s, 1H), 6.79(s, 1H), 6.18(s, 2H), 3.88(s, 3H), 3.70(s, 3H).

### Reference Example 2

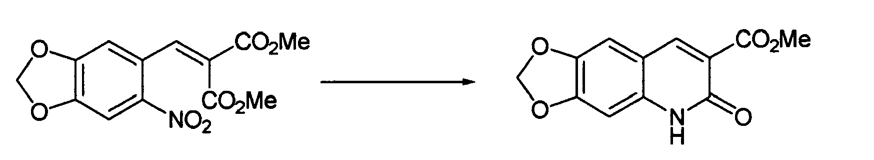

A solution of the compound (1.26 g, 4.07 mmol) obtained in Reference Example 1 in acetic acid (20 ml) was slowly added dropwise to a suspension of iron powder (796 mg, 14.26 mmol) in acetic acid (20 ml) at 80°C. The resulting mixture was stirred with heating for 2 hours and then allowed to cool at room temperature. Chloroform (50 ml) was added to the reaction mixture, followed by filtration through Celite. A 1N aqueous hydrochloric acid solution (100ml) was added to the filtrate, followed by extraction with chloroform. The organic layer was washed with a saturated aqueous sodium chloride solution (50 ml), dried over sodium sulfate and then concentrated under reduced pressure. The resulting residue was washed with ethyl acetate (10 ml) and collected by filtration using a Kiriyama funnel, to obtain the desired compound (644 mg, 64%).
¹H-NMR(DMSO-d6) δ 12.0(brs, 1H), 8.42(s, 1H), 7.31(s, 1H), 6.78(s, 1H), 6.12(s, 2H), 3.75(s, 3H).

### Reference Example 3

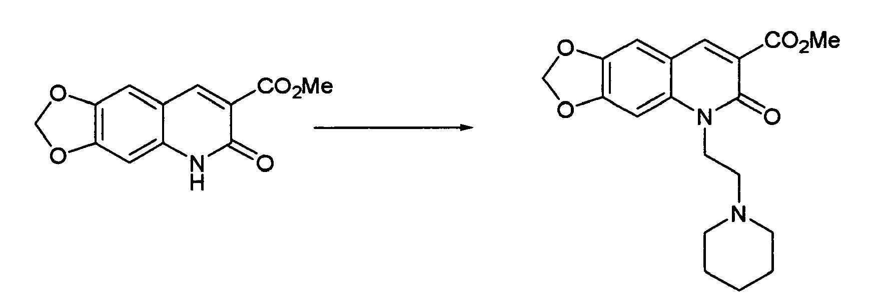

Under a nitrogen atmosphere, potassium carbonate (134 mg, 0.97 mmol), sodium iodide (a catalytic amount) and 1-(2-chloroethyl)piperidine hydrochloride (110 mg, 0.60 mmol) were added to a solution of the compound (100 mg, 0.405 mmol) obtained in Reference Example 2 in N,N'-dimethylformamide (5 ml) at room temperature, and the resulting mixture was heated to 80°C. After 4 hours, the reaction solution was allowed to cool to room temperature, poured into water (50 ml) and then extracted with ethyl acetate (50 ml). The organic layer was washed with a saturated aqueous sodium chloride solution (50 ml), dried over sodium sulfate and then concentrated under reduced pressure. The residue was purified by a column chromatography (chloroform : methanol = 10 : 1) to obtain the desired compound (86 mg, 59%).
¹H-NMR(CDCl₃) δ 8.38(s, 1H), 7.00(s, 2H), 6.11(s, 2H), 4.41(t, 2H, J=7.5Hz), 4.00(s, 3H), 2.63(t, 2H, J=7.5Hz), 2.62-2.55(m, 4H), 1.63-1.58(m, 4H), 1.49-1.40 (m, 2H).

### Reference Example 4

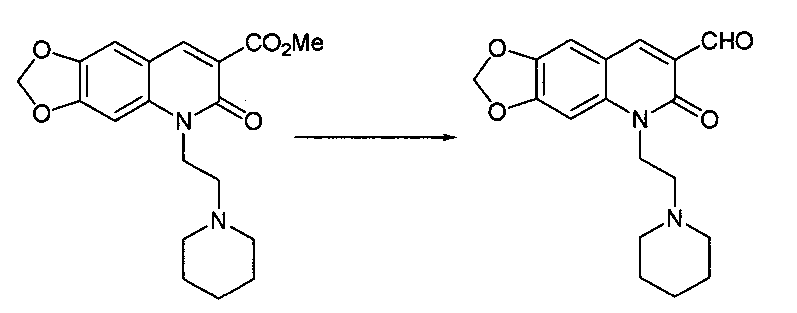

Under a nitrogen atmosphere, diisobutylaluminum hydride (0.95M in n-hexane, 0.52 ml) was slowly added dropwise to a solution (5 ml) of the compound (89 mg, 0.248 mmol) obtained in Reference Example 3 in dichloromethane at -78°C. After 15 minutes, a saturated aqueous potassium sodium tartrate solution (30 ml) and chloroform (30 ml) were added to the reaction solution and the resulting mixture was vigorously stirred at room temperature for 1 hour. This mixture was extracted with chloroform and the organic layer was washed with a saturated aqueous sodium chloride solution (30 ml), dried over sodium sulfate and then concentrated under reduced pressure. The residue was purified by a preparative chromatography (chloroform : methanol = 10 : 1) to obtain the desired compound (43 mg, 52%).
¹H-NMR (CDCl₃) δ 10.4(s, 1H), 8.22(s, 1H), 7.03(s, 2H), 6.12(s, 2H), 4.43(t, 2H, J=7.5Hz), 2.64(t, 2H, J=7.5Hz), 2.60-2.49(m, 4H), 1.66-1.55(m, 4H), 1.50-1.42 (m, 2H).

### Example 1

### 7-{[(2-Fluorobenzyl)amino]methyl}-5-(2-piperidin-1-ylethyl)[1,3]dioxolo[4,5-g]quinolin-6(5H)-one

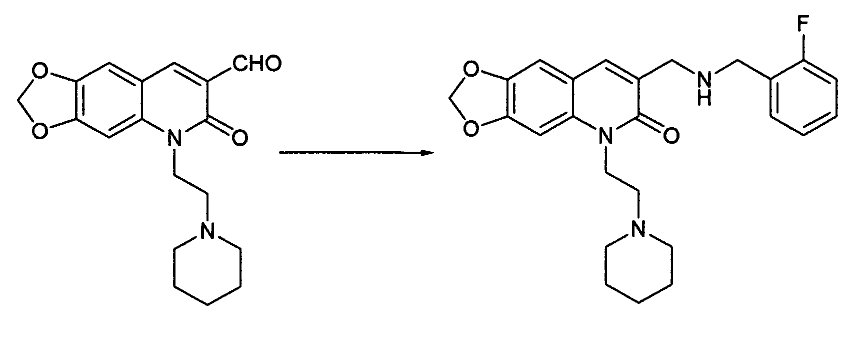

Under a nitrogen atmosphere, 2-fluorobenzylamine (0.022 ml, 0.194 mmol) and sodium triacetoxyborohydride (55 mg, 0.258 mmol) were added to a solution (5 ml) of the compound (43 mg, 0.129 mmol) obtained in Reference Example 4 in dichloromethane under ice-cooling. After 2 hours of stirring, a 2N aqueous sodium hydroxide solution (10 ml) was added to the reaction solution, followed by extraction with chloroform (20 ml). The organic layer was washed with a saturated aqueous sodium chloride solution (20 ml), dried over sodium sulfate and then concentrated under reduced pressure. The residue was purified by a preparative chromatography (chloroform : methanol = 5 : 1) to obtain the desired compound (24 mg, 42%).
¹H-NMR(DMSO-d6) δ 7.57(s, 1H), 7.43-7.39(m, 1H), 7.25-7.23(m, 1H), 7.22-7.19(m, 2H), 7.12-6.97(m, 2H), 6.92(s, 1H), 6.05(s, 2H), 4.39(t, 2H, J=8.0Hz), 3.89(s, 2H), 3.78(s, 2H), 2.60(t, 2H, J=8.0Hz), 2.68-2.48(m, 4H), 1.65-1.59(m, 4H), 1.51-1.42 (m, 2H).

### Example 2

### 5-(2-Piperidin-1-ylethyl)-7-({[2-(trifluoromethyl)benzyl]amino}methyl)[1,3]dioxolo[4,5-g]quinolin-6(5H)-one

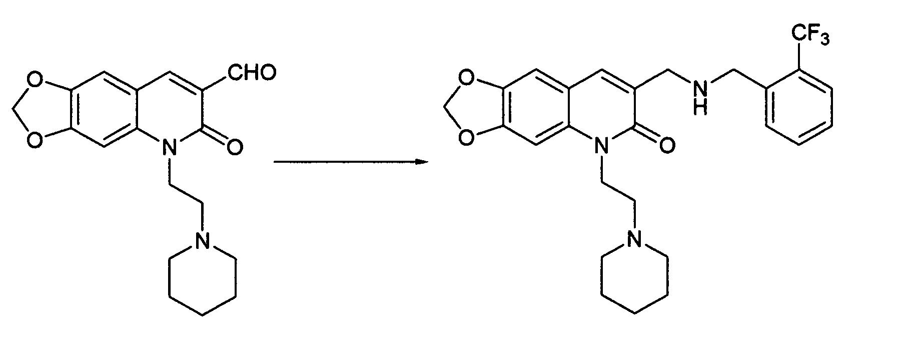

The desired compound (33 mg, 56%) was obtained by the same process as in Example 1.
¹H-NMR(DMSO-d6) δ 7.74(d, 1H, J=8.0Hz), 7.63(d, 1H, J=8.0Hz), 7.55(s, 1H), 7.54(dd, 2H, J=7.5Hz, 7.5Hz), 7.37(dd, 1H, J=7.5Hz, 7.5Hz), 7.27(s, 1H), 6.98(s, 1H), 6.92(s, 1H), 6.06(s, 2H), 4.41(t, 2H, J=8.0Hz), 4.00(s, 2H), 3.79(s, 2H), 2.62(t, 2H, J=8.0Hz), 2.60-2.50(m, 4H), 1.65-1.59(m, 4H), 1.50-1.42 (m, 2H).

### Reference Example 5

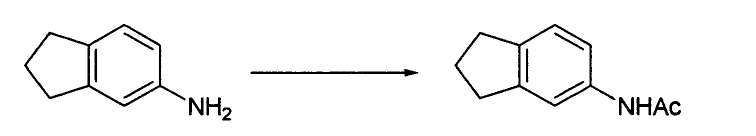

Pyridine (1.4 ml, 17.4 mmol) and acetic anhydride (1.65 ml, 17.4 mmol) were slowly added dropwise to a solution of 5-aminoindan (2.11 g, 15.8 mmol) in ethyl acetate (20 ml) under ice-cooling. The resulting mixture was stirred at the same temperature for 30 minutes, warmed to room temperature and then stirred. After one and a half hours, the reaction solution was poured into a saturated aqueous sodium hydrogencarbonate solution (50 ml) and extracted with ethyl acetate (20 ml). The organic layer was washed with a saturated aqueous sodium chloride solution (50 ml), dried over sodium sulfate and then concentrated under reduced pressure. Diethyl ether (20 ml) was added to the residue and stirred for 3 hours. The precipitate was collected by filtration using a Kiriyama funnel, to obtain the desired compound (1.97 g, 71%).
¹H-NMR(CDCl₃) δ 7.44(s, 1H), 7.14(s, 1H), 7.12(brs, 1H), 2.91-2.84(m, 4H), 2.16(s, 3H), 2.10-2.02(m, 2H).

### Reference Example 6

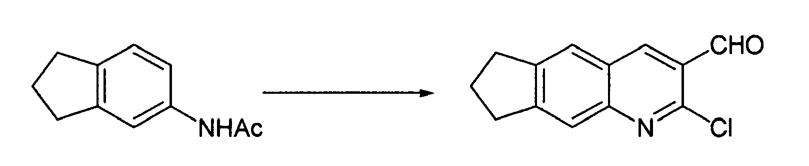

Phosphorus oxychloride (12 ml, 0.13 mol) was ice-cooled and N,N'-dimethylformamide (4.2 ml, 55 mmol) was slowly added dropwise thereto. After 5 minutes, the compound (3.4 g, 19 mmol) obtained in Reference Example 5 was added thereto in small portions. After 10 minutes, the reaction solution was heated to 80°C and stirred for 4 hours. The reaction solution was cooled and then slowly poured onto ice, followed by stirring at room temperature for 1 hour. The precipitate formed was filtered and then dried under reduced pressure to obtain a crude product (3.2 g). Ethyl acetate (30 ml) was added to the crude product and the resulting mixture was heated. After the black insoluble material was removed by filtration, the filtrate was stirred at room temperature and the crystals formed were filtered and then dried under reduced pressure to obtain the desired compound (2.1 g, 47%).
¹H-NMR(DMSO-d6) δ 2.12(m, 2H), 3.03-3.14(m, 4H), 7.85(s, 1H), 8.05(s, 1H), 8.86(s, 1H), 10.35(s, 1H).

### Reference Example 7

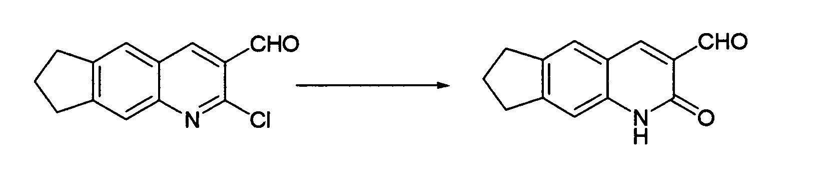

The compound (2.1 g, 9.0 mmol) obtained in Reference Example 6, acetic acid (20 ml) and water (2 ml) were mixed and then stirred at 120°C for 5 hours. After completion of the reaction, the reaction solution was cooled and then stirred overnight at room temperature. The crystals formed were filtered and then dried under reduced pressure to obtain the desired compound (1.5 g, 79%).
¹H-NMR(DMSO-d6) δ 2.04(m, 2H), 2.89(t, J=7.2Hz, 2H), 2.95(t, J=7.2Hz, 2H), 7.21(s, 1H), 7.71(s, 1H), 8.43(s, 1H), 10.22(s, 1H), 12.15(br.s, 1H).

### Reference Example 8

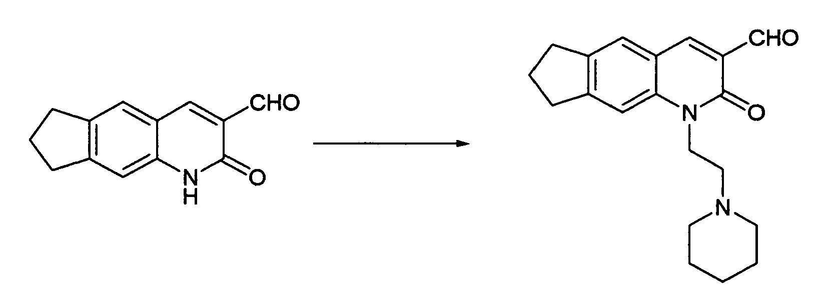

The compound (0.17 g, 0.80 mmol) obtained in Reference Example 7 was suspended in N,N'-dimethylformamide (7 ml) and sodium hydride (0.1 g, 55% in oil, 2.4 mmol) was added thereto under ice-cooling. After 15 minutes, 1-(2-chloroethyl)piperidine hydrochloride (0.22 g, 1.2 mmol) was added thereto. After 20 minutes, the reaction mixture was stirred at 80°C for 30 minutes. After the reaction was terminated by adding water to the reaction mixture, ethyl acetate was added thereto to effect separation and extraction. The organic layer was washed with water, dried and then concentrated, and the residue was purified by the use of a silica gel column (chloroform : methanol = 20 : 1 to 10 : 1) to obtain the desired compound (0.18 g, 70%) and its O-alkyl derivative (0.05 g, 19%).
¹H-NMR(CDCl₃) δ 1.43-1.52(m, 2H), 1.60-1.70(m, 4H), 2.17(m, 2H), 2.54-2.69(m, 6H), 2.99(t, J=7.2Hz, 2H), 3.07(t, J=7.2Hz, 2H), 4.48(m, 2H), 7.37(s, 1H), 7.52(s, 1H), 8.32(s, 1H), 10.46(s, 1H).

### Example 3

### 3-{[(2-Furylmethyl)(methyl)amino]methyl}-1-(2-piperidin-1-ylethyl)-1,6,7,8-tetrahydro-2H-cyclopenta[g]quinolin-2-one

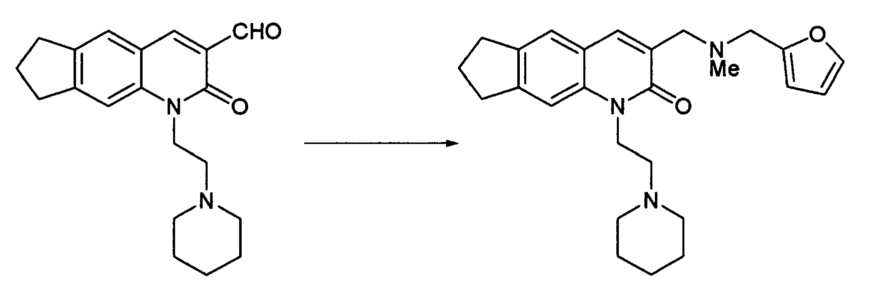

The compound (0.14 g, 0.43 mmol) obtained in Reference Example 8 was dissolved in methylene chloride (10 ml), followed by adding thereto methylamine (30% in ethanol, 0.09 ml, 0.86 mmol) and sodium triacetoxyborohydride (0.36 g, 1.72 mmol) under ice-cooling. After 2 hours, water, chloroform and a saturated aqueous sodium hydrogencarbonate solution were added to the reaction solution to effect separation and extraction. The organic layer was dried and then concentrated to obtain a methylamino compound (0.14 g). In methylene chloride (2 ml) was dissolved 0.04 g of the methylamino compound, and furfural (0.02 ml, 0.24 mmol) and sodium triacetoxyborohydride (0.10 g, 0.47 mmol) were added thereto with cooling and stirred for 2 hours. After 1 hour of stirring at room temperature, water, chloroform and a saturated aqueous sodium hydrogencarbonate solution were added to the reaction solution to effect separation and extraction. The organic layer was dried and then concentrated. The residue was purified by the use of a silica gel column (chloroform : methanol = 10 : 1) to obtain the desired compound (0.03 g, 66% (2 steps)).
¹H-NMR(CDCl₃) δ 1.43-1.52(br.s, 2H), 1.60-1.68(m, 4H), 2.14(m, 2H), 2.34(s, 3H), 2.54-2.68(m, 6H), 2.97(t, J=7.2Hz, 2H), 3.04(t, J=7.2Hz, 2H), 3.61(s, 2H), 3.66 (s, 2H), 4.48(m, 2H), 6.24(d, J=3.0Hz, 1H), 6.32(m, 1H), 7.33(s, 1H), 7.39(m, 1H), 7.41(s, 1H), 7.82(s, 1H).

### Example 4

### 3-{[[1-(2-Furyl)ethyl](methyl)amino]methyl}-1-(2-piperidin-1-ylethyl)-1,6,7,8-tetrahydro-2H-cyclopenta[g]quinolin-2-one

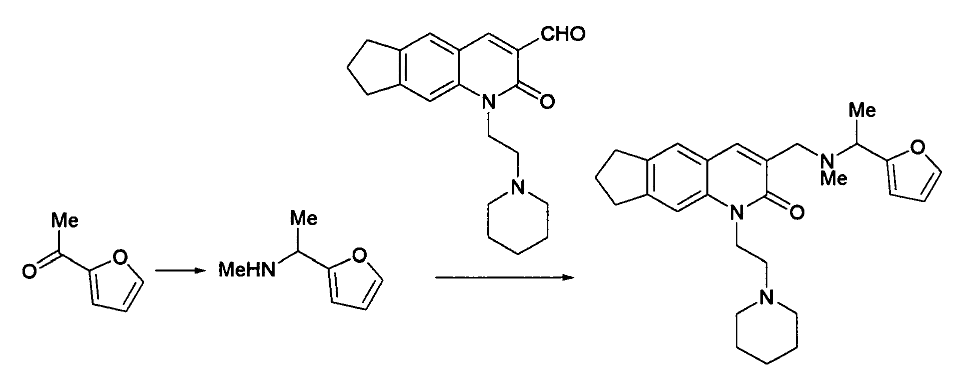

2-Acetylfuran (0.5 g, 4.5 mmol) was dissolved in tetrahydrofuran (15 ml), and methylamine (0.7 ml, 30% in methanol, 6.8 mmol) and sodium triacetoxyborohydride (2.9 g, 13.5 mmol) were added thereto at room temperature and stirred for 9 hours. Water, ethyl acetate and 1N hydrochloric acid were added to the reaction solution to effect separation and extraction. The aqueous layer was made basic with a 1N aqueous sodium hydroxide solution and chloroform was added thereto to effect separation and extraction. The organic layer was dried and then concentrated to obtain a methylamino compound (0.12 g).
The compound (80 mg, 0.25 mmol) obtained in Reference Example 8 was dissolved in tetrahydrofuran (5 ml) and the amine (60 mg, 0.48 mmol) obtained by the above reaction and sodium triacetoxyborohydride (150 mg, 0.71 mmol) were added thereto at room temperature and stirred for 3.5 hours. Water, chloroform and a 1N aqueous sodium hydroxide solution were added to the reaction solution to effect separation and extraction. The organic layer was dried and then concentrated and the residue was purified by the use of a silica gel column (chloroform : methanol = 20 : 1) to obtain the desired compound (50 mg, 47%).
¹H-NMR(CDCl₃) δ 1.43-1.52(m, 2H), 1.45(d, J=7.0Hz, 3H), 1.59-1.70(m, 4H), 2.14(m, 2H), 2.26(s, 3H), 2.54-2.67(m, 6H), 2.96(t, J=7.2Hz, 2H), 3.03(t, J=7.2Hz, 2H), 3.50(d, J=16.5Hz, 1H), 3.67(d, J=16.5Hz, 1H), 4.01(quart, J=7.0Hz, 1H), 4.46(m, 2H), 6.19(d, J=3.2Hz, 1H), 6.31(dd, J=3.2, 1.8Hz, 1H), 7.32(s, 1H), 7.38(m, 1H), 7.41(s, 1H), 7.81(s, 1H).

### Example 5

### 3-{[(2-Fluorobenzyl)(methyl)amino]methyl}-1-(2-piperidin-1-ylethyl)-1,6,7,8-tetrahydro-2H-cyclopenta[g]quinolin-2-one

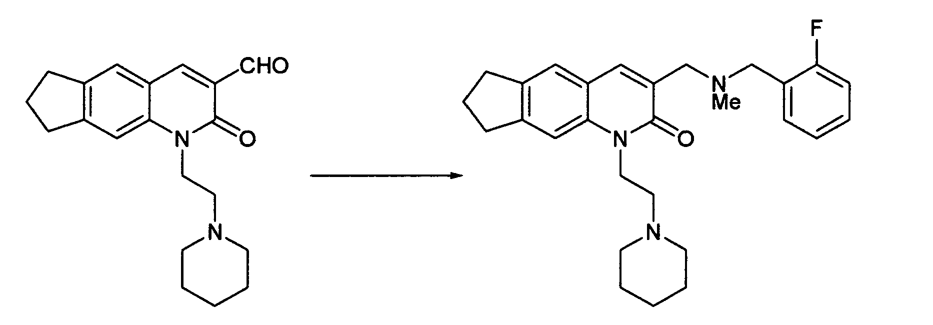

The desired compound was obtained by the same process as in Example 3.
¹H-NMR(CDCl₃) δ 1.42-1.52(m, 2H), 1.60-1.68(m, 4H), 2.14(m, 2H), 2.31(s, 3H), 2.53-2.68(m, 6H), 2.94-3.07(m, 4H), 3.63(s, 2H), 3.69(s, 2H), 4.47(m, 2H), 7.00-7.14(m, 2H), 7.23(m, 1H), 7.32(s, 1H), 7.42(s, 1H), 7.46(m, 1H), 7.86(s, 1H).

### Example 6

### 3-({Methyl[2-(trifluoromethyl)benzyl]amino}-methyl)-1-(2-piperidin-1-ylethyl)-1,6,7,8-tetrahydro-2H-cyclopenta[g]quinolin-2-one

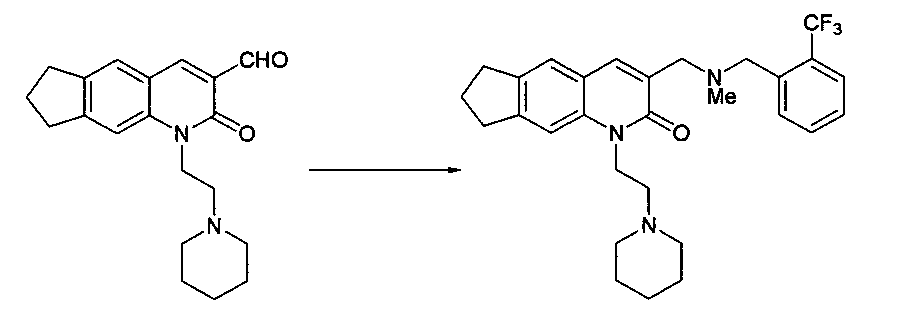

The desired compound was obtained by the same process as in Example 3.
¹H-NMR(CDCl₃) δ 1.42-1.52(m, 2H), 1.59-1.68(m, 4H), 2.14(m, 2H), 2.32(s, 3H), 2.53-2.67(m, 6H), 2.93-3.08(m, 4H), 3.62(s, 2H), 3.82(s, 2H), 4.47(m, 2H), 7.29-7.34(m, 2H), 7.41(s, 1H), 7.52(m, 1H), 7.61(d, J=7.8Hz, 1H), 7.82(s, 1H), 7.92(d, J=7.8Hz, 1H).

### Example 7

### 3-({Methyl[2-(trifluoromethoxy)benzyl]amino}-methyl)-1-(2-piperidin-1-ylethyl)-1,6,7,8-tetrahydro-2H-cyclopenta[g]quinolin-2-one

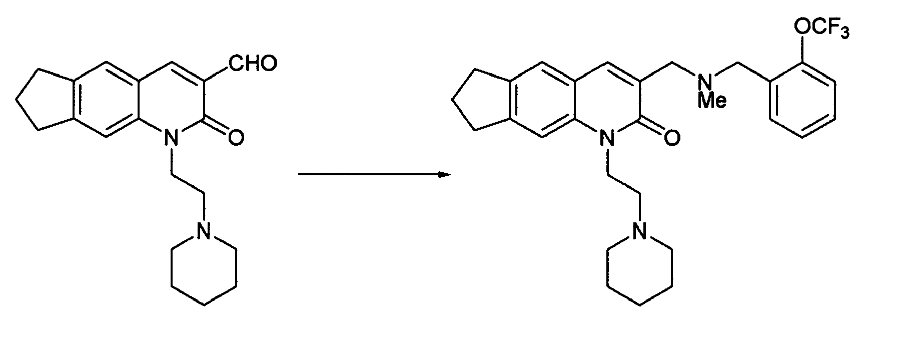

The desired compound was obtained by the same process as in Example 3.
¹H-NMR(CDCl₃) δ 1.42-1.52(m, 2H), 1.60-1.68(m, 4H), 2.14(m, 2H), 2.30(s, 3H), 2.54-2.66(m, 6H), 2.94-3.07(m, 4H), 3.61(s, 2H), 3.71(s, 2H), 4.47(m, 2H), 7.18-7.29(m, 3H), 7.32(s, 1H), 7.40(s, 1H), 7.65(m, 1H), 7.83(s, 1H).
The following compounds of Example 8 to Example 27 were prepared by the same processes as in the above working examples.
These compounds were identified by their retention time in high performance liquid chromatography and LC/MS spectra. The analysis conditions are described below.
<LC-ESIMS analysis conditions>
Instrument used: Waters ZQ (two modes, i.e., positive and
negative modes, MS detection), Waters 600 (HPLC), Waters 996 (Photodiode Array)
Column: MIGHTYSIL RP-18 (4.6 mm x 50 mm, GP 5 µm, Kanto Chemical Co., Ltd.)
Detection wavelength: 260 nm
Flow rate: 3.5 ml/ml
Composition of eluent under acidic conditions: A; 0.5% HCOOH aqueous solution, B; MeOH
Composition of eluent under neutral conditions: A; 10 mM NH₄OAc aqueous solution, B; MeOH
Gradient (B concentration): 10%(0 min) → 99%(3.5 min) → 99%(5.5 min)

**[Table 1]**

| | | | | | | | |
|---|---|---|---|---|---|---|---|
| 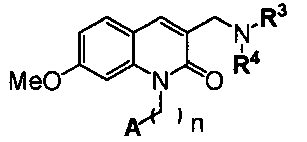 | | | | | | | |

| No. | n | 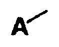 | 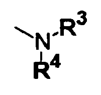 | Exact Mass | Found value Mass (m/e) | Retention time (min) | Conditions |
|---|---|---|---|---|---|---|---|
| 8 | 1 | 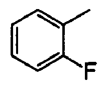 | 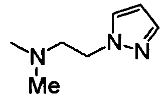 | 420 | 421 | 3.17 | Acidic |

**[Table 2]**

| | | | | | | | |
|---|---|---|---|---|---|---|---|
| 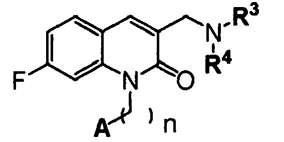 | | | | | | | |

| No. | n | 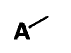 | 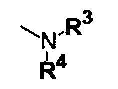 | Exact Mass | Found value Mass (m/e) | Retention time | Conditions |
|---|---|---|---|---|---|---|---|
| 9 | 1 | 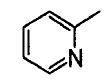 | 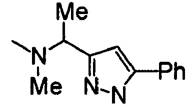 | 467 | 468 | 3.05 | Acidic |
| 10 | 1 | 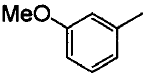 | 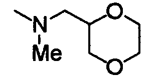 | 426 | 427 | 3.01 | Acidic |
| 11 | 2 | 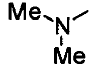 | 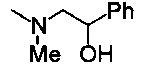 | 397 | 398 | 3.88 | Neutral |
| 12 | 2 | 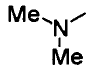 | 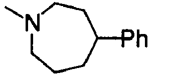 | 421 | 422 | 4.12 | Neutral |
| 13 | 2 | 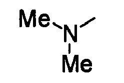 | 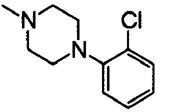 | 442 | 443 | 4.63 | Neutral |
| 14 | 2 | 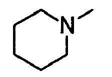 | 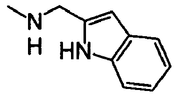 | 432 | 433 | 4.41 | Neutral |
| 15 | 2 | 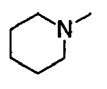 | 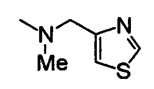 | 414 | 415 | 3.87 | Neutral |
| 16 | 2 | 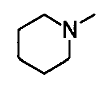 | 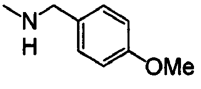 | 423 | 424 | 4.01 | Neutral |
| 17 | 2 | 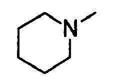 | 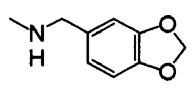 | 437 | 438 | 4.05 | Neutral |

**[Table 3]**

| | | | | | | | |
|---|---|---|---|---|---|---|---|
| 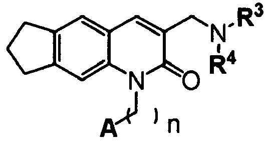 | | | | | | | |

| No. | n | 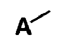 | 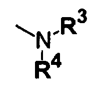 | Exact Mass | Found value Mass (m/e) | Retention time (min) | Conditions |
|---|---|---|---|---|---|---|---|
| 18 | 1 | 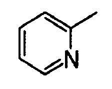 | 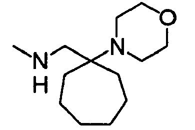 | 500 | 501 | 4.21 | Neutral |
| 19 | 1 | 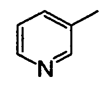 | 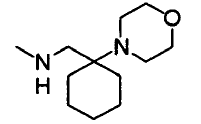 | 486 | 487 | 3.95 | Neutral |
| 20 | 1 | 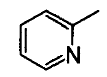 | 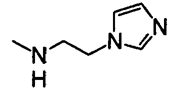 | 399 | 400 | 2.32 | Acidic |
| 21 | 1 | 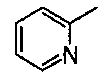 | 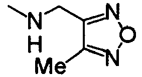 | 401 | 402 | 2.93 | Acidic |
| 22 | 1 | 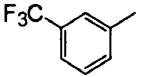 | 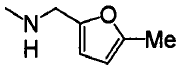 | 466 | 467 | 3.70 | Acidic |
| 23 | 1 | 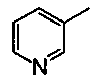 | 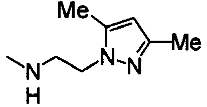 | 427 | 428 | 2.70 | Acidic |

**[Table 4]**

| | | | | | | | |
|---|---|---|---|---|---|---|---|
| 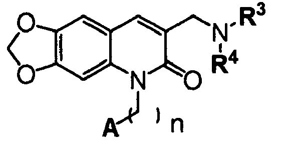 | | | | | | | |

| No. | n | 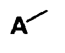 | 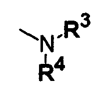 | Exact Mass | Found value Mass (m/e) | Retention time (min) | Conditions |
|---|---|---|---|---|---|---|---|
| 24 | 1 | 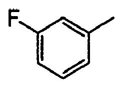 | 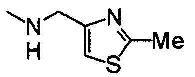 | 437 | 438 | 3.09 | Acidic |
| 25 | 1 | 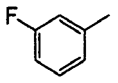 | 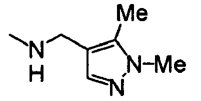 | 434 | 435 | 2.95 | Acidic |
| 26 | 2 | 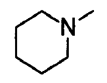 | 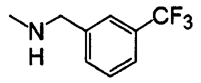 | 487 | 488 | 4.49 | Neutral |
| 27 | 2 | 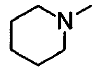 | 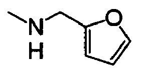 | 409 | 410 | 3.68 | Neutral |

### Reference Example 9

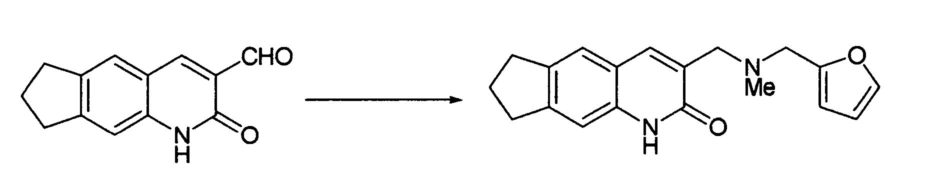

From the compound obtained in Reference Example 7, the desired compound was obtained by the same process as in Example 3.
¹H-NMR (CDCl₃) δ 2.12(m, 2H), 2.37(s, 3H), 2.93-3.02(m, 4H), 3.65(s, 2H), 3.69(s, 2H), 6.27(d, J=3.1Hz, 1H), 6.33(dd, J=3.1, 1.8Hz, 1H), 7.13(s, 1H), 7.39-7.43(m, 2H), 7.90(s, 1H), 10.54(s, 1H).

### Example 28

### 3-{[(2-Furylmethyl)(methyl)amino]methyl}-1-(2-pyrrolidin-1-ylethyl)-1,6,7,8-tetrahydro-2H-cyclopenta[g]quinolin-2-one

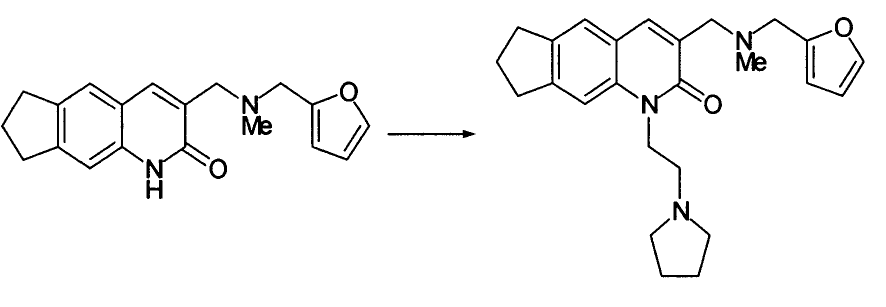

From the compound obtained in Reference Example 9, the desired compound was obtained by the same process as in Reference Example 8.
¹H-NMR (CDCl₃) δ 1.80-1.88(m, 4H), 2.14(m, 2H), 2.68-2.75(m, 4H), 2.80(m, 2H), 2.97(t, J=7.4Hz, 2H), 3.04(t, J=7.4Hz, 2H), 3.61(s, 2H), 3.67(s, 2H), 4.49(m, 2H), 6.24(br.d, J=3.1Hz, 1H), 6.32(dd, J=3.1, 1.8Hz, 1H), 7.33(s, 1H), 7.39(dd, J=1.8, 0.8Hz, 1H), 7.41(s, 1H), 7.82(s, 1H).
The following compounds of Example 29 to Example 61 were prepared by the same processes as in Reference Examples 5, 6 and 7 and Example 28.

**[Table 5]**

| No. | Structural formula | ¹H-NMR (CDCl₃) δ |
|---|---|---|
| 29 | 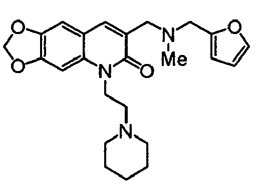 | 1.47-1.50(m, 2H), 1.62-1.67(m, 4H), 2.33(s, 3H), 2.60(m, 4H), 2.64(t, J=7.8Hz, 2H), 3.58(m, 2H), 3.65(s, 2H), 4.43(t, J=7.8Hz, 2H), 6.05(s, 2H), 6.23(d, J=3.1Hz, 1H), 6.31(dd, J=3.1, 1.8Hz, 1H), 6.96(s, 1H), 7.01(s, 1H), 7.39(m, 1H), 7.74(s, 1H) |
| 30 | 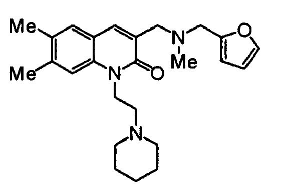 | 1.44-1.52(m, 2H), 1.60-1.70(m, 4H), 2.32(s, 3H), 2.33(s, 3H), 2.40(s, 3H), 2.55-2.67(m, 6H), 3.60(s, 2H), 3.67 (s, 2H), 4.46(m, 2H), 6.24(dd, J=3.1, 0.8Hz, 1H), 6.32(dd, J=3.1, 1.8Hz, 1H), 7.23 (s, 1H), 7.33(s, 1H), 7.39(dd, J=1.8, 0.8Hz, 1H), 7.79(s, 1H) |
| 31 | 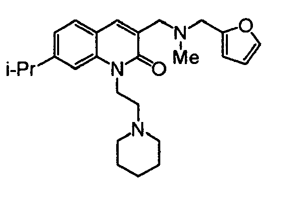 | 1.32(d, J=6.9Hz, 6H), 1.44-1.52(m, 2H), 1.60-1.70(m, 4H), 2.34(s, 3H), 2.55-2.69(m, 6H), 3.05(m, 1H), 3.61(s, 2H), 3.67(s, 2H), 4.49(m, 2H), 6.24(dd, J=3.1, 0.8Hz, 1H), 6.32(dd, J=3.1, 1.8Hz, 1H), 7.12(dd, J=8.0, 1.2Hz, 1H), 7,29(br.s, 1H), 7.39(dd, J=1.8, 0.8Hz, 1H), 7.53(d, J=8.0Hz, 1H), 7.84(s, 1H) |
| 32 | 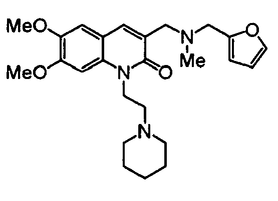 | 1.43-1.52(m, 2H), 1.58-1.68(m, 4H), 2.34(s, 3H), 2.53-2.70(m, 6H), 3.60(s, 2H), 3.68(s, 2H), 3.93(s, 3H), 4.00(s, 3H), 4.47(m, 2H), 6.25(dd, J=3.1, 0.8Hz, 1H), 6.32(dd, J=3.1, 1.8Hz, 1H), 6.97(br.s, 1H), 7.00(s, 1H), 7.39(dd, J=1.8, 0.8Hz, 1H), 7.78(s, 1H) |
| 33 | 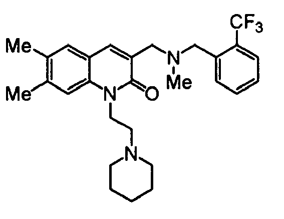 | 1.43-1.52(m, 2H), 1.56-1.68(m, 4H), 2.31(s, 3H), 2.32(s, 3H), 2.40(s, 3H), 2.55-2.65(m, 6H), 3.61(s, 2H), 3.82(s, 2H), 4.45(m, 2H), 7.23(s, 1H), 7.32(br.t, J=7.7Hz, 1H), 7.33(s, 1H), 7.52(br.t, J=7.8Hz, 1H), 7.61(br.d, J=7.7Hz, 1H), 7.80(s, 1H), 7.92(br.d, J=7.8Hz, 1H) |

**[Table 6]**

| No. | Structural formula | ¹H-NMR (CDCl₃) δ |
|---|---|---|
| 34 | 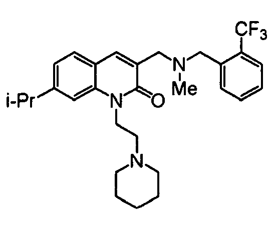 | 1.32(d, J=6.9Hz, 6H), 1.44-1.52(m, 2H), 1.60-1.67(m, 4H), 2.32(s, 3H), 2.55-2.68(m, 6H), 3.04(m, 1H), 3.62(s, 2H), 3.82 (s, 2H), 4.49(m, 2H), 7.12(dd, J=8.0, 1.2Hz, 1H), 7.28-7.35 (m, 2H), 7.49-7.55(m, 2H), 7.61(brd, J=7.8Hz, 1H), 7.84(s, 1H), 7.91(brd, J=7.8Hz, 1H) |
| 35 | 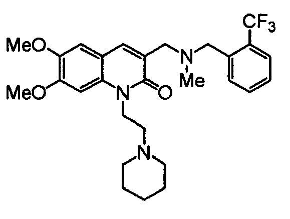 | 1.40-1.52(m, 2H), 1.56-1.65(m, 4H), 2.32(s, 3H), 2.53-2.68(m, 6H), 3.61(s, 2H), 3.83(s, 2H), 3.95(s, 3H), 4.00(s, 3H), 4.47 (m, 2H), 6.98(s, 1H), 6.99(s, 1H), 7.32(br.t, J=7.7Hz, 1H), 7.53(br.t, J=7.7Hz, 1H), 7.62(br.d, J=7.7Hz, 1H), 7.78 (s, 1H), 7.91(br.d, J=7.7Hz, 1H) |
| 36 | 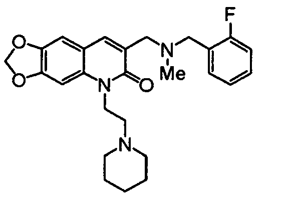 | 1.48-1.49(m, 2H), 1.60-1.68(m, 4H), 2.30(s, 3H), 2.62(m, 4H), 2.66(t, J=7.9Hz, 2H), 3.60(s, 2H), 3.69(s, 2H), 4.44(t, J=7.9Hz, 2H), 6.05(s, 2H), 6.96(s, 1H), 7.01-7.05(m, 2H), 7.05-7.12(m, 1H), 7.20-7.25(m, 1H), 7.42-7.46(m, 1H), 7.78(s, 1H) |
| 37 | 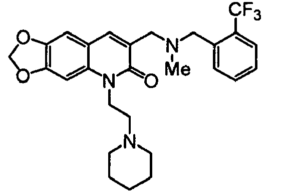 | 1.39-1.41(m, 2H), 1.54-1.60(m, 4H), 2.24(s, 3H), 2.52-2.58(m, 6H), 3.53(s, 2H), 3.61(s, 2H), 4.36(m, 2H), 5.96(s, 2H), 6.89 (s, 1H), 6.93-6.98(m, 2H), 7.01-7.05(m, 1H), 7.13-7.18(m, 1H), 7.35-7.39(m, 1H), 7.71(s, 1H) |
| 38 | 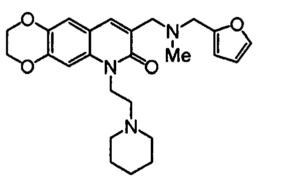 | 1.42-1.50(m, 2H), 1.58-1.68(m, 4H), 2.33(s, 3H), 2.53-2.65(m, 6H), 3.58(s, 2H), 3.65(s, 2H), 4.27-4.41(m, 6H), 6.24(br.d, J=3.1Hz, 1H), 6.31(dd, J=3.1, 1.9Hz, 1H), 6.94(s, 1H), 7.06(s, 1H), 7.39(br.s, 1H), 7,71(s, 1H) |

**[Table 7]**

| No. | Structural formula | ¹H-NMR (CDCl₃) δ |
|---|---|---|
| 39 | 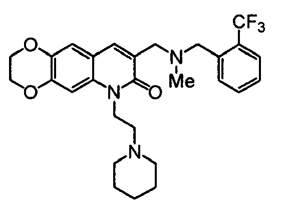 | 1.42-1.50(m, 2H), 1.58-1.66(m, 4H), 2.31(s, 3H), 2.53-2.64(m, 6H), 3.59(s, 2H), 3.80(s, 2H), 4.26-4.41 (m, 6H), 6.94 (s, 1H), 7.05(s, 1H), 7.31(br.t, J=7.7Hz, 1H), 7.51(br.t, J=7.7Hz, 1H), 7.61(br.d, J=7.7Hz, 1H), 7.71(s, 1H), 7.90(br.d, J=7.7Hz, 1H) |
| 40 | 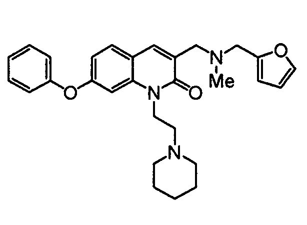 | 1.37-1.46(m, 2H), 1.52-1.60(m, 4H), 2.34(s, 3H), 2.40-2.47(m, 4H), 2.55(m, 2H), 3.59(s, 2H), 3.67(s, 2H), 4.31(m, 2H), 6.24(br.d, J=3.1Hz, 1H), 6.32(dd, J=3.1, 1.8Hz, 1H), 6.89(dd, J=8.6, 2.1Hz, 1H), 6.96(d, J=2.1Hz, 1H), 7.07-7.12(m, 2H), 7.19(m, 1H), 7.37-7.42(m, 3H), 7.53(d, J=8.6Hz, 1H), 7.83(s, 1H) |
| 41 | 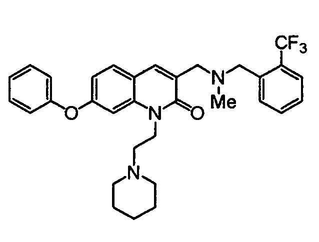 | 1.37-1.46(m, 2H), 1.51-1.58(m, 4H), 2.32(s, 3H), 2.40-2.47(m, 4H), 2.54(m, 2H), 3.60(s, 2H), 3.82(s, 2H), 4.31(m, 2H), 6.89(dd, J=8.6, 2.1Hz, 1H), 6.96(br.d, J=2.1Hz, 1H), 7.07-7.11(m, 2H), 7.19(m, 1H), 7.32(br.t, J=7.8Hz, 1H), 7.37-7.42(m, 2H), 7.52(br.t, J=7.8Hz, 1H), 7.53(d, J=8.6Hz, 1H), 7.62(br.d, J=7.8Hz, 1H), 7.82(s, 1H), 7.90(br.d, J=7.8Hz, 1H) |
| 42 | 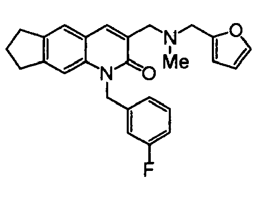 | 2.09(m, 2H), 2.38(s, 3H), 2.90-2.98(m, 4H), 3.67(s, 2H), 3.70(s, 2H), 5.55(br.s, 2H), 6.26(br.d, J=3.1Hz, 1H), 6.33(dd, J=3.1, 1.8Hz, 1H), 6.86(m, 1H), 6.91(m, 1H), 7.00(m, 1H), 7.05(s, 1H), 7.26(m, 1H), 7.40(dd, J=1.8, 0.8Hz, 1H), 7.43(s, 1H), 7.90(s, 1H) |

**[Table 8]**

| No. | Structural formula | ¹H-NMR (CDCl₃) δ |
|---|---|---|
| 43 | 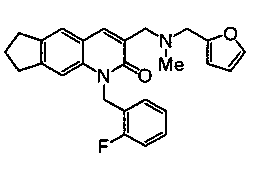 | 2.08(m, 2H), 2.38(s, 3H), 2.90-2.96(m, 4H), 3.68(s, 2H), 3.70(s, 2H), 5.61(s, 2H), 6.27(br.d, J=3.1Hz, 1H), 6.33(dd, J=3.1, 1.8Hz, 1H), 6.89(m, 1H), 6.97(m, 1H), 7.08(s, 1H), 7.10(m, 1H), 7.21(m, 1H), 7.41(dd, J=1.8, 0.8Hz, 1H), 7.43(s, 1H), 7.90(s, 1H) |
| 44 | 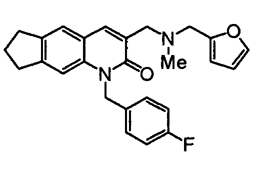 | 2.09(m, 2H), 2.37(s, 3H), 2.90-2.97(m, 4H), 3.67(s, 2H), 3.69(s, 2H), 5.52(br.s, 2H), 6.26(br.d, J=3.1Hz, 1H), 6.33(dd, J=3.1, 1.8Hz, 1H), 6.94-7.01(m, 2H), 7.09(s, 1H), 7.16-7.22(m, 2H), 7.40(dd, J=1.8, 0.8Hz, 1H), 7.43 (s, 1H), 7.89(s, 1H) |
| 45 | 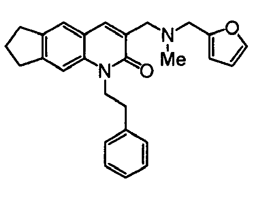 | 2.16(m, 2H), 2.35(s, 3H), 2.95-3.09(m, 6H), 3.63(s, 2H), 3.68(s, 2H), 4.50(m, 2H), 6.26(br.d, J=3.1Hz, 1H), 6.33(dd, J=3.1, 1.8Hz, 1H), 7.23-7.28(m, 2H), 7.32-7.39(m, 4H), 7.40(dd, J=1.8, 0.8Hz, 1H), 7.44(s, 1H), 7.84(s, 1H) |
| 46 | 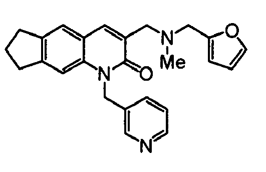 | 2.09(m, 2H), 2.38(s, 3H), 2.90-2.96(m, 4H), 3.66(s, 2H), 3.70(s, 2H), 5.57(br.s, 2H), 6.26(br.d, J=3.1Hz, 1H), 6.33(dd, J=3.1, 1.8Hz, 1H), 7.08(s, 1H), 7.21(m, 1H), 7.40(dd, J=1.8, 0.8Hz, 1H), 7.43(s, 1H), 7.49(m, 1H), 7.90(s, 1H), 8.49(dd, J=4.8, 1.6Hz, 1H), 8.59(br.d, J=1.6Hz, 1H) |
| 47 | 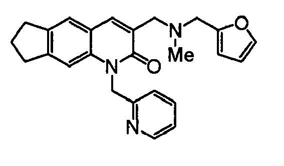 | 2.08(m, 2H), 2.38(s, 3H), 2.88-2.95(m, 4H), 3.68(s, 2H), 3.70(s, 2H), 5.68(br.s, 2H), 6.27(br.d, J=3.1Hz, 1H), 6.33(dd, J=3.1, 1.8Hz, 1H), 7.06(br.d, J=7.7Hz, 1H), 7.16(m, 1H), 7.25(s, 1H), 7.40-7.43(m, 2H), 7.54(td, J=7.7, 1.8Hz, 1H), 7.90(s, 1H), 8.59(m, 1H) |

**[Table 9]**

| No. | Structural formula | ¹H-NMR (CDCl₃) δ |
|---|---|---|
| 48 | 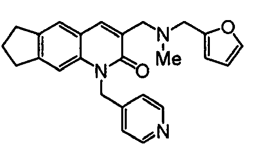 | 2.09(m, 2H), 2.38(s, 3H), 2.88-2.98(m, 4H), 3.66(s, 2H), 3.70(s, 2H), 5.56(br.s, 2H), 6.26(br.d, J=3.1Hz, 1H), 6.33(dd, J=3.1, 1.8Hz, 1H), 6.94(s, 1H), 7.09(m, 2H), 7.41(dd, J=1.8, 0.8Hz, 1H), 7.45(s, 1H), 7.93(s, 1H), 8.51(m, 2H) |
| 49 | 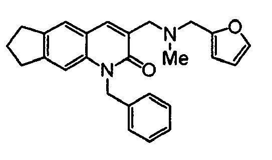 | 2.07(m, 2H), 2.38(s, 3H), 2.88-2.96(m, 4H), 3.68(s, 2H), 3.70(s, 2H), 5.56(br.s, 2H), 6.26(br.d, J=3.1Hz, 1H), 6.33(dd, J=3.1, 1.8Hz, 1H), 7.12(s, 1H), 7.18-7.32(m, 5H), 7.40(dd, J=1.8, 0.8Hz, 1H), 7.42(s, 1H), 7.90(s, 1H) |
| 50 | 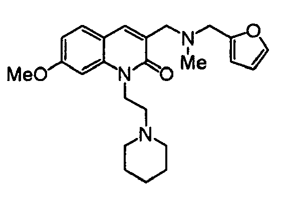 | 1.42-1.50(m, 2H), 1.59-1.65(m, 4H), 2.34(s, 3H), 2.53-2.67(m, 6H), 3.59(s, 2H), 3.67(s, 2H), 3.91(s, 3H), 4.44 (m, 2H), 6.24 (br.d, J=3.1Hz, 1H), 6.32(dd, J=3.1, 1.8Hz, 1H), 6.82(dd, J=8.7, 2.1Hz, 1H), 6.93(br.d, J=2.1Hz, 1H), 7.39(m, 1H), 7.49(d, J=8.7Hz, 1H), 7.79(s, 1H) |
| 51 | 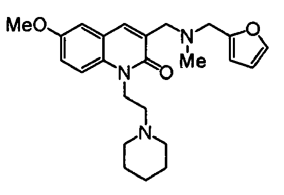 | 1.42-1.50(m, 2H), 1.58-1.65(m, 4H), 2.35(s, 3H), 2.53-2.67(m, 6H), 3.62(s, 2H), 3.68(s, 2H), 3.87(s, 3H), 4.45(m, 2H), 6.25(br.d, J=3.1Hz, 1H), 6.32(dd, J=3.1, 1.8Hz, 1H), 7.05(d, J=2.9Hz, 1H), 7.15(dd, J=9.3, 2.9Hz, 1H), 7.38(d, J=9.3Hz, 1H), 7.39(dd, J=1.8, 0.8Hz, 1H), 7.83(s, 1H) |
| 52 | 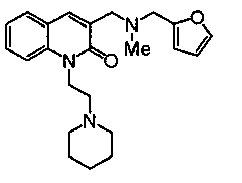 | 1.42-1.50(m, 2H), 1.60-1.67(m, 4H), 2.35(s, 3H), 2.55-2.67(m, 6H), 3.62(s, 2H), 3.68(s, 2H), 4.48(m, 2H), 6.25(br.d, J=3.1Hz, 1H), 6.32(dd, J=3.1, 1.8Hz, 1H), 7.22(m, 1H), 7.39(m, 1H), 7.44(br.d, J=8.5Hz, 1H), 7.53(m, 1H), 7.60(dd, J=7.8, 1.3Hz, 1H), 7.88(m, 1H) |

**[Table 10]**

| No. | Structural formula | ¹H-NMR (CDCl₃) δ |
|---|---|---|
| 53 | 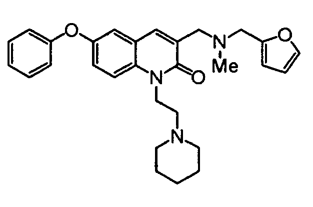 | 1.42-1.50(m, 2H), 1.60-1.67(m, 4H), 2.33(s, 3H), 2.54-2.67(m, 6H), 3.61(s, 2H), 3.66(s, 2H), 4.46(m, 2H), 6.23(br.d, J=3.1Hz, 1H), 6.30(dd, J=3.1, 1.8Hz, 1H), 7.01-7.05(m, 2H), 7.13(m, 1H), 7.20(br.d, J=2.7Hz, 1H), 7.25(m, 1H), 7.34-7.39(m, 3H), 7.43(d, J=9.2Hz, 1H), 7.78(s, 1H) |
| 54 | 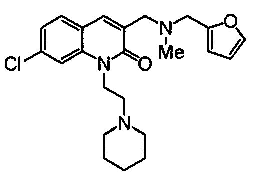 | 1.42-1.50(m, 2H), 1.58-1.66(m, 4H), 2.35(s, 2H), 2.52-2.64(m, 6H), 3.60(s, 2H), 3.66(s, 2H), 4.40(m, 2H), 6.24(br.d, J=3.1Hz, 1H), 6.31(dd, J=3.1, 1.8Hz, 1H), 7.18(dd, J=8.4, 1.8Hz, 1H), 7.39(dd, J=1.8, 0.8Hz, 1H), 7.44(br.d, J=1.8Hz, 1H), 7.51 (d, J=8.4Hz, 1H), 7.84(s, 1H) |
| 55 | 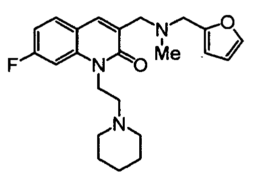 | 1.42-1.50(m, 2H), 1.58-1.66(m, 4H), 2.35(s, 3H), 2.52-2.65(m, 6H), 3.59(s, 2H), 3.67(s, 2H), 4.39(m, 2H), 6.24(br.d, J=3.1Hz, 1H), 6.32(dd, J=3.1, 1.8Hz, 1H), 6.95(td, J=8.6, 2.2Hz, 1H), 7.16(dd, J=11.3, 2.2Hz, 1H), 7.39(br.s, 1H), 7.56(dd, J=8.6, 6.3Hz, 1H), 7.84(s, 1H) |
| 56 | 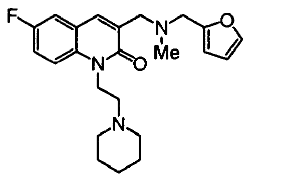 | 1.42-1.50(m, 2H), 1.58-1.66(m, 4H), 2.35(s, 3H), 2.52-2.65(m, 6H), 3.62(s, 2H), 3.67(s, 2H), 4.45(m, 2H), 6.24(br.d, J=3.1Hz, 1H), 6.32(dd, J=3.1, 1.8Hz, 1H), 7.22-7.30(m, 2H), 7.38-7.44(m, 2H), 7.83(s, 1H) |
| 57 | 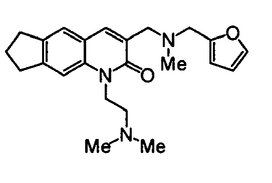 | 2.14(m, 2H), 2.34(s, 3H), 2.40(s, 6H), 2.62(m, 2H), 2.97(t, J=7.4Hz, 2H), 3.04(t, J=7.4Hz, 2H), 3.61(s, 2H), 3.67(s, 2H), 4.44(m, 2H), 6.24(br.d, J=3.1Hz, 1H), 6.32(dd, J=3.1, 1.8Hz, 1H), 7.27(s, 1H), 7.39(dd, J=1.8, 0.8Hz, 1H), 7.41(s, 1H), 7.82(s, 1H) |

**[Table 11]**

| No. | Structural formula | ¹H-NMR (CDCl₃) δ |
|---|---|---|
| 58 | 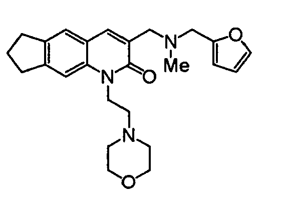 | 2.15(m, 2H), 2.34(s, 3H), 2.60-2.72(m, 6H), 2.98(t, J=7.4Hz, 2H), 3.04(t, J=7.4Hz, 2H), 3.61(s, 2H), 3.67(s, 2H), 3.72-3.78(m, 4H), 4.47(m, 2H), 6.25(br.d, J=3.1Hz, 1H), 6.32(dd, J=3.1, 1.8Hz, 1H), 7.27(s, 1H), 7.40(dd, J=1.8, 0.8Hz, 1H), 7.42(s, 1H), 7.82(s, 1H) |
| 59 | 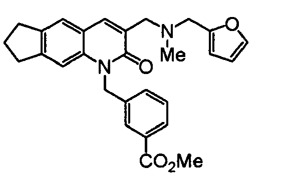 | 2.07(m, 2H), 2.38(s, 3H), 2.88-2.96(m, 4H), 3.68(s, 2H), 3.70(s, 2H), 3.88(s, 3H), 5.60(br.s, 2H), 6.27(br.d, J=3.1Hz, 1H), 6.33(dd, J=3.1, 1.8Hz, 1H), 7.04(s, 1H), 7.30-7.37(m, 2H), 7.41(dd, J=1.8, 0.8Hz, 1H), 7.43(s, 1H), 7.89-7.96 (m, 3H) |
| 60 | 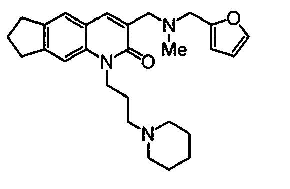 | 1.42-1.50(m, 2H), 1.57-1.67(m, 4H), 1.95(m, 2H), 2.14(m, 2H), 2.34(s, 3H), 2.40-2.52(m, 6H), 2.97(t, J=7.4Hz, 2H), 3.03(t, J=7.4Hz, 2H), 3.61(s, 2H), 3.66(s, 2H), 4.34(m, 2H), 6.25(d, J=3.1Hz, 1H), 6.32(dd, J=3.1, 2.0Hz, 1H), 7.35(s, 1H), 7.39-7.42(m, 2H), 7.81(s, 1H) |
| 61 | 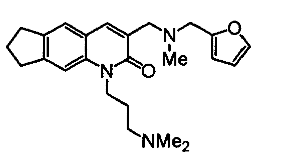 | 1.92(m, 2H), 2.14(m, 2H), 2.28(s, 6H), 2.34(s, 3H), 2.45(t, J=7.0Hz, 2H), 2.94-3.07(m, 4H), 3.62(s, 2H), 3.67(s, 2H), 4.35(m, 2H), 6.25(br.d, J=3.1Hz, 1H), 6.32(dd, J=3.1, 1.8Hz, 1H), 7.32(s, 1H), 7.40(m, 1H), 7.41(s, 1H), 7.82(s, 1H) |

### Reference Example 10

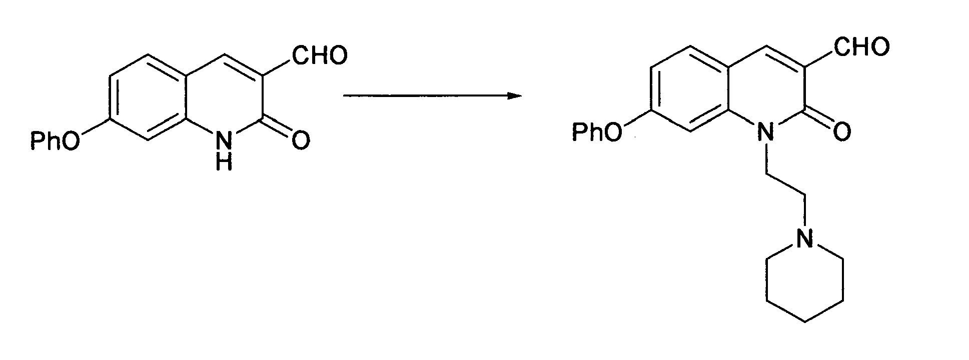

A 2-quinolone derivative (3.0 g, 11.3 mmol) obtained from 3-phenoxyaniline by the same processes as in Reference Examples 5, 6 and 7 was dissolved in N,N'-dimethylformamide (50 ml) and cesium carbonate (9.2 g, 28.3 mmol) and 1-(2-chloroethyl)piperidine hydrochloride (2.5 g, 13.6 mmol) were added thereto at room temperature. After the reaction solution was stirred at 80°C for 1.5 hours, water and ethyl acetate were added thereto to effect separation and extraction. The organic layer was washed with water, dried and then concentrated, and the residue was purified by the use of a silica gel column (chloroform : methanol = 50 : 1 to 20 : 1) to obtain the desired compound (2.9 g, 68%) and its O-alkyl derivative (0.45 g, 11%).
¹H-NMR (CDCl₃) δ 1.38-1.60(m, 6H), 2.37-2.45(m, 4H), 2.56(m, 2H), 4.30(m, 2H), 6.91-6.95(m, 2H), 7.11-7.16(m, 2H), 7.27(m, 1H), 7.42-7.48(m, 2H), 7.66(d, J=9.0Hz, 1H), 8.32(s, 1H), 10.43(s, 1H).

### Example 62

### N'-{[2-Oxo-7-phenoxy-1-(2-piperidin-1-ylethyl)-1,2-dihydroquinolin-3-yl]methyl}-L-alaninamide

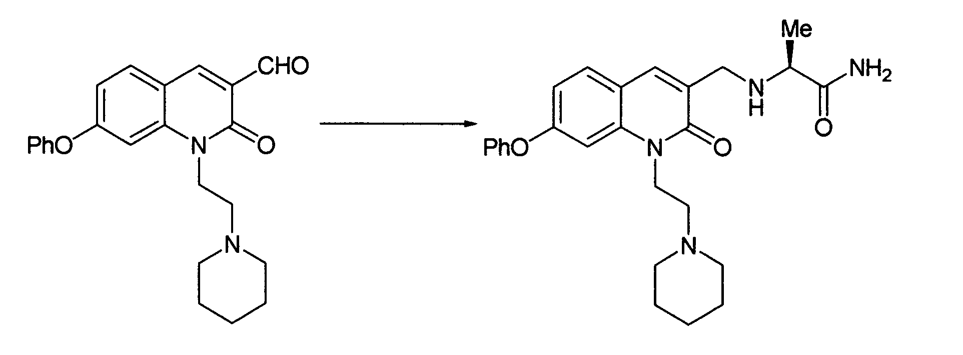

The compound (0.70 g, 1.86 mmol) obtained in Reference Example 10 was dissolved in dichloromethane, and (L)-alaninamide hydrochloride (0.30 g, 2.42 mmol) and sodium triacetoxyborohydride (0.51 g, 2.42 mmol) were added thereto under ice-cooling and stirred for 30 minutes. The reaction solution was warmed to room temperature and stirred overnight, and water and chloroform were added thereto to effect separation and extraction. The aqueous layer was made basic with a 1N aqueous sodium hydroxide solution and chloroform was added thereto to effect separation and extraction. The organic layer was dried and then concentrated and the residue was recrystallized from ethyl acetate-hexane to obtain the desired compound (0.56 g, 67%).
¹H-NMR (CDCl₃) δ 1.35(d, J=7.0Hz, 3H), 1.40-1.47(m, 2H), 1.53-1.61(m, 4H), 2.40-2.48(m, 4H), 2.57(t, J= 7.9Hz, 2H), 3.26(quart, J=7.0Hz, 1H), 3.62(d, J=13.3Hz, 1H), 3.85(d, J=13.3Hz, 1H), 4.24-4.41(m, 2H), 5.36(br.s, 1H), 6.92(dd, J=8.6, 2.2Hz, 1H), 6.99(br.d, J=2.2Hz, 1H), 7.09-7.14(m, 2H), 7.22(m, 1H), 7.40-7.46(m, 2H), 7.51(d, J=8.6Hz, 1H), 7.59(s, 1H), 7.78(br.s, 1H).
The following compounds of Examples 63 to 115 were prepared by the same processes as in Reference Examples 5, 6, 7 and 10 and Example 62.

**[Table 12]**

| No. | Structural formula | ¹H-NMR (CDCl₃) δ |
|---|---|---|
| 63 | 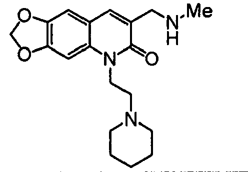 | 1.46-1.47(m, 2H), 1.57-1.63(m, 4H), 2.46(s, 3H), 2.56-2.63(m, 6H), 3.70(s, 2H), 4.40(m, 2H), 6.04(s, 2H), 6.92(s, 1H), 6.98(s, 1H), 7.53(s, 1H) |
| 64 | 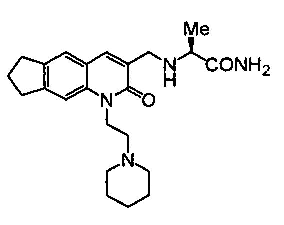 | 1.33(d, J=7.0Hz, 3H), 1.43-1.51(m, 2H), 1.58-1.66(m, 4H), 2.15(m, 2H), 2.53-2.66(m, 6H), 2.97(t, J=7.4Hz, 2H), 3.05(t, J=7.4Hz, 2H), 3.25(quart, J=7.0Hz, 1H), 3.62(d, J=13.0Hz, 1H), 3.84(d, J=13.0Hz, 1H), 4.39-4.55(m, 2H), 5.36(br.s, 1H), 7.34(s, 1H), 7.36(s, 1H), 7.56(s, 1H), 7.86(br.s, 1H) |
| 65 | 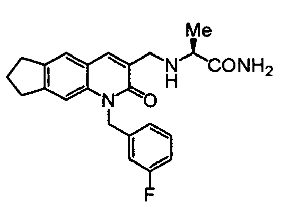 | 1.37(d, J=7.0Hz, 3H), 2.11(m, 2H), 2.92-3.00(m, 4H), 3.30(quart, J=7.0Hz, 1H), 3.69(d, J=13.1Hz, 1H), 3.93(d, J=13.1Hz, 1H), 5.41(br.s, 1H), 5.49(br.d, J=15.5Hz, 1H), 5.64(br.d, J=15.5Hz, 1H), 6.86(m, 1H), 6.95(m, 1H), 7.00(m, 1H), 7.06(s, 1H), 7.29(m, 1H), 7.39(s, 1H), 7. 66 (s, 1H), 7.86(br.s, 1H) |
| 66 | 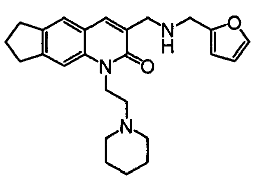 | 1.47-1.48(m, 2H), 1.61-1.66(m, 4H), 2.14(tt, J=7.4, 7.4Hz, 2H), 2.58-2.65(m, 6H), 2.96(t, J=7.4Hz, 2H), 3.04(t, J=7.4Hz, 2H), 3.79(s, 2H), 3.83(s, 2H), 4.44-4.48(m, 2H), 6.20-6.21(m, 1H), 6.29-6.30(m, 1H), 7.32(s, 1H), 7.35-7.36(m, 2H), 7.62(s, 1H) |
| 67 | 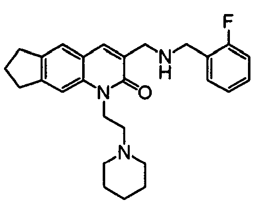 | 1.46-1.48(m, 2H), 1.61-1.66(m, 4H), 2.14(tt, J=7.3, 7.3Hz, 2H), 2.58-2.65(m, 6H), 2.96(t, J=7.3Hz, 2H), 3.03(t, J=7.3Hz, 2H), 3.80(s, 2H), 3.89(d, J=5.1Hz, 2H), 4.43-4.47(m, 2H), 6.99-7.05(m, 1H), 7.08-7.12(m, 1H), 7.19-7.24(m, 1H), 7.32(s, 1H), 7.37(s, 1H), 7.40-7.43(m, 1H), 7.65(s, 1H) |

**[Table 13]**

| No. | Structural formula | ¹H-NMR (CDCl₃) δ |
|---|---|---|
| 68 | 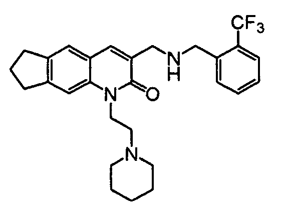 | 1.47-1.50(m, 2H), 1.61-1.67(m, 4H), 2.14(tt, J=7.4, 7.4Hz, 2H), 2.59-2.66(m, 6H), 2.97(t, J=7.4Hz, 2H), 3.04(t, J=7.4Hz, 2H), 3.82(s, 2H), 4.01(d, J=9.5Hz, 2H), 4.45-4.49(m, 2H), 7.32-7.37(m, 2H), 7.52-7.58(m, 2H), 7.62-7.65(m, 2H), 7.75(m, 1H) |
| 69 | 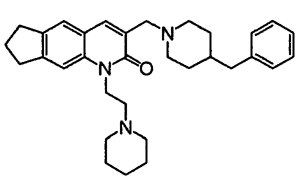 | 1.36-1.66(m, 11H), 2.05-2.10(m, 2H), 2.14(tt, J=7.4Hz, 2H), 2.55-2.65(m, 8H), 2.94-2.98(m, 4H), 3.04(t, J=7.4Hz, 2H), 3.54(s, 2H), 4.45-4.49(m, 2H), 7.14-7.20(m,, 3H), 7.25-7.29(m, 2H), 7.32(s, 1H), 7.40(s, 1H), 7.72(s, 1H) |
| 70 | 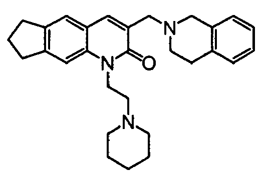 | 1.48-1.51(m, 2H), 1.63-1.69(m, 4H), 2.14(tt, J=7.4, 7.4Hz, 2H), 2.62-2.69(m, 6H), 2.86(t, J=6.1Hz, 2H), 2.94-2.98(m, 4H), 3.04(t, J=7.4Hz, 2H), 3.73(s, 2H), 3.77(s, 2H), 4.49-4.52(m, 2H), 7.00-7.02(m, 1H), 7.09-7.14(m, 3H), 7.35(s, 1H), 7.38(s, 1H), 7.82 (s, 1H) |
| 71 | 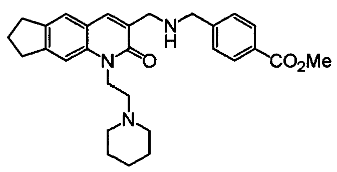 | 1.47-1.50(m, 2H), 1.61-1.67(m, 4H), 2.14(tt, J=7.0, 7.0Hz, 2H), 2.60-2.66(m, 6H), 2.97(t, J=7.0Hz, 2H), 3.04(t, J=7.0Hz, 2H), 3.78(s, 2H), 3.89(s, 2H), 3.91(s, 3H), 4.45-4.49(m, 2H), 7.33(s, 1H), 7.36(s, 1H), 7.43(d, J=8.2Hz, 2H), 7.59(s, 1H), 7.99(d, J=8.2Hz, 2H) |

**[Table 14]**

| No. | Structural formula | ¹H-NMR (CDCl₃) δ |
|---|---|---|
| 72 | 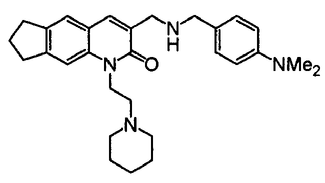 | 1.47-1.48(m, 2H), 1.62-1.67(m, 4H), 2.15(tt, J=7.3, 7.3Hz, 2H), 2.59-2.65(m, 6H), 2.92(s, 6H), 2.96(t, J=7.3Hz, 2H), 3.05(t, J=7.3Hz, 2H), 3.77(s, 2H), 3.80(s, 2H), 4.44-4.48(m, 2H), 6.71(d, J=8.6Hz, 2H), 7.23(d, J=8.6Hz, 2H), 7.32(s, 1H), 7.36(s, 1H), 7.63(s, 1H) |
| 73 | 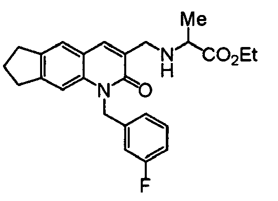 | 1.26(t, J=7.1Hz, 3H), 1.38(d, J=7.0Hz, 3H), 2.09(m, 2H), 2.89-2.97(m, 4H), 3.45(quart, J=7.0Hz, 1H), 3.78(d, J=14.7Hz, 1H), 3.85(d, J=14.7Hz, 1H), 4.14(m, 2H), 5.54(br.s, 2H), 6.85-6.95(m, 2H), 7.00(m, 1H), 7.04(s, 1H), 7.26(m, 1H), 7.39(s, 1H), 7.73(s, 1H) |
| 74 | 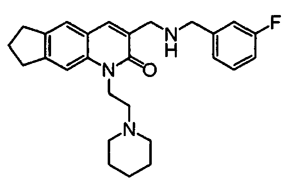 | 1.47-1.50(m, 2H), 1.61-1.66(m, 4H), 2.14(tt, J=7.4, 7.4Hz, 2H), 2.58(m, 4H), 2.61-2.65(m, 2H), 2.97(t, J=7.4Hz, 2H), 3.04(t, J=7.4Hz, 2H), 3.78(s, 2H), 3.83(s, 2H), 4.44-4.48(m, 2H), 6.90-6.97(m, 1H), 7.07-7.14(m, 2H), 7.24-7.38(m, 3H), 7.61(s, 1H) |
| 75 | 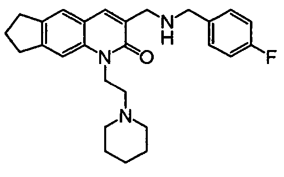 | 1.45-1.50(m, 2H), 1.60-1.67(m, 4H), 2.14(tt, J=7.3, 7.3Hz, 2H), 2.58-2.65 (m, 6H), 2.97 (t, J=7.3Hz, 2H), 3.04(t, J=7.3Hz, 2H), 3.77(s, 2H), 3.80(s, 2H), 4.46(t, J=7.9Hz, 2H), 6.97-7.03(m, 2H), 7.26-7.36(m, 4H), 7.60(s, 1H) |
| 76 | 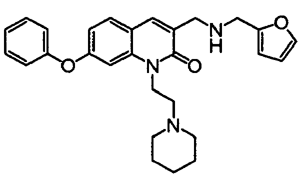 | 1.41-1.44(m, 2H), 1.52-1.58(m, 4H), 2.43(m, 4H), 2.53-2.56(m, 2H), 3.78(s, 2H), 3.83(s, 2H), 4.28-4.32(m, 2H), 6.21(m, 1H), 6.29-6.31(m, 1H), 6.88(dd, J=8.6, 2.0Hz, 1H), 6.96(d, J=2.0Hz, 1H), 7.08-7.11(m, 2H), 7.17-7.21(m, 1H), 7.35-7.36(m, 1H), 7.37-7.43(m, 2H), 7.49(d, J=8.6Hz, 1H), 7.64(s, 1H) |

**[Table 15]**

| No. | Structural formula | ¹H-NMR (CDCl₃) δ |
|---|---|---|
| 77 | 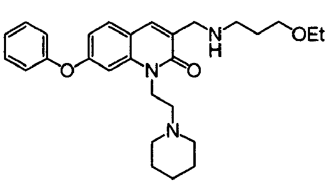 | 1.19(t, J=7.0Hz, 3H), 1.42-1.44(m, 2H), 1.50-1.58(m, 4H), 1.83(tt, J=6.3, 6.3Hz, 2H), 2.43(m, 4H), 2.53-2.57(m, 2H), 2.75(t, J=6.3Hz, 2H), 3.47(q, J=7.0Hz, 2H), 3.51(t, J=6.3Hz, 2H), 3.76(s, 2H), 4.29-4.32(m, 2H), 6.88(dd, J=8.5, 1.8Hz, 1H), 6.96(d, J=1.8Hz, 1H), 7.08-7.10(m, 2H), 7.18-7.21(m, 1H), 7.38-7.42(m, 2H), 7.49(d, J=8.5Hz, 1H), 7.64(s, 1H) |
| 78 | 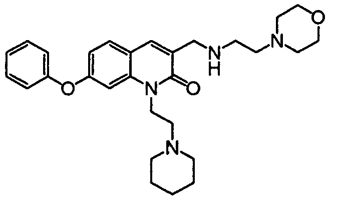 | 1.42-1.45(m, 2H), 1.51-1.58(m, 4H), 2.19(m, 4H), 2.43-2.48(m, 8H), 2.54(t, J=6.3Hz, 2H), 2.76(t, J=6.3Hz, 2H), 3.70-3.72(m, 2H), 3.78(s, 2H), 4.28-4.32(m, 2H), 6.89(dd, J=8.6, 2.0Hz, 1H), 6.96(d, J=2.0Hz, 1H), 7.08-7.11(m, 2H), 7.18-7.22(m, 1H), 7.38-7.43(m, 2H), 7.49(d, J=8.6Hz, 1H), 7.65(s, 1H) |
| 79 | 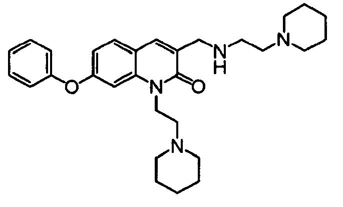 | 1.42-1.43(m, 4H), 1.53-1.61(m, 8H), 2.39-2.43(m, 8H), 2.50 (t, J=6.5Hz, 2H), 2.52-2.56(m, 2H), 2.77(t, J=6.5Hz, 2H), 3.78(s, 2H), 4.29-4.33(m, 2H), 6.88(dd, J=8.6, 2.0Hz, 1H), 6.96(d, J=2.0Hz, 1H), 7.08-7.11(m, 2H), 7.17-7.22(m, 1H), 7.38-7.43(m, 2H), 7.50(d, J=8.6Hz, 1H), 7.66(s, 1H) |

**[Table 16]**

| No. | Structural formula | ¹H-NMR (CDCl₃) δ |
|---|---|---|
| 80 | 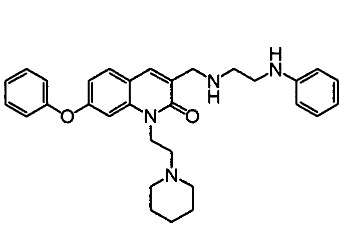 | 1.42-1.45(m, 2H), 1.54-1.59(m, 4H), 2.45(m, 4H), 2.53-2.57(m, 2H), 2.94(t, J=5.6Hz, 2H), 3.29(t, J=5.6Hz, 2H), 3.80(s, 2H), 4.29-4.33(m, 2H), 6.63-6.66(m, 2H), 6.67-6.72(m, 1H), 6.88(dd, J=8.6, 2.2Hz, 1H), 6.97(d, J=2.2Hz, 1H), 7.08-7.11(m, 2H), 7.14-7.22(m, 3H), 7.38-7.43(m, 2H), 7.46(d, J=8.6Hz, 1H), 7.61(s, 1H) |
| 81 | 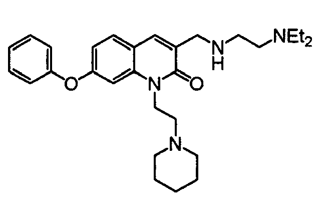 | 1.04(t, J=7.1Hz, 6H), 1.43-1.46(m, 2H), 1.54-1.60(m, 4H), 2.16(m, 4H), 2.45(m, 2H), 2.55(q, J=7.1Hz, 4H), 2.63 (t, J=6.7Hz, 2H), 2.75(t, J=6.7Hz, 2H), 3.80(s, 2H), 4.31-4.35(m, 2H), 6.90(dd, J=8.6, 2.1Hz, 1H), 6.98(d, J=2.1Hz, 1H), 7.10-7.13(m, 2H), 7.19-7.23(m, 1H), 7.40-7.45(m, 2H), 7.51(d, J=8.6Hz, 1H), 7.67(s, 1H) |
| 82 | 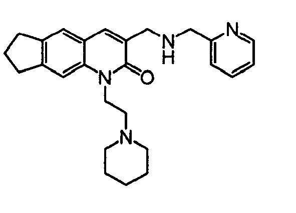 | 1.47-1.49(m, 2H), 1.61-1.66(m, 4H), 2.14(tt, J=7.4, 7.4Hz, 2H), 2.58(m, 4H), 2.61-2.65(m, 2H), 2.96(t, J=7.4Hz, 2H), 3.04(t, J=7.4Hz, 2H), 3.84(s, 2H), 3.98(s, 2H), 4.44-4.48(m, 2H), 7.14-7.17(m, 1H), 7.32(s, 1H), 7.37(s, 1H), 7.40(s, 1H), 7.63-7.66(m, 1H), 7.68(s, 1H), 8.54-8.55(m, 1H) |

**[Table 17]**

| No. | Structural formula | ¹H-NMR (CDCl₃) δ |
|---|---|---|
| 83 | 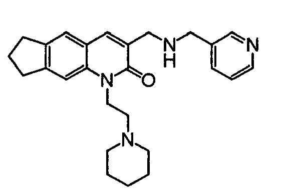 | 1.47-1.48(m, 2H), 1.61-1.67(m, 4H), 2.15(tt, J=7.4, 7.4Hz, 2H), 2.59(m, 4H), 2.61-2.65(m, 2H), 2.97(t, J=7.4Hz, 2H), 3.05(t, J=7.4Hz, 2H), 3.78(s, 2H), 3.84(s, 2H), 4.45-4.49(m, 2H), 7.25-7.28(m, 1H), 7.33(s, 1H), 7.37(s, 1H), 7.60(s, 1H), 7.74-7.75(m, 1H), 8.49-8.51(m, 1H), 8.57(s, 1H) |
| 84 | 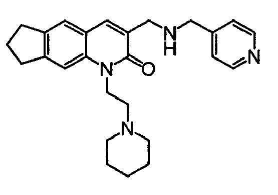 | 1.47-1.48(m, 2H), 1.61-1.66(m, 4H), 2.15(tt, J=7.4, 7.4Hz, 2H), 2.58(m, 4H), 2.63(m, 2H), 2.97(t, J=7.4Hz, 2H), 3.05(t, J=7.4Hz, 2H), 3.78(s, 2H), 3.84(s, 2H), 4.47(m, 2H), 7.30-7.33(m, 3H), 7.37(s, 1H), 7.59(s, 1H), 8.53-8.54(m, 2H) |
| 85 | 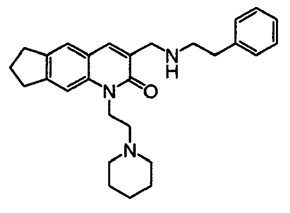 | 1.47-1.50(m, 2H), 1.61-1.67(m, 4H), 2.14(tt, J=7.4, 7.4Hz, 2H), 2.58-2.64(m, 6H), 2.83-2.94(m, 4H), 2.96(t, J=7.4Hz, 2H), 3.04(t, J=7.4Hz, 2H), 3.80(s, 2H), 4.43-4.47(m, 2H), 7.18-7.22(m, 3H), 7.27-7.31(m, 3H), 7.34(s, 1H), 7.56(s, 1H) |
| 86 | 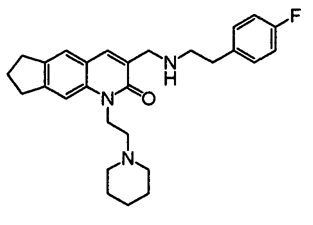 | 1.47-1.50(m, 2H), 1.61-1.67(m, 4H), 2.14(tt, J=7.4, 7.4Hz, 2H), 2.58-2.63(m, 6H), 2.79-2.90(m, 4H), 2.96(t, J=7.4Hz, 2H), 3.04(t, J=7.4Hz, 2H), 3.78(s, 2H), 4.43-4.47(m, 2H), 6.93-6.99(m, 2H), 7.13-7.18(m, 2H), 7.31(s, 1H), 7.34(s, 1H), 7.55(s, 1H) |

**[Table 18]**

| No. | Structural formula | ¹H-NMR (CDCl₃) δ |
|---|---|---|
| 87 | 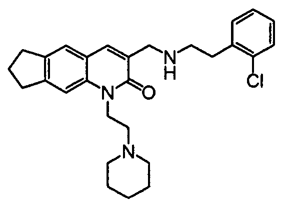 | 1.47-1.50(m, 2H), 1.61-1.69(m, 4H), 2.14(tt, J=7.3, 7.3Hz, 2H), 2.58-2.64(m, 6H), 2.88-3.01(m, 6H), 3.04(t, J=7.3Hz, 2H), 3.82(s, 2H), 4.44-4.48(m, 2H), 7.12-7.20(m, 2H), 7.24-7.27(m, 1H), 7.28-7.34(m, 3H), 7.59(s, 1H) |
| 88 | 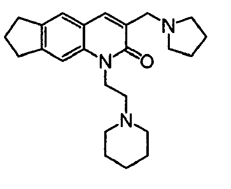 | 1.47-1.50(m, 2H), 1.61-1.67(m, 4H), 1.80-1.84(m, 4H), 2.14(tt, J=7.4, 7.4Hz, 2H), 2.59-2.65(m, 10H), 2.96(t, J=7.4Hz, 2H), 3.04(t, J=7.4Hz, 2H), 3.68(s, 2H), 4.45-4.49(m, 2H), 7.32(s, 1H), 7.39(s, 1H), 7.72(s, 1H) |
| 89 | 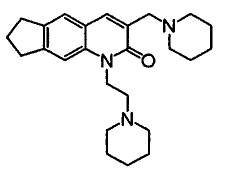 | 1.47(m, 4H), 1.63-1.65(m, 8H), 2.14(tt, J=7.2, 7.2Hz, 2H), 2.50(m, 4H), 2.58(m, 4H), 2.61-2.69(m, 2H), 2.96(t, J=7.2Hz, 2H), 3.03(t, J=7.2Hz, 2H), 3.51(s, 2H), 4.45-4.49(m, 2H), 7.31(s, 1H), 7.40(s, 1H), 7.72(s, 1H) |
| 90 | 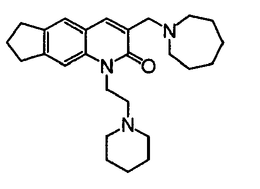 | 1.47-1.48(m, 2H), 1.61-1.71(m, 12H), 2.14(tt, J=7.2, 7.2Hz, 2H), 2.58-2.65(m, 6H), 2.73-2.75(m, 4H), 2.97(t, J=7.2Hz, 2H), 3.04 (t, J=7.2Hz, 2H), 3.68(s, 2H), 4.45-4.49(m, 2H), 7.32(s, 1H), 7.41(s, 1H), 7.80(s, 1H) |
| 91 | 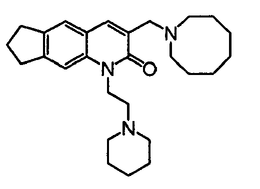 | 1.47-1.50(m, 2H), 1.61-1.71(m, 14H), 2.14(tt, J=7.4, 7.4Hz, 2H), 2.59-2.65(m, 6H), 2.67-2.69(m, 4H), 2.97(t, J=7.4Hz, 2H), 3.04(t, J=7.4Hz, 2H), 3.68(s, 2H), 4.45-4.49(m, 2H), 7.32(s, 1H), 7.41(s, 1H), 7.84 (s, 1H) |

**[Table 19]**

| No. | Structural formula | ¹H-NMR (CDCl₃) δ |
|---|---|---|
| 92 | 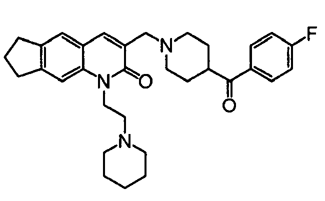 | 1.47-1.50(m, 2H), 1.61-1.73(m, 6H), 1.82-1.85(m, 2H), 2.15(tt, J=7.4, 7.4Hz, 2H), 2.58-2.65(m, 6H), 2.76(td, J=12.2, 2.6Hz, 2H), 2.97(t, J=7.4Hz, 2H), 3.05(t, J=7.4Hz, 2H), 3.17-3.21(m, 2H), 3.35(tt, J=11.3, 3.6Hz, 1H), 4.45-4.49(m, 2H), 4.65(s, 2H), 7.12-7.16(m, 2H), 7.34(s, 1H), 7.39(s, 1H), 7.63(s, 1H), 7.95-8.00(m, 2H) |
| 93 | 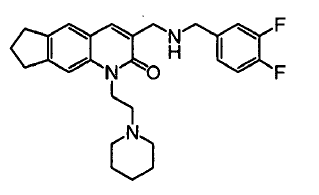 | 1.47-1.48(m, 2H), 1.61-1.66(m, 4H), 2.15(tt, J=7.4, 7.4Hz, 2H), 2.59(m, 4H), 2.61-2.66(m, 2H), 2.97(t, J=7.4Hz, 2H), 3.04(t, J=7.4Hz, 2H), 3.76(s, 2H), 3.78(s, 2H), 4.45-4.48(m, 2H), 7.04-7.12(m, 2H), 7.19-7.23(m, 1H), 7.33(s, 1H), 7.37(s, 1H), 7.59(s, 1H) |
| 94 | 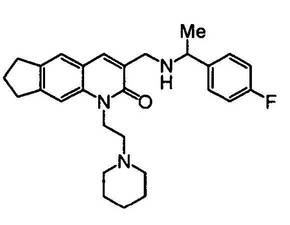 | 1.36(d, J=6.5Hz, 3H), 1.47-1.50(m, 2H), 1.58-1.66(m, 4H), 2.14(tt, J=7.4, 7.4Hz, 2H), 2.57(m, 4H), 2.62(t, J=8.0Hz, 2H), 2.96(t, J=7.4Hz, 2H), 3.04(t, J=7.4Hz, 2H), 3.55(d, J=14.2Hz, 1H), 3.61(d, J=14.2Hz, 1H), 3.80(q, J=6.5Hz, 1H), 4.43-4.47(m, 2H), 6.98-7.04(m, 2H), 7.31-7.35(m, 4H), 7.48(s, 1H) |
| 95 | 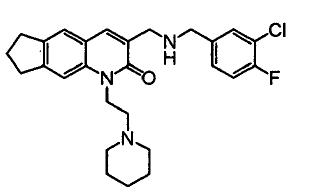 | 1.47-1.50(m, 2H), 1.61-1.66(m, 4H), 2.14(tt, J=7.4, 7.4Hz, 2H), 2.58(m, 4H), 2.61-2.65(m, 2H), 2.97(t, J=7.4Hz, 2H), 3.04(t, J=7.4Hz, 2H), 3.76(s, 2H), 3.77(s, 2H), 4.44-4.48(m, 2H), 7.05-7.10(m, 1H), 7.20-7.24(m, 1H), 7.33(s, 1H), 7.37(s, 1H), 7.41-7.43(m, 1H), 7.59(s, 1H) |

**[Table 20]**

| No. | Structural formula | ¹H-NMR (CDCl₃) δ |
|---|---|---|
| 96 | 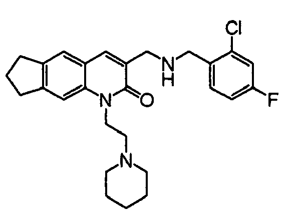 | 1.47-1.49(m, 2H), 1.61-1.66(m, 4H), 2.14(tt, J=7.4, 7.7Hz, 2H), 2.58(m, 4H), 2.61-2.65(m, 2H), 2.97(t, J=7.4Hz, 2H), 3.04(t, J=7.4Hz, 2H), 3.80(s, 2H), 3.88(s, 2H), 4.44-4.48(m, 2H), 6.61-7.01(m, 1H), 7.08-7.10(m, 1H), 7.32(s, 1H), 7.37(s, 1H), 7.44-7.48(m, 1H), 7.64(s, 1H) |
| 97 | 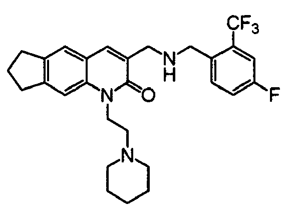 | 1.47-1.50(m, 2H), 1.61-1.67(m, 4H), 2.15(tt, J=7.4, 7.4Hz, 2H), 2.59(m, 4H), 2.62-2.65(m, 2H), 2.97(t, J=7.4Hz, 2H), 3.04(t, J=7.4Hz, 2H), 3.80(s, 2H), 3.95(s, 2H), 4.44-4.49(m, 2H), 7.21-7.25(m, 1H), 7.30-7.34(m, 2H), 7.37(s, 1H), 7.61(s, 1H), 7.74-7.78(m, 1H) |
| 98 | 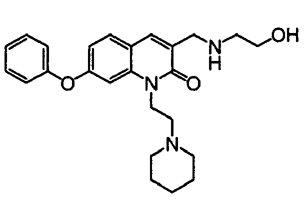 | 1.40-1.43(m, 2H), 1.52-1.57(m, 4H), 2.43(m, 4H), 2.55(t, J=7.8Hz, 2H), 2.82(t, J=5.1Hz, 2H), 3.69(t, J=5.1Hz, 2H), 3.77(s, 2H), 4.31(t, J=7.8Hz, 2H), 6.89(dd, J=8.6, 2.0Hz, 1H), 6.96(d, J=2.0Hz, 1H), 7.08-7.10(m, 2H), 7.18-7.22(m, 1H), 7.38-7.42(m, 2H), 7.49(d, J=8.6Hz, 1H), 7.63(s, 1H) |
| 99 | 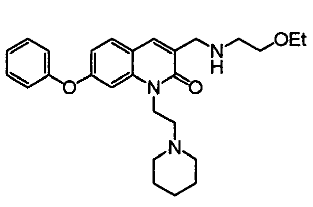 | 1.21(t, J=7.0Hz, 3H), 1.41-1.42(m, 2H), 1.53-1.56(m, 4H), 2.43(m, 4H), 2.55(t, J=7.9Hz, 2H), 2.85(t, J=5.4Hz, 2H), 3.51(q, J=7.0Hz, 2H), 3.59(t, J=5.4Hz, 2H), 3.79(d, J=1.0Hz, 2H), 4.31(t, J=7.9Hz, 2H), 6.88(dd, J=8.5, 2.0Hz, 1H), 6.96(d, J=2.0Hz, 1H), 7.08-7.10(m, 2H), 7.17-7.21(m, 1H), 7.38-7.42(m, 2H), 7.49(d, J=8.5Hz, 1H), 7.67(s, 1H) |

**[Table 21]**

| No. | Structural formula | ¹H-NMR (CDCl₃) δ |
|---|---|---|
| 100 | 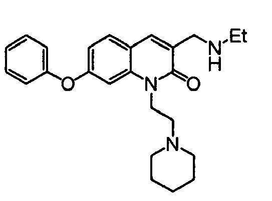 | 1.26(t, J=7.1Hz, 3H), 1.42-1.43(m, 2H), 1.53-1.58(m, 4H), 2.45(m, 4H), 2.56(t, J=7.7Hz, 2H), 2.85(q, J=7.1Hz, 2H), 3.89(s, 2H), 4.31(t, J=7.7Hz, 2H), 6.89(dd, J=8.5, 2.2Hz, 1H), 6.96(d, J=2.2Hz, 1H), 7.08-7.11(m, 2H), 7.19-7.23(m, 1H), 7.39-7.43(m, 2H), 7.52(d, J=8.5Hz, 1H), 7.78(s, 1H) |
| 101 | 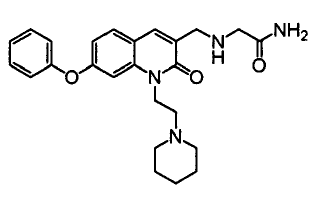 | 1.37-1.60(m, 6H), 2.38-2.48(m, 4H), 2.56(m, 2H), 3.30(s, 2H), 3.73(s, 2H), 4.31(m, 2H), 5.56(br.s, 1H), 6.91(dd, J=8.6, 2.0Hz, 1H), 6.98(d, J=2.0Hz, 1H), 7.10(m, 2H), 7.21(m, 1H), 7.42(m, 2H), 7.50(d, J=8.6Hz, 1H), 7.58 (s, 1H), 7.77(br.s, 1H) |
| 102 | 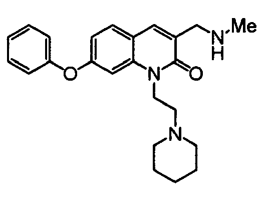 | 1.41-1.43(m, 2H), 1.52-1.59(m, 4H), 2.42-2.45(m, 4H), 2.47(s, 3H), 2.55(m, 2H), 3.71(d, J=1.0Hz, 2H), 4.31(m, 2H), 6.88(dd, J=8.5, 2.2Hz, 1H), 6.96(d, J=2.2Hz, 1H), 7.08-7.11(m, 2H), 7.17-7.22(m, 1H), 7.38-7.43(m, 2H), 7.49(d, J=8.5Hz, 1H), 7.63(s, 1H) |
| 103 | 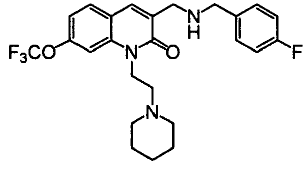 | 1.40-1.65(m, 6H), 2.50-2.65(m, 6H), 3.78(s, 2H), 3.81(s, 2H), 4.40(m, 2H), 7.01(m, 2H), 7.09(br.d, J=8.6Hz, 1H), 7.29-7.36(m, 3H), 7.57(d, J=8.6Hz, 1H), 7.67(s, 1H) |

**[Table 22]**

| No. | Structural formula | ¹H-NMR (CDCl₃) δ |
|---|---|---|
| 104 | 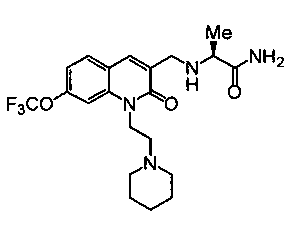 | 1.34(d, J=7.0Hz, 3H), 1.40-1.68(m, 6H), 2.50-2.66(m, 6H), 3.25(quart, J=7.0Hz, 1H), 3.64(d, J=13.4Hz, 1H), 3.85(d, J=13.4Hz, 1H), 4.41(m, 2H), 5.36(br.s, 1H), 7.12(br.d, J=8.6Hz, 1H), 7.34(s, 1H), 7.58(d, J=8.6Hz, 1H), 7.62(s, 1H), 7.66(br.s, 1H) |
| 105 | 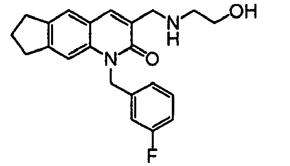 | 2.09(m, 2H), 2.82-2.98(m, 6H), 3.72(m, 2H), 3.85(s, 2H), 5.55(br.s, 2H), 6.82-7.02(m, 3H), 7.06(s, 1H), 7.27(m, 1H), 7.40(s, 1H), 7.71(s, 1H) |
| 106 | 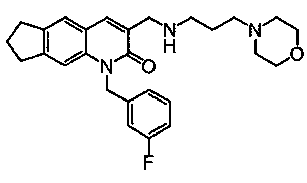 | 1.82(m, 2H), 2.10(m, 2H), 2.42-2.50(m, 6H), 2.84(t, J=6.6Hz, 2H), 2.90-2.99(m, 4H), 3.65-3.70(m, 4H), 3.88(s, 2H), 5.54(br.s, 2H), 6.81-7.01(m, 3H), 7.07(s, 1H), 7.26(m, 1H), 7.41(s, 1H), 7.78(s, 1H) |
| 107 | 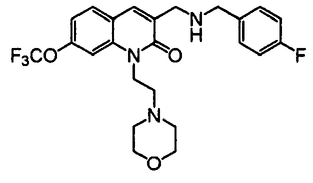 | 2.56-2.70(m, 6H), 3.70-3.75(m, 4H), 3.78(s, 2H), 3.82(s, 2H), 4.41(m, 2H), 7.01 (m, 2H), 7.11(br.d, J=8.6Hz, 1H), 7.26-7.35(m, 3H), 7.59(d, J=8.6Hz, 1H), 7.69(s, 1H) |
| 108 | 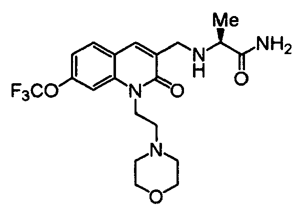 | 1.35(d, J=7.0Hz, 3H), 2.56-2.71(m, 6H), 3.25(quart, J=7.0Hz, 1H), 3.64(d, J=13.4Hz, 1H), 3.68-3.76(m, 4H), 3.86(d, J=13.4Hz, 1H), 4.41(m, 2H), 5.41(br.s, 1H), 7.13(d, J=8.6Hz, 1H), 7.29(s, 1H), 7.58-7.65(m, 3H) |

**[Table 23]**

| No. | Structural formula | ¹H-NMR (CDCl₃) δ |
|---|---|---|
| 109 | 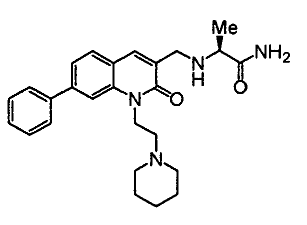 | 1.35(d, J=7.1Hz, 3H), 1.45-1.49(m, 2H), 1.59-1.65(m, 4H), 2.59(m, 4H), 2.69(t, J=7.8Hz, 2H), 3.27(quart, J=7.1Hz, 1H), 3.66(d, J=13.1Hz, 1H), 3.88(d, J=13.1Hz, 1H), 4.50-4.60(m, 2H), 5.55(br.s, 1H), 7.41-7.52(m, 4H), 7.62(d, J=8.0Hz, 1H), 7.65-7.68(m, 4H), 7.77(br.s, 1H) |
| 110 | 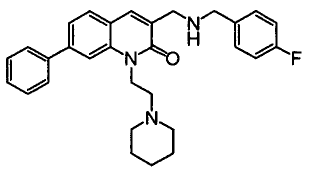 | 1.43-1.47(m, 2H), 1.58-1.65(m, 4H), 2.57-2.60(m, 4H), 2.68(t, J=7.7Hz, 2H), 3.81(s, 2H), 3.82(s, 2H), 4.53(t, J=7.7Hz, 2H), 6.98-7.04(m, 2H), 7.26-7.52(m, 6H), 7.60-7.70(m, 5H) |
| 111 | 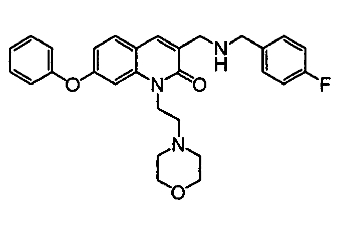 | 2.09(br.s, 1H), 2.47(t, J=4.5Hz, 4H), 2.58(t, J=7.8Hz, 2H), 3.66(t, J=4.5Hz, 4H), 3.76(s, 2H), 3.81(s, 2H), 4.30(t, J=7.8Hz, 2H), 6.90(dd, J=8.5, 2.1Hz, 1H), 6.93(d, J=2.1Hz, 1H), 6.98-7.03(m, 2H), 7.09-7.12(m, 2H), 7.19-7.23(m, 1H), 7.31-7.34(m, 2H), 7.39-7.43(m, 2H), 7.50(d, J=8.5Hz, 1H), 7.63(s, 1H) |
| 112 | 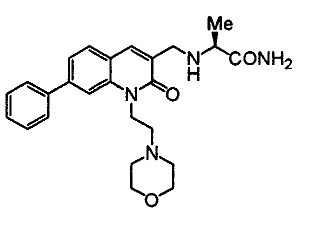 | 1.35(d, J=6.8Hz, 3H), 2.62-2.64(m, 4H), 2.72-2.75(m, 2H), 3.28(q, J=6.8Hz, 1H), 3.66(d, J=13.3Hz, 1H), 3.71-3.74(m, 4H), 3.88(d, J=13.3Hz, 1H), 4.48-4.61(m, 2H), 5.58(br.s, 1H), 7.41-7.53(m, 4H), 7.62-7.67(m, 5H), 7.73(br.s, 1H) |

**[Table 24]**

| No. | Structural formula | ¹H-NMR (CDCl₃) δ |
|---|---|---|
| 113 | 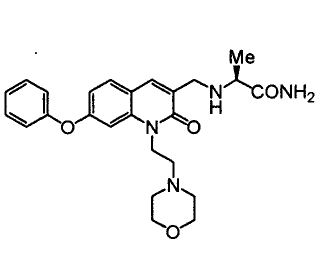 | 1.34(d, J=6.8Hz, 3H), 2.48-2.57(m, 4H), 2.57-2.60(m, 2H), 3.25(q, J=6.8Hz, 1H), 3.61(d, J=13.2Hz, 1H), 3.65-3.67(m, 4H), 3.84(d, J=13.2Hz, 1H), 4.23-4.38(m, 2H), 5.39(br.s, 1H), 6.90-6.93(m, 2H), 7.10-7.12(m, 2H), 7.20-7.24(m, 1H), 7.40-7.44(m, 2H), 7.50(d, J=8.5Hz, 1H), 7.59(s, 1H), 7.75(br.s, 1H) |
| 114 | 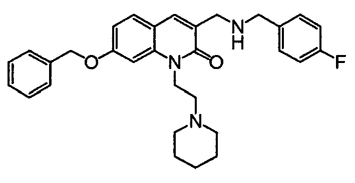 | 1.45(m, 2H), 1.59-1.64(m, 4H), 2.53-2.60 (m, 6H), 3.74(s, 2H), 3.79(s, 2H), 4.37-4.41(m, 2H), 5.18(s, 2H), 6.88-6.91(m, 1H), 6.97-7.02(m, 3H), 7.30-7.48(m, 8H), 7.58-7.59(m, 1H) |
| 115 | 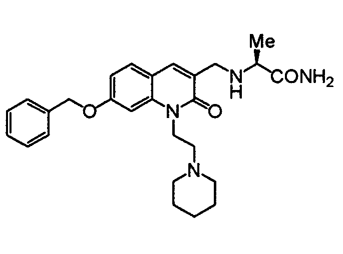 | 1.33(d, J=7.1Hz, 3H), 1.46-1.47(m, 2H), 1.59-1.65(m, 4H), 2.01(br.s, 1H), 2.53-2.60(m, 6H), 3.24(q, J=7.1Hz, 1H), 3.59(d, J=13.2Hz, 1H), 3.82(d, J=13.2Hz, 1H), 4.32-4.47(m, 2H), 5.19(s, 2H), 5.50(br.s, 1H), 6.91(dd, J=8.5, 2.2Hz, 1H), 7.00(d, J=2.2Hz, 1H), 7.34-7.47(m, 6H), 7.55(s, 1H), 7.80(br.s, 1H) |

### Reference Example 11

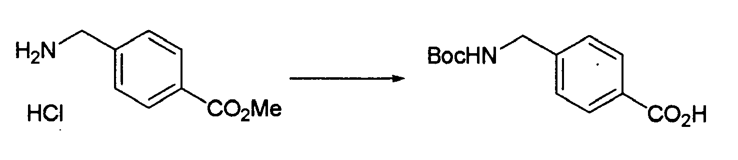

Triethylamine (1 ml) and di-tert-butyl dicarbonate (1.2 g, 5.5 mmol) were added to a solution of methyl 4-(aminomethyl)-benzoate hydrochloride (1.0 g, 5.0 mmol) in methanol (10 ml). After 30 minutes of stirring, water was added thereto, followed by extraction with ethyl acetate. The extract solution was washed with a saturated aqueous sodium chloride solution and then concentrated under reduced pressure. To the resulting residue were added methanol (15 ml) and a 1N aqueous sodium hydroxide solution (5 ml), and the resulting mixture was heated to 60°C. After 1 hour of stirring, water was added thereto and the aqueous layer was washed with diethyl ether, followed by adding thereto a 2N-aqueous hydrochloric acid solution. The crystals were collected by filtration using a Kiriyama funnel, and dried under reduced pressure to obtain the desired compound (0.88 g, 70%).
¹H-NMR (CDCl₃) δ 1.47(s, 9H), 4.39(br.s, 2H), 4.96(br.s., 1H), 7.38(d, J=8.3Hz, 2H), 8.07(d, J=8.3Hz, 2H).

### Reference Example 12

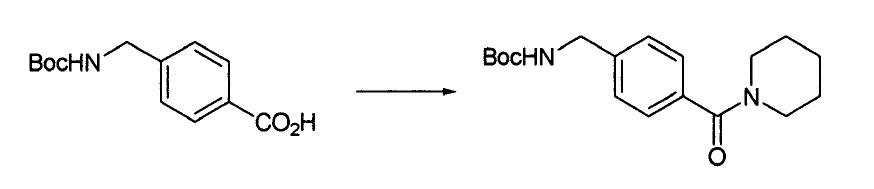

Piperidine (52 mg, 0.61 mmol), N-(3-dimethylaminopropyl)-N'-ethylcarbodiimide (87 mg, 0.56 mmol) and 1-hydroxybenzotriazole (76 mg, 0.56 mmol) were added to a solution of the compound (0.13 g, 0.51 mmol) obtained in Reference Example 11 in N,N'-dimethylformamide (5.1 ml). After one and a half hours of stirring, a saturated aqueous ammonium chloride solution was added thereto, followed by extraction with ethyl acetate. The extract solution was washed with water, a saturated aqueous sodium hydrogencarbonate solution and a saturated aqueous sodium chloride solution, dried over sodium sulfate and then concentrated under reduced pressure to obtain the desired compound (0.13 g, 80%).
¹H-NMR (CDCl₃) δ 1.44-1.45(m, 11H), 1.66(m, 4H), 3.32(m, 2H), 3.68(m, 2H), 4.29(m, 2H), 5.31-5.40(m, 1H), 7.28-7.35(m, 4H).

### Reference Example 13

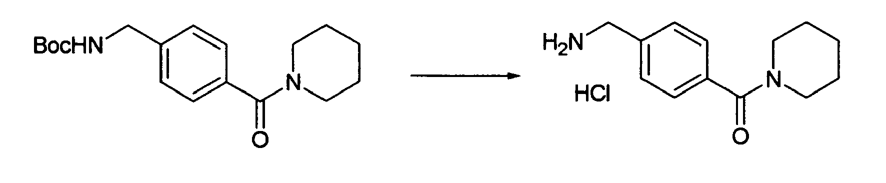

To the compound (0.13 g, 0.41 mmol) obtained in Reference Example 12 was added 4N hydrochloric acid/ethyl acetate (4.1 ml). The resulting mixture was stirred for 20 minutes and then concentrated under reduced pressure to obtain the desired compound (98 mg, 94%).
¹H-NMR (CDCl₃) δ 1.63-1.84(m, 6H), 3.59(m, 4H), 4.27(m, 2H), 7.34-7.36(m, 2H), 7.72-7.74(m, 2H), 8.64(br.s, 3H).

### Reference Example 14

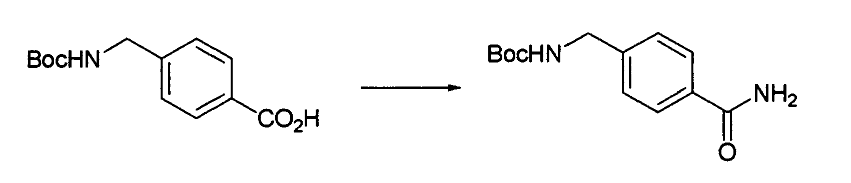

Thionyl chloride (0.11 ml, 1.6 mmol) was added to a solution of the compound (0.13 g, 0.52 mmol) obtained in Reference Example 11 in dichloromethane (5.2 ml), and the resulting mixture was heated to 40°C. The mixture was stirred for 2 hours and then concentrated under reduced pressure. After dichloromethane (5.2 ml) was added thereto, the resulting mixture was cooled to 0°C and aqueous ammonia (a 28% aqueous solution, 1.7 ml) was slowly added dropwise thereto. After 5 minutes of stirring, water was added thereto, followed by extraction with chloroform. The extract solution was washed with a saturated aqueous sodium chloride solution, dried over sodium sulfate and then concentrated under reduced pressure to obtain the desired compound (43 mg, 33%). ¹H-NMR (CDCl₃) δ 1.46(s, 9H), 4.35-4.37(m, 2H), 5.03(m, 1H), 5.91-6.20(m, 2H), 7.34(d, J=8.3Hz, 2H), 7.77(d, J=8.3Hz, 2H).

### Reference Example 15

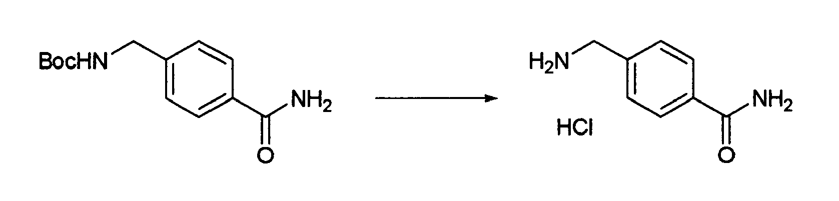

From the compound obtained in Reference Example 14, the desired compound was obtained by the same process as in Example 13.
¹H-NMR(DMSO-d₆) δ 4.05-4.09(m, 2H), 7.43(br.s, 1H), 7.55(d, J=8.3Hz, 2H), 7.90(d, J=8.3Hz, 2H), 8.03(br.s, 1H), 8.47(m, 3H).

### Reference Example 16

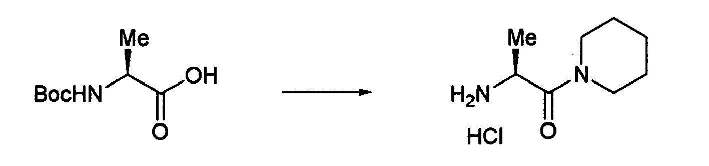

The desired compound was obtained from N-Boc-(L)-alanine by the same processes as in Reference Examples 12 and 13.
¹H-NMR(DMSO-d₆) δ 1.30(d, J=6.8Hz, 3H), 1.40-1.65(m, 6H), 3.34-3.58(m, 4H), 4.32(q, J=6.8Hz, 1H), 8.27(br, 3H).

### Example 116

### 3-({[4-(Piperidin-1-ylcarbonyl)benzyl]amino}-methyl)-1-(2-piperidin-1-ylethyl)-1,6,7,8-tetrahydro-2H-cyclopenta[g]quinolin-2-one

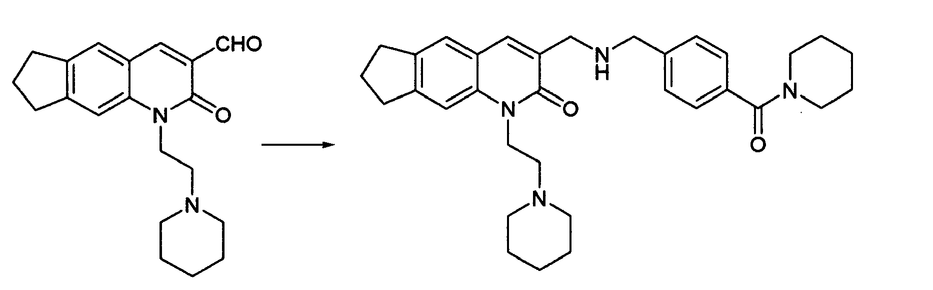

From the compound obtained in Reference Example 13, the desired compound was obtained by the same process as in Example 62.
¹H-NMR (CDCl₃) δ 1.47-1.67(m, 12H), 2.14(tt, J=7.3, 7.3Hz, 2H), 2.60-2.67(m, 6H), 2.97(t, J=7.3Hz, 2H), 3.04(t, J=7.3Hz, 2H), 3.35(m, 2H), 3.70(m, 2H), 3.79(s, 2H), 3.85(s, 2H), 4.48(t, J=7.9Hz, 2H), 7.34-7.41(m, 6H), 7.61(s, 1H).
The following compounds of Examples 117 to 123 were prepared by the same processes as in Reference Examples 5, 6, 7 and 11 to 16 and Example 116.

**[Table 25]**

| No. | Structural formula | ¹H-NMR (CDCl₃) δ |
|---|---|---|
| 117 | 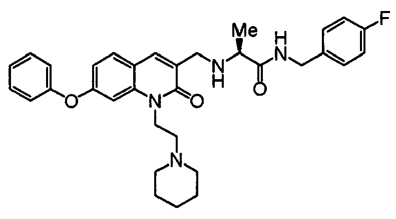 | 1.32(d, J=7.0Hz, 3H), 1.40-1.60(m, 6H), 2.37-2.45(m, 4H), 2.52(t, J=7.9Hz, 2H), 3.29(quart, J=7.0Hz, 1H), 3.57(d, J=13.2Hz, 1H), 3.71(d, J=13.2Hz, 1H), 4.27(m, 2H), 4.45(d, J=6.0Hz, 2H), 6.85-7.50(m, 13H), 8.24(t, J=6.0Hz, 1H) |
| 118 | 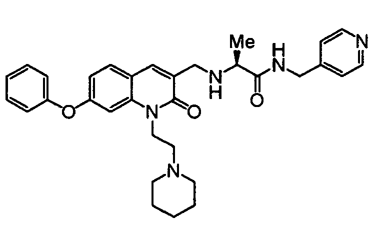 | 1.36(d, J=7.0Hz, 3H), 1.35-1.60(m, 6H), 2.35-2.60(m, 6H), 3.33(quart, J=7.0Hz, 1H), 3.60(d, J=12.8Hz, 1H), 3.77(d, J=12.8Hz, 1H), 4.30(m, 2H), 4.48(dd, J=15.9, 6.4Hz, 1H), 4.55(dd, J=15.9, 6.4Hz, 1H), 6.87-7.55(m, 11H), 8.54(m, 2H), 8.63(t, J=6.4Hz, 1H) |
| 119 | 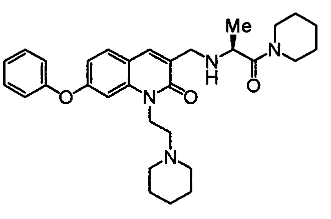 | 1.29(d, J=6.8Hz, 3H), 1.35-1.60(m, 12H), 2.38-2.58(m, 6H), 3.38-3.75(m, 7H), 4.30(m, 2H), 6.87(dd, J=8.4, 2.0Hz, 1H), 6.95(d, J=2.0Hz, 1H), 7.09(m, 2H), 7.19(m, 1H), 7.40(m, 2H), 7.50(d, J=8.4Hz, 1H), 7.72(s, 1H) |

**[Table 26]**

| No. | Structural formula | ¹H-NMR (CDCl₃) δ |
|---|---|---|
| 120 | 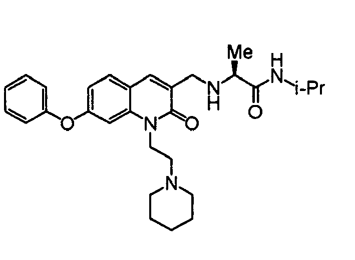 | 1.18(d, J=6.8Hz, 3H), 1.21(d, J=6.8Hz, 3H), 1.28(d, J=7.0Hz, 3H), 1.35-1.60(m, 6H), 2.38-2.60(m, 6H), 3.18(quart, J=7.0Hz, 1H), 3.54(d, J=13.4Hz, 1H), 3.77(d, J=13.4Hz., 1H), 4.10(m, 1H), 4.31(m, 2H), 6.88(dd, J=8.4, 2.2Hz, 1H), 6.96(d, J=2.2Hz, 1H), 7.10(m, 2H), 7.21(m, 1H), 7.38-7.63(m, 5H) |
| 121 | 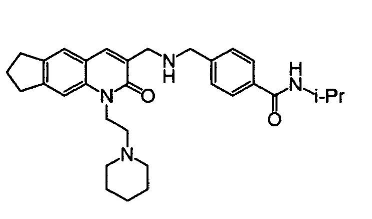 | 1.26(d, J=6.6Hz, 6H), 1.47-1.48(m, 2H), 1.61-1.66(m, 4H), 2.14(tt, J=7.4, 7.4Hz, 2H), 2.58-2.66(m, 6H), 2.97(t, J=7.4Hz, 2H), 3.04(t, J=7.4Hz, 2H), 3.91(s, 2H), 4.28(qq, J=6.6, 6.6Hz, 1H), 4.47(t, J=7.9Hz, 2H), 4.64(s, 2H), 6.01(br.s, 1H), 7.33-7.40(m, 4H), 7.62(s, 1H), 7.70-7.74(m, 2H) |
| 122 | 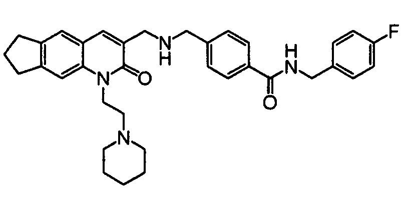 | 1.45-1.52(m, 2H), 1.62-1.69(m, 4H), 2.14(tt, J=7.1, 7.1Hz, 2H), 2.63-2.68(m, 6H), 2.97(t, J=7.1Hz, 2H), 3.05(t, J=7.1Hz, 2H), 3.77(s, 2H), 3.87(s, 2H), 4.48(t, J=7.7Hz, 2H), 4.62(d, J=5.7Hz, 2H), 7.01-7.07(m, 2H), 7.31-7.36(m, 6H), 7.44(d, J=8.2Hz, 2H), 7.59(s, 1H), 7.75(d, J=8.2Hz, 2H) |

**[Table 27]**

| No. | Structural formula | ¹H-NMR (CDCl₃) δ |
|---|---|---|
| 123 | 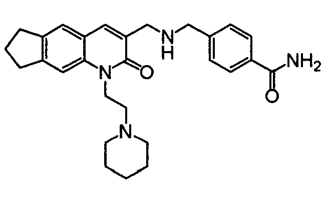 | 1.47-1.51(m, 2H), 1.61-1.68(m, 4H), 2.14(tt, J=7.4, 7.4Hz, 2H), 2.60(m, 4H), 2.65(t, J=7.7Hz, 2H), 2.97(t, J=7.4Hz, 2H), 3.04(t, J=7.4Hz, 2H), 3.77(s, 2H), 3.88(s, 2H), 4.48(t, J=7.7Hz, 2H), 7.34(s, 1H), 7.36(s, 1H), 7.44(d, J=8.2Hz, 2H), 7.60(s, 1H), 7.78(d, J=8.2Hz, 2H) |

### Reference Example 17

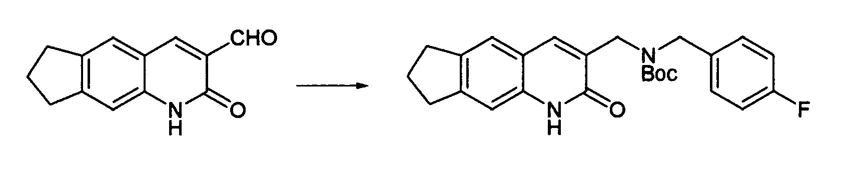

2-Fluorobenzylamine (0.23 g, 1.8 mmol), acetic acid (a catalytic amount) and sodium triacetoxyborohydride (0.39 g, 1.8 mmol) were added to a solution of the compound (0.33 g, 1.5 mmol) obtained in Reference Example 7 in N,N'-dimethylformamide (15 ml). After 1 hour of stirring, di-tert-butyl dicarbonate (0.40 g, 1.8 mmol) was added thereto and stirred overnight. Water was added to the reaction solution and the crystals were collected by filtration using a Kiriyama funnel, and dried under reduced pressure to obtain the desired compound (0.58 g, 89%).
¹H-NMR (CDCl₃) δ 1.48(s, 9H), 2.08-2.18(m, 2H), 2.94-3.01(m, 4H), 4.36-4.44(m, 2H), 4.59(s, 2H), 6.98-7.04(m, 2H), 7.17(br.s, 1H), 7.26-7.37(m, 4H).

### Reference Example 18

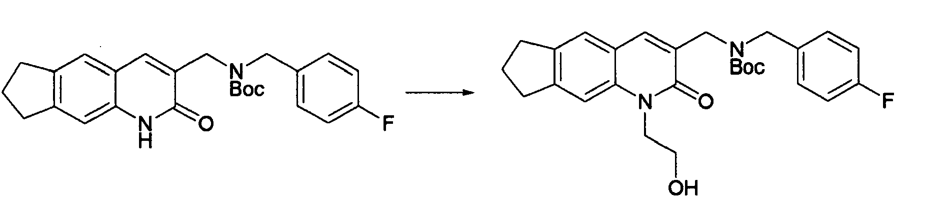

Sodium hydride (0.11 g, 60% in oil, 2.8 mmol) and (2-bromoethoxy)-tert-butyldimethylsilane (0.66 g, 2.8 mmol) were added to a solution of the compound (0.97 g, 2.3 mmol) obtained in Reference Example 17 in N,N'-dimethylformamide (23 ml), and the resulting mixture was heated to 80°C. After 1 hour, the reaction solution was allowed to cool to room temperature and water was added thereto, followed by extraction with ethyl acetate. The extract solution was washed with water and a saturated aqueous sodium chloride solution, dried over sodium sulfate and then concentrated under reduced pressure. To the resulting residue were added tetrahydrofuran (26 ml) and tetrabutylammonium fluoride (3.0 ml, a 1M tetrahydrofuran solution, 3.0 mmol).
After two and a half hours of stirring, water was added thereto, followed by extraction with ethyl acetate. The extract solution was washed with a saturated aqueous sodium chloride solution, dried over sodium sulfate and then concentrated under reduced pressure. The resulting residue was purified by the use of a silica gel column (hexane : ethyl acetate = 70 : 30 to 50 : 50, chloroform : methanol = 90 : 10) to obtain the desired compound (0.71 g, 66%) and its O-alkyl derivative (0.15 g, 14%).
¹H-NMR (CDCl₃) δ 1.46(s, 9H), 2.11-2.19(m, 2H), 2.98-3.05(m, 4H), 3.53(br.s, 1H), 4.01(br.s, 2H), 4.32-4.40(m, 2H), 4.53(s, 4H), 6.95-7.01(m, 2H), 7.27-7.46 (m, 5H).

### Reference Example 19

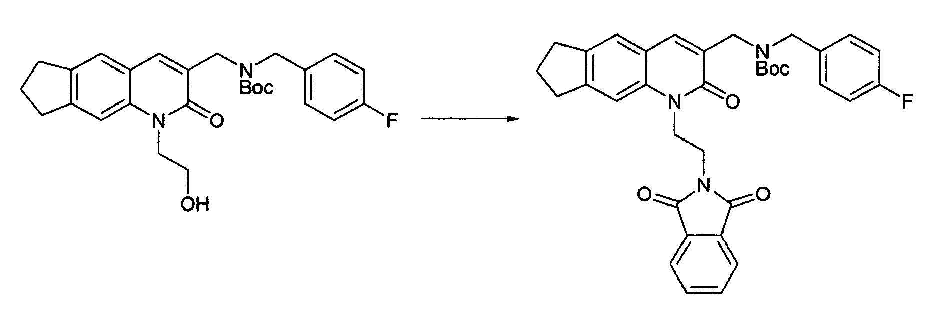

Triethylamine (72 mg, 0.72 mmol) was added to a solution of the compound (0.11 g, 0.24 mmol) obtained in Reference Example 18 in dichloromethane (2.4 ml), and the resulting mixture was cooled to -10°C. Methanesulfonyl chloride (54 mg, 0.48 mmol) was added thereto and stirred for 30 minutes. A solution of potassium phthalimide (0.18 g, 0.96 mmol) in tetrahydrofuran (2.4 ml) was added thereto and the resulting mixture was warmed to room temperature. After stirring overnight, water was added thereto, followed by extraction with chloroform. The extract solution was washed with a saturated aqueous potassium carbonate solution and a saturated aqueous sodium chloride solution, dried over sodium sulfate and then concentrated under reduced pressure. The resulting residue was purified by the use of a silica gel column to obtain the desired compound (22 mg, 16%).
¹H-NMR (CDCl₃) δ 1.46(s, 9H), 2.04-2.08(m, 2H), 2.88-2.96(m, 4H), 4.10(t, J=6.0Hz, 2H), 4.22(br.s, 1H), 4.32(m, 1H), 4.39-4.42(m, 2H), 4.62(t, J=6.0Hz, 2H), 6.94-7.00(m, 2H), 7.21-7.41(m, 5H), 7.62-7.68(m, 2H), 7.76-7.79(m, 2H).

### Reference Example 20

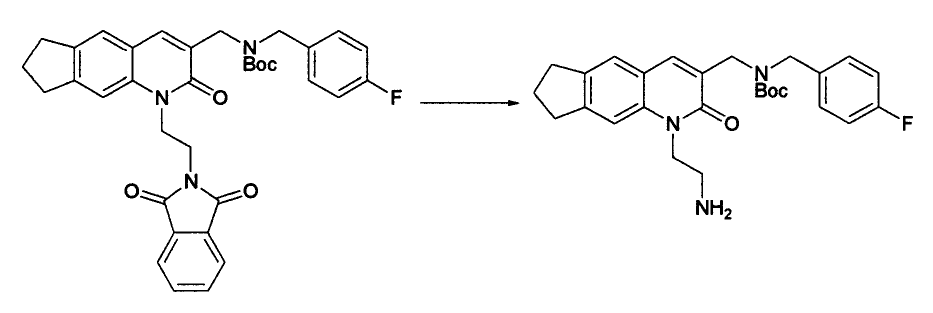

Hydrazine hydrate (21 mg, 0.65 mmol) was added to a solution of the compound (0.26 g, 0.43 mmol) obtained in Reference Example 19 in methanol (4.3 ml), and the resulting mixture was heated to 60°C. After 2 hours of stirring, the reaction solution was concentrated under reduced pressure and water was added thereto, followed by extraction with chloroform. The extract solution was washed with a saturated aqueous sodium chloride solution, dried over sodium sulfate and then concentrated under reduced pressure to obtain the desired compound (0.17 g, 82%).
¹H-NMR (CDCl₃) δ 1.46(s, 9H), 2.12-2.16(m, 2H), 2.83-3.08(m, 6H), 4.33-4.40(m, 4H), 4.46(s, 2H), 6.96(m, 2H), 7.28-7.44(m, 5H).

### Example 124

### 1-(2-Aminoethyl)-3-{[(4-fluorobenzyl)amino]-methyl}-1,6,7,8-tetrahydro-2H-cyclopenta[g]quinolin-2-one

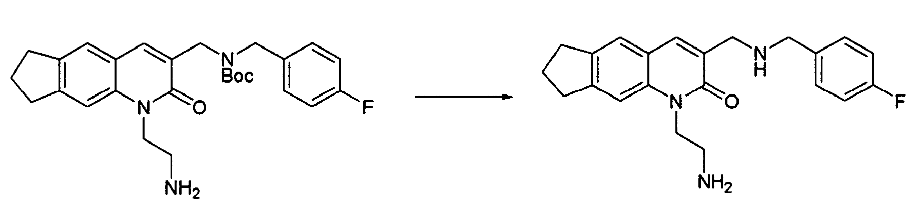

To a solution of the compound (24 mg, 52 µmol) obtained in Reference Example 20 in methanol (5.0 ml) was added 4N hydrochloric acid/ethyl acetate (1.0 ml), and stirred overnight. Water was added thereto and the aqueous layer was washed with chloroform, followed by adding thereto a 1N aqueous sodium hydroxide solution. After extraction with chloroform, the extract solution was washed with a saturated aqueous sodium chloride solution, dried over sodium sulfate and then concentrated under reduced pressure to obtain the desired compound (9.0 mg, 47%).
¹H-NMR (CDCl₃) δ 2.15(tt, J=7.1, 7.1Hz, 2H), 2.97(t, J=7.1Hz, 2H), 3.02-3.06(m, 2H), 3.08(t, J=7.1Hz, 2H), 3.77(s, 2H), 3.80(s, 2H), 4.38-4.41(m, 2H), 6.98-7.03(m, 2H), 7.27(s, 1H), 7.31-7.35(m, 2H), 7.37(s, 1H), 7.62(s, 1H).

### Example 125

### 1-(2-Aminoethyl)-3-{[(4-fluorobenzyl)amino]-methyl}-7-phenoxyquinolin-2-one

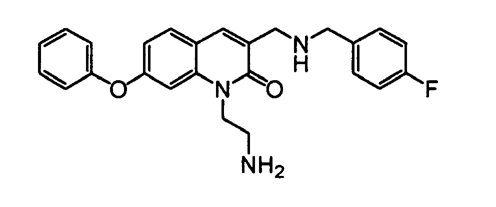

The desired compound was obtained by the same process as in Example 124.
¹H-NMR (CDCl₃) δ 3.01-3.04(m, 2H), 3.77(s, 2H), 3.82(s, 2H), 4.28-4.31(m, 2H), 6.84(dd, J=8.8, 2.2Hz, 1H), 6.98-7.02(m, 3H), 7.06-7.08(m, 2H), 7.17-7.21(m, 1H), 7.31-7.35(m, 2H), 7.37-7.41(m, 2H), 7.49(d, J=8.6Hz, 1H), 7.64(s, 1H).

### Reference Example 21

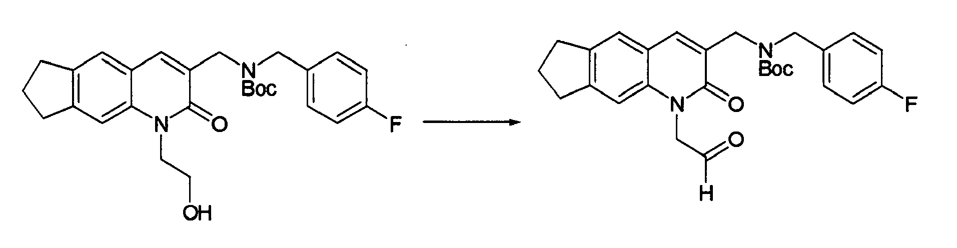

A solution of oxalyl chloride (0.15 g, 1.1 mmol) in dichloromethane (5.4 ml) was cooled to -78°C and dimethyl sulfoxide (0.11 g, 1.5 mmol) was added thereto. After 10 minutes of stirring, the compound (0.25 g, 0.54 mmol) obtained in Reference Example 18 was added thereto and stirred for another 30 minutes, and then triethylamine (0.55 ml. 3.9 mmol) was added thereto. The resulting mixture was warmed to 0°C and stirred for 30 minutes and a saturated aqueous ammonium chloride solution was added thereto, followed by extraction with ethyl acetate. The extract solution was washed with a saturated aqueous sodium chloride solution, dried over sodium sulfate and then concentrated under reduced pressure to obtain the desired compound (0.28 g) as a crude product.
¹H-NMR (CDCl₃) δ 1.39(s, 9H), 2.03(m, 2H), 2.89(m, 4H), 4.24-4.32(m, 2H), 4.47(s, 2H), 5.06(s, 2H), 6.83-6.91(m, 3H), 7.18-7.60 (m, 4H), 9.60(s, 1H).

### Example 126

### 1-[2-(Cyclohexylamino)ethyl]-3-{[(4-fluorobenzyl)amino]methyl}-1,6,7,8-tetrahydro-2H-cyclopenta[g]quinolin-2-one

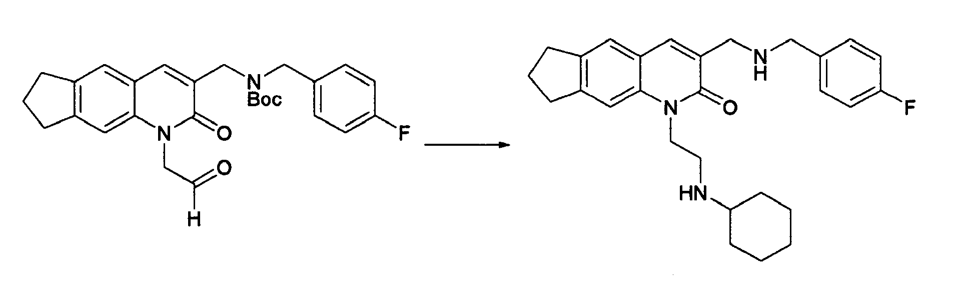

Cyclohexylamine (13 mg, 0.12 mmol) and acetic acid (a catalytic amount) were added to a solution of the compound (48 mg, 0.10 mmol) obtained in Reference Example 21 in dichloromethane (3.0 ml) and stirred for 10 minutes, and then sodium triacetoxyborohydride (27 mg, 0.12 mmol) was added thereto. After 30 minutes of stirring, 4N hydrochloric acid/ethyl acetate (1.0 ml) was added thereto. After 1 hour of stirring, water was added thereto, followed by washing with chloroform, and a 1N aqueous sodium hydroxide solution was added to the aqueous layer. After extraction with chloroform, the extract solution was washed with a saturated aqueous sodium hydrogencarbonate solution and a saturated aqueous sodium chloride solution, dried over sodium sulfate and then concentrated under reduced pressure to obtain the desired compound (17.3 mg, 37%).
¹H-NMR (CDCl₃) δ 1.30-1.34(m, 4H), 1.59-1.75(m, 2H), 1.89-1.92(m, 4H), 2.14(tt, J=7.3, 7.3Hz, 2H), 2.47-2.54(m, 1H), 2.95-3.00(m, 4H), 3.04(t, J=7.3Hz, 2H), 3.77(s, 2H), 3.80(s, 2H), 4.40(t, J=7.3Hz, 2H), 6.98-7.02(m, 2H), 7.28-7.39(m, 4H), 7.61(s, 1H).
The following compounds of Examples 127 to 133 were prepared by the same processes as in Reference Examples 5, 6, 7, 17, 18 and 21 and Example 126.

**[Table 28]**

| No. | Structural formula | ¹H-NMR (CDCl₃) δ |
|---|---|---|
| 127 | 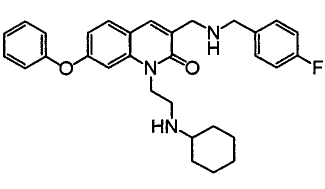 | 0.98-1.25(m, 4H), 1.58-1.84(m, 6H), 2.39-2.46(m, 1H), 2.91(t, J=7.6Hz, 2H), 3.77(s, 2H), 3.80(s, 2H), 4.27(t, J=7.6Hz, 2H), 6.86(dd, J=8.5, 1.9Hz, 1H), 6.98-7.03(m, 3H), 7.05-7.09(m, 2H), 7.17-7.21(m, 1H), 7.31-7.34(m, 2H), 7.37-7.41(m, 2H), 7.48(d, J=8.5Hz, 1H), 7.63(s, 1H) |
| 128 | 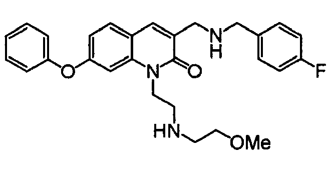 | 2.80(t, J=5.1Hz, 2H), 2.92(t, J=7.3Hz, 2H), 3.33(s, 3H), 3.45(t, J=5.1Hz, 2H), 3.77(s, 2H), 3.81(s, 2H), 4.31(t, J=7.3Hz, 2H), 6.85(dd, J=8.6, 2.2Hz, 1H), 6.98-7.03(m, 3H), 7.06-7.09(m, 2H), 7.16-7.21(m, 1H), 7.31-7.35(m, 2H), 7.37-7.41(m, 2H), 7.48(d, J=8.6Hz, 1H), 7.63(s, 1H) |
| 129 | 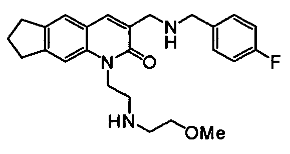 | 2.14(tt, J=7.6, 7.6Hz, 2H), 2.89(t, J=4.9Hz, 2H), 2.95-3.06(m, 6H), 3.36(s, 3H), 3.51(t, J=4.9Hz, 2H), 3.77(s, 2H), 3.80(s, 2H), 4.43(t, J=7.6Hz, 2H), 6.98-7.03(m, 2H), 7.31-7.36(m, 4H), 7.61(s, 1H) |
| 130 | 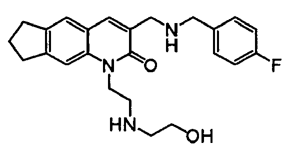 | 2.14(tt, J=7.6, 7.6Hz, 2H), 2.86(t, J=5.0Hz, 2H), 2.97(t, J=7.6Hz, 2H), 3.01-3.06(m, 4H), 3.64(t, J=5.0Hz, 2H), 3.76(s, 2H), 3.80(s, 2H), 4.46(t, J=6.8Hz, 2H), 6.98-7.03(m, 2H), 7.27(s, 1H), 7.31-7.34(m, 2H), 7.38(s, 1H), 7.62(s, 1H) |
| 131 | 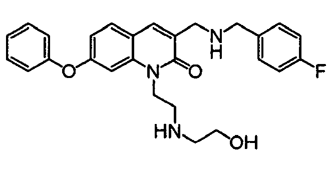 | 2.11(br.s, 1H), 2.78(t, J=5.1Hz, 2H), 2.95(t, J=6.8Hz, 2H), 3.58(t, J=5.1Hz, 2H), 3.76(s, 2H), 3.81(s, 2H), 4.33(t, J=6.8Hz, 2H), 6.86(dd, J=8.5, 1.9Hz, 1H), 6.97-7.03(m, 3H), 7.03-7.09(m, 2H), 7.18-7.22(m, 1H), 7.31-7.34(m, 2H), 7.38-7.42(m, 2H), 7.49(d, J=8.5Hz, 1H), 7.64(s, 1H) |

**[Table 29]**

| No. | Structural formula | ¹H-NMR (CDCl₃) δ |
|---|---|---|
| 132 | 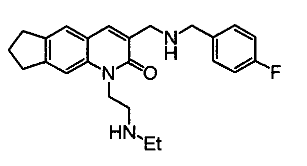 | 1.14(t, J=7.1Hz, 3H), 2.14(tt, J=7.5, 7.5Hz, 2H), 2.76(q, J=7.1Hz, 2H), 2.94-3.06(m, 6H), 3.78(s, 2H), 3.81(s, 2H), 4.45(t, J=7.1Hz, 2H), 6.98-7.03(m, 2H), 7.31-7.37(m, 4H), 7.62(s, 1H) |
| 133 | 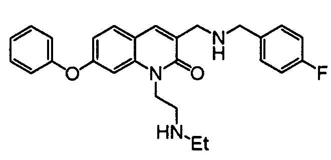 | 1.09(t, J=7.1Hz, 3H), 2.67(q, J=7.1Hz, 2H), 2.93(t, J=7.0Hz, 2H), 3.77(s, 2H), 3.82(s, 2H), 4.33(t, J=7.0Hz, 2H), 6.85(dd, J=8.5, 2.0Hz, 1H), 6.98-7.08(m, 5H), 7.16-7.21(m, 1H), 7.31-7.42(m, 4H), 7.48(d, J=8.5Hz, 1H), 7.64(s,1H) |

### Reference Example 22

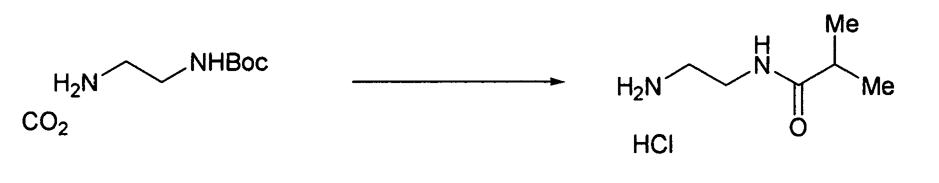

Ethyl acetate (2.3 ml) was added to a solution of tert-butyl-N-(2-aminoethyl)carbamate carbonate (0.11 g, 0.55 mmol) in N,N'-dimethylformamide (2.3 ml), and the resulting mixture was cooled to 0°C. Pyridine (48 mg, 0.61 mmol) and isobutyric anhydride (95 mg, 0.61 mmol) were added thereto. After 2 hours of stirring, water was added thereto, followed by extraction with ethyl acetate. The extract solution was washed with water and a saturated aqueous sodium chloride solution, dried over sodium sulfate and then concentrated under reduced pressure. To the resulting residue was added 4N hydrochloric acid/ethyl acetate (1.0 ml), and stirred for 10 minutes and the resulting mixture was concentrated under reduced pressure to obtain the desired compound (0.10 g) as a crude product.
¹H-NMR (CDCl₃) δ 1.13(d, J=6.8Hz, 6H), 2.52(qq, J=6.8, 6.8Hz, 1H), 3.07-3.10(m, 2H), 3.46-3.49(m, 2H).

### Example 134

### 2-Methyl-N-[2-(([2-oxo-1-(2-piperidin-1-ylethyl)-2,6,7,8-tetrahydro-1H-cyclopenta[g]quinolin-3-yl]methyl}amino)ethyl]propanamide

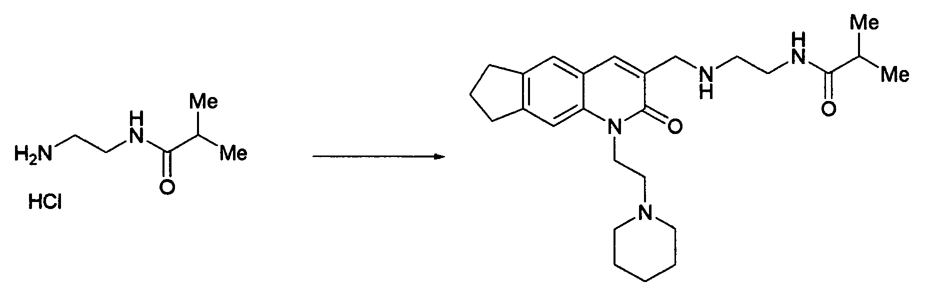

From the compound obtained in Reference Example 22, the desired compound was obtained by the same process as in Example 62.
¹H-NMR (CDCl₃) δ 1.16(d, J=6.8Hz, 6H), 1.48(br.s, 2H), 1.63-1.66(m, 4H), 2.14(tt, J=7.3, 7.3Hz, 2H), 2.41(qq, J=6.6, 6.6Hz, 1H), 2.59(m, 4H), 2.63(t, J=7.8Hz, 2H), 2.78(t, J=5.6Hz, 2H), 2.97(t, J=7.3Hz, 2H), 3.04(t, J=7.3Hz, 2H), 3.38-3.43(m, 2H), 3.77(s, 2H), 4.67(t, J=7.8Hz, 2H), 6.57(br.s, 1H), 7.33(s, 1H), 7.37(s, 1H), 7.61(s, 1H).
The following compounds of Examples 135 to 143 were prepared by the same processes as in Reference Examples 5, 6, 7, 10 and 22 and Example 134.

**[Table 30]**

| No. | Structural formula | ¹H-NMR (CDCl₃) δ |
|---|---|---|
| 135 | 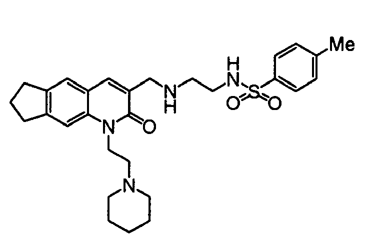 | 1.47(m, 2H), 1.62-1.65(m, 4H), 2.14(tt, J=7.3, 7.3Hz, 2H), 2.40(s, 3H), 2.58(m, 4H), 2.62(t, J=7.9Hz, 2H), 2.71(t, J=6.3Hz, 2H), 2.96(t, J=7.3Hz, 2H), 3.02-3.04(m, 2H), 3.04(t, J=7.3Hz, 2H), 3.62(s, 2H), 4.45(t, J=7.9Hz, 2H), 7.27(d, J=8.2Hz, 2H), 7.32(s, 1H), 7.36(s, 1H), 7.53(s, 1H), 7.76(d, J=8.2Hz, 2H) |
| 136 | 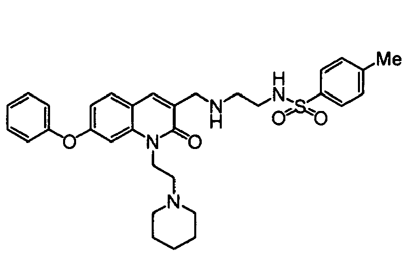 | 1.41-1.43(m, 2H), 1.52-1.58(m, 4H), 2.41(s, 3H), 2.43(m, 4H), 2.54(t, J=7.9Hz, 2H), 2.71-2.73(m, 2H), 3.01-3.04(m, 2H), 3.62(s, 2H), 4.29(t, J=7.9Hz, 2H), 6.90(dd, J=8.5, 2.2Hz, 1H), 6.96(d, J=2.2Hz, 1H), 7.08-7.11(m, 2H), 7.18-7.23(m, 1H), 7.27-7.29(m, 2H), 7.39-7.43(m, 2H), 7.50(d, J=8.5Hz, 1H), 7.54(s, 1H), 7.75-7.77(m, 2H) |
| 137 | 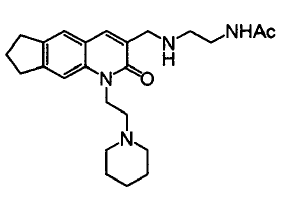 | 1.47-1.53(m, 2H), 1.65-1.72(m, 4H), 2.00(s, 3H), 2.14(tt, J=7.3, 7.3Hz, 2H), 2.65(m, 4H), 2.70(t, J=7.7Hz, 2H), 2.88(t, J=5.7Hz, 2H), 2.96(t, J=7.3Hz, 2H), 3.05(t, J=7.3Hz, 2H), 3.41-3.47(m, 2H), 3.70(s, 2H), 4.51(t, J=7.7Hz, 2H), 7.39(s, 2H), 7.70(s, 1H) |

**[Table 31]**

| No. | Structural formula | ¹H-NMR (CDCl₃) δ |
|---|---|---|
| 138 | 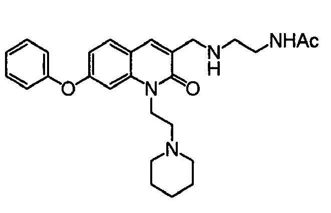 | 1.45-1.47(m, 2H), 1.61-1.62(m, 4H), 2.00(s, 3H), 2.55(m, 4H), 2.67(t, J=7.3Hz, 2H), 3.02(t, J=5.5Hz, 2H), 3.49-3.54(m, 2H), 3.97(s, 2H), 4.37(t, J=7.3Hz, 2H), 6.88(dd, J=8.6, 2.0Hz, 1H), 6.99(d, J=2.0Hz, 1H), 7.07-7.11(m, 2H), 7.16-7.26(m, 1H), 7.39-7.44(m, 2H), 7.55(d, J=8.6Hz, 1H), 7.86(s, 1H) |
| 139 | 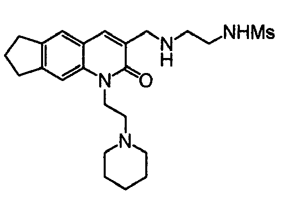 | 1.43-1.50(m, 2H), 1.60-1.67(m, 4H), 2.14(tt, J=7.3, 7.3Hz, 2H), 2.57(m, 4H), 2.62(t, J=7.8Hz, 2H), 2.82-2.86(m, 2H), 2.97(t, J=7.3Hz, 2H), 2.98(s, 3H), 3.04(t, J=7.3Hz, 2H), 3.22-3.26(m, 2H), 3.74(s, 2H), 4.45(t, J=7.8Hz, 2H), 7.32(s, 1H), 7.37(s, 1H), 7.59(s, 1H) |
| 140 | 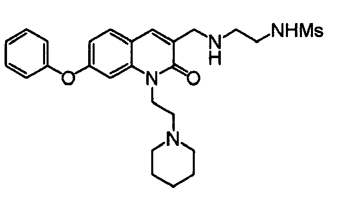 | 1.41-1.45(m, 2H), 1.52-1.57(m, 4H), 2.43 (m, 4H), 2.54 (t, J=7.9Hz, 2H), 2.83-2.86(m, 2H), 2.98(s, 3H), 3.22-3.26(m, 2H), 3.73(s, 2H), 4.30(t, J=7.9Hz, 2H), 6.89(dd, J=8.6, 2.3Hz, 1H), 6.96(d, J=2.3Hz, 1H), 7.08-7.11(m, 2H), 7.18-7.23(m, 1H), 7.38-7.44(m, 2H), 7.51(d, J=8.6Hz, 1H), 7.61(s, 1H) |
| 141 | 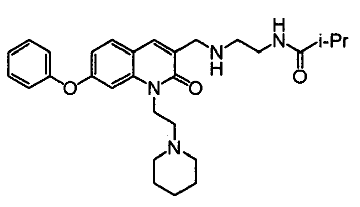 | 1.16(d, J=6.8Hz, 6H), 1.42-1.45(m, 2H), 1.53-1.58(m, 4H), 2.35-2.44(m, 5H), 2.55(t, J=7.8Hz, 2H), 2.78(t, J=5.9Hz, 2H), 3.38-3.42(m, 2H), 3.76(s, 2H), 4.30(t, J=7.8Hz, 2H), 6.90(dd, J=8.6, 2.2Hz, 1H), 6.96(d, J=2.2Hz, 1H), 7.08-7.43(m, 5H), 7.50(d, J=8.6Hz, 1H), 7.62(s, 1H) |

**[Table 32]**

| No. | Structural formula | ¹H-NMR (CDCl₃) δ |
|---|---|---|
| 142 | 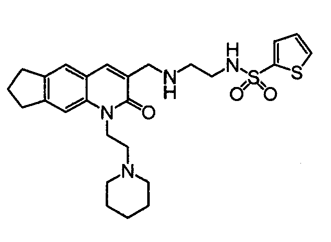 | 1.47-1.52(m, 2H), 1.61-1.67(m, 4H), 2.14(tt, J=7.3, 7.3Hz, 2H), 2.60(m, 4H), 2.64(t, J=7.9Hz, 2H), 2.76(t, J=5.1Hz, 2H), 2.96(t, J=7.3Hz, 2H), 3.04(t, J=7.3Hz, 2H), 3.14(t, J=5.1Hz, 2H), 3.66(s, 2H), 4.45(t, J=7.9Hz, 2H), 7.05(dd, J=5.1, 3.7Hz, 1H), 7.33(s, 1H), 7.36(s, 1H), 7.53(dd, J=5.1, 1.2Hz, 1H), 7.55(s, 1H), 7.61(dd, J=3.7, 1.2Hz, 1H) |
| 143 | 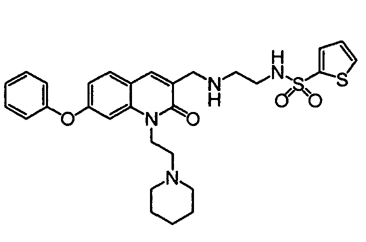 | 1.41-1.42(m, 2H), 1.52-1.57(m, 4H), 2.43(m, 4H), 2.54(t, J=7.8Hz, 2H), 2.77(t, J=5.5Hz, 2H), 3.14(t, J=5.5Hz, 2H), 3.66(s, 2H), 4.29(t, J=7.8Hz, 2H), 6.89(dd, J=8.6, 1.9Hz, 1H), 6.95(d, J=1.9Hz, 1H), 7.04(dd, J=4.9, 3.7Hz, 1H), 7.08-7.26(m, 3H), 7.38-7.42(m, 2H), 7.49(d, J=8.6Hz, 1H), 7.53(dd, J=4.9, 1.2Hz, 1H), 7.57(s, 1H), 7.61(dd, J=3.7, 1.2Hz, 1H) |

### Reference Example 23

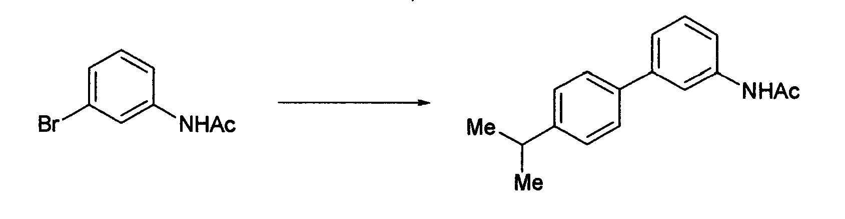

An aqueous sodium carbonate solution (2M, 8.0 ml), ethanol (5.4 ml), 4-isopropylphenylboronic acid (1.6 g, 9.6 mmol) and tetrakis(triphenylphosphine)palladium (0.19 g, 0.16 mmol) were added to a solution of 3-bromoacetanilide (1.7 g, 8.0 mmol) obtained from 3-bromoaniline by the same process as in Reference Example 5 in toluene (20 ml), and the resulting mixture was heated to 110°C. After 3 hours of stirring, a saturated aqueous sodium chloride solution was added thereto, followed by extraction with chloroform. The extract solution was washed with a saturated aqueous sodium chloride solution, dried over sodium sulfate and then concentrated under reduced pressure. The resulting residue was purified by the use of a silica gel column (chloroform : methanol = 50 : 1). Then, the purified residue was recrystallized from chloroform/hexane to obtain the desired compound (1.8 g, 90%).
¹H-NMR (CDCl₃) δ 1.28(d, J=7.0Hz, 6H), 1.58-1.59(m, 1H), 2.20(s, 3H), 2.95(tt, J=7.0, 7.0Hz, 1H), 7.26-7.40(m, 4H), 7.49-7.53(m, 3H), 7.69(br.s, 1H).
The following compounds of Examples 144 to 151 were prepared by the same processes as in Reference Examples 23, 6, 7 and 10 and Example 62.

**[Table 33]**

| No. | Structural formula | ¹H-NMR (CDCl₃) δ |
|---|---|---|
| 144 | 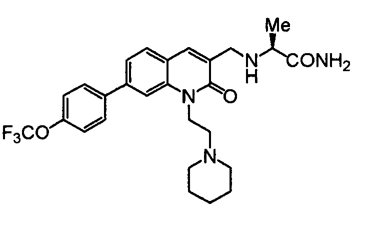 | 1.35(d, J=7.1Hz, 3H), 1.48(m, 2H), 1.66(m, 4H), 2.64(m, 4H), 2.71-2.75(m, 2H), 3.27(q, J=7.1Hz, 1H), 3.66(d, J=13.2Hz, 1H), 3.87(d, J=13.2Hz, 1H), 4.55-4.61(m, 2H), 7.34(d, J=8.0Hz, 2H), 7.44(dd, J=8.3, 1.5Hz, 1H), 7.62(d, J=8.3Hz, 1H), 7.66-7.71(m, 4H) |
| 145 | 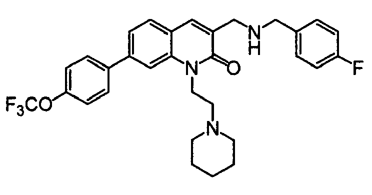 | 1.46-1.48(m, 2H), 1.59-1.63(m, 4H), 2.59(m, 4H), 2.66-2.70(m, 2H), 3.81(s, 2H), 3.83(s, 2H), 4.52-4.55(m, 2H), 6.99-7.04(m, 2H), 7.32-7.36(m, 4H), 7.41-7.44(m, 1H), 7.61-7.71(m, 5H) |
| 146 | 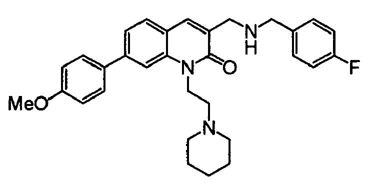 | 1.49(m, 2H), 1.69(m, 4H), 2.68-2.76(m, 6H), 3.81(s, 2H), 3.84(s, 2H), 3.86(s, 3H), 4.60(m, 2H), 6.99-7.04(m, 4H), 7.33-7.37(m, 2H), 7.40-7.42(m, 1H), 7.56-7.62(m, 3H), 7.67-7.69(m, 2H) |
| 147 | 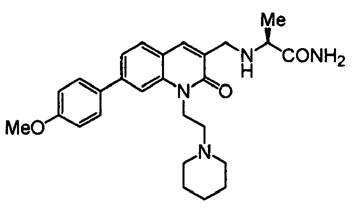 | 1.35(d, J=7.0Hz, 3H), 1.42-1.52(m, 2H), 1.58-1.67(m, 4H), 2.55-2.72 (m, 6H), 3.27 (quart, J=7.0Hz, 1H), 3.65(d, J=13.2Hz, 1H), 3.87(d, J=13.2Hz, 1H), 3.89(s, 3H), 4.57(m, 2H), 5.44(br, 1H), 7.01-7.06(m, 2H), 7.44(dd, J=8.0, 1.1Hz, 1H), 7.57-7.65(m, 5H), 7.80(br, 1H) |

**[Table 34]**

| No. | Structural formula | ¹H-NMR (CDCl₃) δ |
|---|---|---|
| 148 | 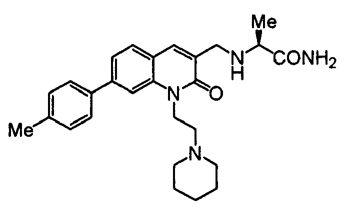 | 1.35(d, J=7.0Hz, 3H), 1.42-1.51(m, 2H), 1.58-1.64(m, 4H), 2.43(s, 3H), 2.52-2.72(m, 6H), 3.27(quart, J=7.0Hz, 1H), 3.65(d, J=13.2Hz, 1H), 3.87(d, J=13.2Hz, 1H), 4.54(m, 2H), 5.44(br, 1H), 7.28-7.33(m, 2H), 7.46(dd, J=8.0, 1.2Hz, 1H), 7.53-7.67(m, 5H), 7.78(br, 1H) |
| 149 | 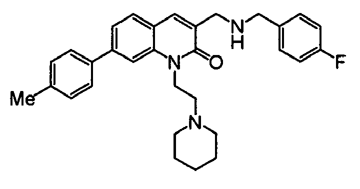 | 1.44-1.49(m, 2H), 1.59-1.65(m, 4H), 2.43(s, 3H), 2.59(m, 4H), 2.66-2.70(m, 2H), 3.81(s, 2H), 3.82(s, 2H), 4.51-4.55(m, 2H), 6.98-7.04(m, 2H), 7.26-7.35(m, 4H), 7.55(dd, J=8.0, 1.5Hz, 1H), 7.55-7.60 (m, 3H), 7.64(s, 1H), 7.69(s, 1H) |
| 150 | 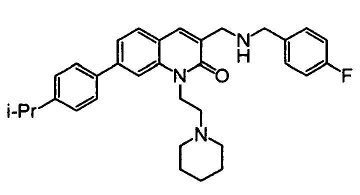 | 1.31(d, J=7.1Hz, 6H), 1.46-1.47(m, 2H), 1.60-1.65(m, 4H), 2.59(m, 4H), 2.66-2.70(m, 2H), 2.99(qq, J=7.1, 7.1Hz, 1H), 3.80(s, 2H), 3.82(s, 2H), 4.51-4.55(m, 2H), 6.98-7.04(m, 2H), 7.32-7.37(m, 4H), 7.45(dd, J=8.0, 1.5Hz, 1H), 7.59-7.61(m, 3H), 7.65(s, 1H), 7.69(s,1H) |
| 151 | 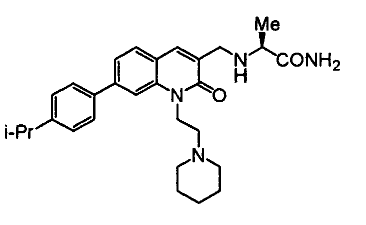 | 1.31(d, J=6.8Hz, 6H), 1.35(d, J=7.1Hz, 3H), 1.47-1.50(m, 2H), 1.62-1.66(m, 4H), 2.59(m, 4H), 2.67-2.71(m, 2H), 2.99(qq, J=6.8, 6.8Hz, 1H), 3.27(q, J=7.1Hz, 1H), 3.65(d, J=13.2Hz, 1H), 3.88(d, J=13.2Hz, 1H), 4.47-4.61(m, 2H), 5.43(br.s, 1H), 7.36-7.38(m, 2H), 7.47(dd, J=8.0, 1.5Hz, 1H), 7.59-7.61(m, 3H), 7.65-7.67(m, 2H), 7.78(br.s, 1H) |

### Reference Example 24

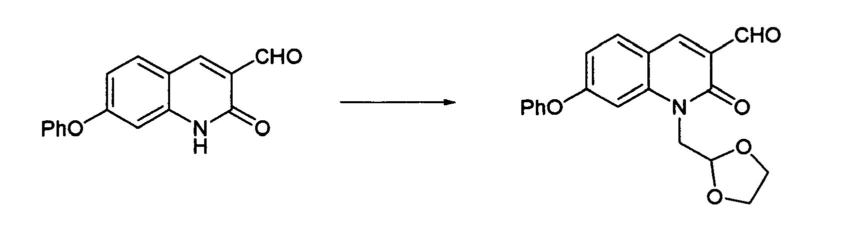

A 2-quinolone derivative (0.20 g, 0.75 mmol) obtained from 2-phenoxyaniline by the same processes as in Reference Examples 5, 6 and 7 was dissolved in N,N'-dimethylformamide (10 ml), and cesium carbonate (0.49 g, 1.51 mmol) and 2-bromomethyl-1,3-dioxolane (0.16 ml, 1.51 mmol) were added thereto at room temperature. After the reaction solution was stirred at 80°C for 1.5 hours, 2-bromomethyl-1,3-dioxolane (0.20 ml, 1.89 mmol) and cesium carbonate (0.49 g, 1.51 mmol) were further added thereto. After 2 hours, water and ethyl acetate were added thereto to effect separation and extraction. The organic layer was washed with water, dried and then concentrated, and the residue was purified by the use of a silica gel column (ethyl acetate : hexane = 1 : 3 to 1 : 1) to obtain the desired compound (0.12 g, 44%) and its O-alkyl derivative (0.09 g, 34%).
¹H-NMR (CDCl₃) δ 3.80-3.93(m, 4H), 4.40(d, J=4.4Hz, 2H), 5.22(t, J=4.4Hz, 1H), 6.91(dd, J=8.8, 2.2Hz, 1H), 7.11-7.16(m, 3H), 7.26(m, 1H), 7.42-7.48(m, 2H), 7.65(d, J=8.8Hz, 1H), 8.34(s, 1H), 10.42(s, 1H).

### Reference Example 25

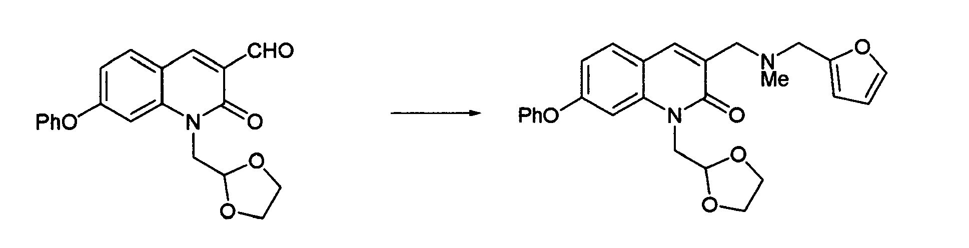

From the compound obtained in Reference Example 24, the desired compound was obtained by the same process as in Example 3.
¹H-NMR (CDCl₃) δ 2.34(s, 3H), 3.60(s, 2H), 3.67(s, 2H), 3.78-3.94(m, 4H), 4.39(d, J=4.6Hz, 2H), 5.22(t, J= 4.6Hz, 1H), 6.24(d, J=3.1Hz, 1H), 6.32(dd, J=3.1, 2.0Hz, 1H), 6.88(dd, J=8.6, 2.2Hz, 1H), 7.07-7.21(m, 4H), 7.35-7.43(m, 3H), 7.52(d, J=8.6Hz, 1H), 7.85(s, 1H).

### Reference Example 26

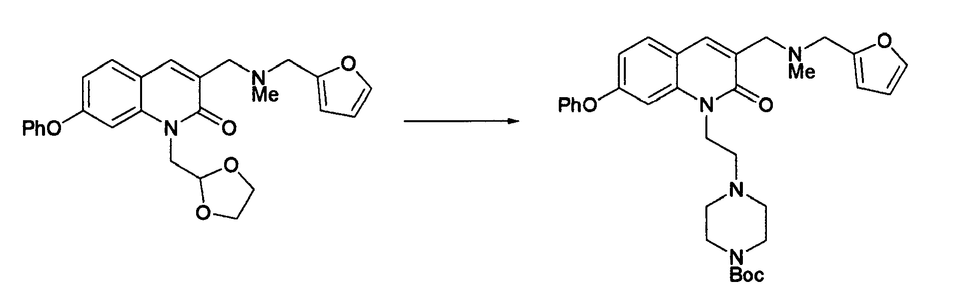

The compound (0.08 g, 0.23 mmol) obtained in Reference Example 25 was dissolved in dioxane (2 ml), followed by adding thereto water (2 ml) and sulfuric acid (0.5 ml), and the resulting mixture was heated at 80°C for 6 hours. The reaction solution was poured into a 1N aqueous sodium hydroxide solution and ethyl acetate was added thereto to effect separation and extraction. The organic layer was washed with water, dried and then concentrated to obtain a crude product (0.06 g). The crude product was dissolved in dichloromethane (2 ml), followed by adding thereto 1-Boc-piperazine (56 mg, 0.30 mmol), sodium triacetoxyborohydride (64 mg, 0.30 mmol) and acetic acid (3 drops), and the resulting mixture was stirred at room temperature for 2.5 hours. Water, ethyl acetate and a saturated aqueous sodium hydrogencarbonate solution were added to the reaction solution to effect separation and extraction. The organic layer was washed with water, dried and then concentrated, and the residue was purified by the use of a silica gel column (only ethyl acetate) to obtain the desired compound (63 mg, 74%).
¹H-NMR (CDCl₃) δ 1.47(s, 9H), 2.34(s, 3H), 2.37-2.44(m, 4H), 2.59(m, 2H), 3.35-3.42(m, 4H), 3.58(s, 2H), 3.67(s, 2H), 4.30(m, 2H), 6.24(d, J=3.1Hz, 1H), 6.32(dd, J=3.1, 1.8Hz, 1H), 6.88-6.92(m, 2H), 7.07-7.12(m, 2H), 7.21(m, 1H), 7.37-7.43(m, 3H), 7.54(d, J=9.0Hz, 1H), 7.83(s, 1H).

### Example 152

### 1-[2-(4-Acetylpiperazin-1-yl)ethyl]-3-{[(2-furylmethyl)(methyl)amino]methyl}-7-phenoxyquinolin-2(1H)-one

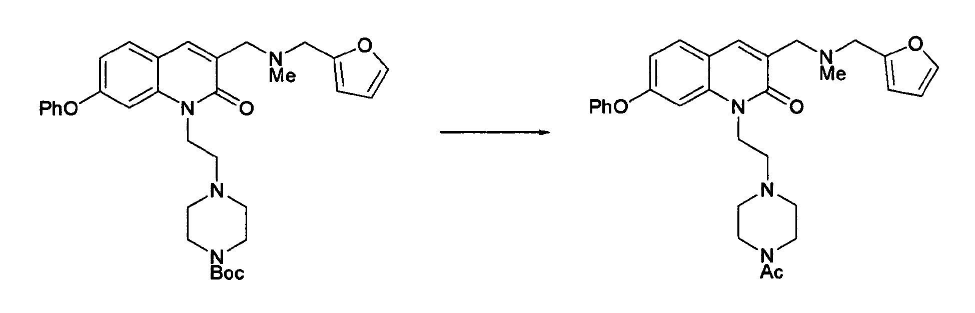

The compound (63 mg, 0.11 mmol) obtained in Reference Example 26 was dissolved in a 4N-hydrochloric acid-ethyl acetate solution (3 ml) and the resulting solution was stirred at room temperature for 2.5 hours. The reaction solution was concentrated and then dried under reduced pressure. To 2 ml of a solution of the thus obtained crude product in dichloromethane (6 ml) were added triethylamine (25 µl, 0.18 mmol) and acetic anhydride (9 µl, 0.11 mmol), and the resulting mixture was stirred at room temperature. After 30 minutes, water, ethyl acetate and a saturated aqueous sodium hydrogencarbonate solution were added thereto to effect separation and extraction. The organic layer was washed with water, dried and then concentrated to obtain the desired compound (20 mg, a quantitative yield).
¹H-NMR (CDCl₃) δ 2.08(s, 3H), 2.35(s, 3H), 2.42(t, J=5.1Hz, 2H), 2.51(t, J=5.1Hz, 2H), 2.62(m, 2H), 3.41(t, J=5.1Hz, 2H), 3.56(t, J=5.1Hz, 2H), 3.59(s, 2H), 3.67(s, 2H), 4.31(m, 2H), 6.25(d, J=2.0Hz, 1H), 6.33(dd, J=3.1, 2.0Hz, 1H), 6.87-6.93(m, 2H), 7.07-7.12(m, 2H), 7.21(m, 1H), 7.37-7.43(m, 3H), 7.55(d, J=8.5Hz, 1H), 7.84(s, 1H)
The following compounds of Examples 153 and 154 were prepared by the same processes as in Reference Examples 5, 6, 7, 24, 25 and 26 and Example 152.

**[Table 35]**

| No. | Structural formula | ¹H-NMR (CDCl₃) δ |
|---|---|---|
| 153 | 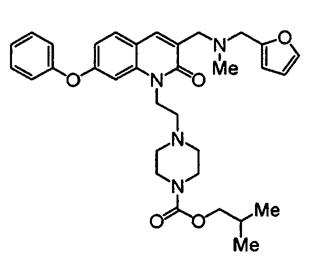 | 0.93(d, J=6.6Hz, 6H), 1.94(m, 1H), 2.34(s, 3H), 2.40-2.47(m, 4H), 2.60(m, 2H), 3.41-3.46(m, 4H), 3.59(s, 2H), 3.67(s, 2H), 3.87(d, J=6.8Hz, 2H), 4.30(m, 2H), 6.25(d, J=3.2Hz, 1H), 6.32(dd, J=3.2, 2.0Hz, 1H), 6.87-6.93(m, 2H), 7.07-7.12(m, 2H), 7.21(m, 1H), 7.38-7.44(m, 3H), 7.54(d, J=9.0Hz, 1H), 7.83(s, 1H) |
| 154 | 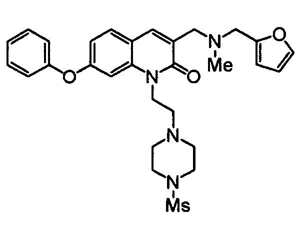 | 2.34(s, 3H), 2.56-2.69(m, 6H), 2.75(s, 3H), 3.16-3.23(m, 4H), 3.59(s, 2H), 3.67(s, 2H), 4.30(m, 2H), 6.26(d, J=3.2Hz, 1H), 6.32(dd, J=3.2, 1.8Hz, 1H), 6.87-6.93(m, 2H), 7.07-7.12(m, 2H), 7.21(m, 1H), 7.38-7.45(m, 3H), 7.55(d, J=9.1Hz, 1H), 7.84(s, 1H) |

### Test Example 1

Experiment on the inhibition of the TTX-resistant Na channel in human-SNS-gene expression cells
1) Construction of cells having human SNS expressed therein and confirmation of SNS function exhibition
   The whole of human SNS α subunit gene was inserted into an expression plasmid (pcDNA3.1Zeo(+)) having Zeocin resistance gene and the whole of Annexin II light chain gene was introduced into an expression plasmid (pcDNA3.1(+)) containing Neomycin resistance gene. These two genes were introduced into CHO-K1 cells at the same time by the use of lipofectamine 2000, and the cells were cultured on F-12 medium containing Neomycin and Zeocin, to select cells resistant to both agents, i.e., cells having both genes inserted therein. These strains resistant to both agents were subjected twice to limiting dilution, followed by cloning of cells having SNS gene inserted therein. The genetic introduction of SNS was confirmed by RT-PCR. In addition, the exhibition of the function of SNS was confirmed by detecting a TTX-resistant component responsive to a stimulus to the Na channel by the use of a fluorescent indicator sensitive to membrane potential.
2) Pharmacological effect on the TTX-resistant Na channel in the human-SNS-gene expression cells
   Using the cells having human SNS expressed therein which had been obtained in the above item 1, the SNS inhibitory effect of compounds of the present invention was evaluated. That is, each test compound was previously added to the cells having human SNS expressed therein. After about 30 minutes, veratridine (50 µM), a Na channel stimulator was added in the presence of TTX (1 µM) to increase the membrane potential through the TTX-resistant Na channel, whereby the inhibitory effect of the test compound on the membrane potential increase was evaluated.
3) Pharmacological evaluation method
   The SNS inhibition rate of the test compound was calculated by the following calculation equation: SNS inhibition rate (%) = 100 x [(peak value obtained by only veratridine stimulation without compound to be evaluated) - (peak value obtained by veratridine stimulation in the presence of compound to be evaluated)] / [(peak value obtained by only veratridine stimulation without compound to be evaluated) - (reference value obtained without stimulation)]
4) Test results
   For the compounds obtained in the working examples, the inhibitory effect on the TTX-resistant Na channel in the cells having human SNS expressed therein (SNS inhibition rate) was evaluated to find that the compounds of the present invention have SNS inhibitory effect as follows.

**[Table 36]**

| Compound | Compound concentration (µM) | SNS inhibition rate (%) |
|---|---|---|
| Example 1 | 13 | 75 |
| Example 2 | 13 | 100 |
| Example 3 | 13 | 86 |
| Example 4 | 14 | 97 |
| Example 28 | 12.5 | 68 |
| Example 31 | 12.5 | 80 |
| Example 33 | 12.5 | 89 |
| Example 37 | 12.5 | 63 |
| Example 38 | 12.5 | 71 |
| Example 41 | 12.5 | 94 |
| Example 43 | 12.5 | 99 |
| Example 47 | 12.5 | 60 |
| Example 50 | 12.5 | 43 |
| Example 52 | 12.5 | 34 |
| Example 53 | 12.5 | 100 |
| Example 56 | 12.5 | 68 |
| Example 57 | 12.5 | 87 |
| Example 58 | 12.5 | 57 |
| Example 59 | 12.5 | 100 |
| Example 61 | 12.5 | 40 |
| Example 62 | 12.5 | 79 |
| Example 68 | 12.5 | 92 |
| Example 69 | 12.5 | 98 |
| Example 70 | 12.5 | 97 |
| Example 71 | 12.5 | 95 |
| Example 77 | 12.5 | 84 |
| Example 83 | 12.5 | 83 |
| Example 86 | 12.5 | 87 |

**[Table 37]**

| Compound | Compound concentration (µM) | SNS inhibition rate (%) |
|---|---|---|
| Example 103 | 12.5 | 100 |
| Example 107 | 12.5 | 97 |
| Example 115 | 12.5 | 61 |
| Example 116 | 12.5 | 100 |
| Example 117 | 12.5 | 100 |
| Example 119 | 12.5 | 100 |
| Example 128 | 12.5 | 96 |
| Example 136 | 12.5 | 100 |
| Example 142 | 12.5 | 90 |
| Example 144 | 12.5 | 87 |
| Example 153 | 12.5 | 100 |

### INDUSTRIAL APPLICABILITY

The novel 2-quinolone derivatives of the present invention are usable as excellent therapeutic or prophylactic agents for pathoses attributable to SNS, specifically, a disease such as neuropathic pain, nociceptive pain, urinary disturbance or multiple sclerosis.

## Claims

1. A compound represented by the formula (1): 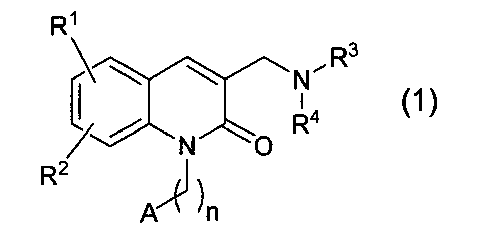 wherein R¹ and R² are independently a hydrogen atom, a halogen atom, a cyano group, a nitro group, a carboxyl group, an alkyl group of 1 to 4 carbon atoms, a haloalkyl group of 1 to 4 carbon atoms, an alkoxy group of 1 to 4 carbon atoms, a haloalkoxy group of 1 to 4 carbon atoms, an alkylthio group of 1 to 4 carbon atoms, an alkoxycarbonyl group of 2 to 4 carbon atoms, an alkylcarbonyl group of 2 to 4 carbon atoms, or a group represented by the formula: -L-Ar wherein L is a single bond, -O-, -OCH₂- or -CH₂-, and Ar is a substituted or unsubstituted phenyl group or a substituted or unsubstituted pyridyl group, or R¹ and R², when taken together, may form a 5- to 7-membered ring,
n is 1 to 3,
A is a substituted or unsubstituted aryl group, a substituted or unsubstituted heteroaromatic group, or a group represented by the formula: -N(R⁵)R⁶ wherein R⁵ and R⁶ are independently a hydrogen atom, a substituted or unsubstituted alkyl group or a substituted or unsubstituted cycloalkyl group, or R⁵ and R⁶, when taken together with the nitrogen atom to which they are bonded, may form a substituted or unsubstituted 5- to 8-membered saturated or unsaturated nitrogen-containing heterocyclic ring, said nitrogen-containing heterocyclic ring containing 0 or 1 oxygen atom, 0 or 1 sulfur atom and 1 or 2 nitrogen atoms, and
R³ and R⁴ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted and saturated or unsaturated aliphatic heterocyclic group, a substituted or unsubstituted aryl group or a substituted or unsubstituted heteroaromatic group, or R³ and R⁴, when taken together with the nitrogen atom to which they are bonded, may form a substituted or unsubstituted 5- to 10-membered saturated or unsaturated nitrogen-containing heterocyclic ring, said nitrogen-containing heterocyclic ring containing 0 to 2 oxygen atoms, 0 to 2 sulfur atoms and 1 to 3 nitrogen atoms, provided that when R³ and R⁴ are taken together with the nitrogen atom to which they are bonded, to form a piperidine ring, R¹ and R² are not hydrogen atoms at the same time, or a pharmaceutically acceptable salt thereof.

2. A compound or a pharmaceutically acceptable salt thereof according to claim 1, wherein R³ and R⁴ are independently a hydrogen atom or a substituted or unsubstituted alkyl group, or R³ and R⁴, when taken together with the nitrogen atom to which they are bonded, may form a substituted or unsubstituted 5- to 10-membered saturated or unsaturated nitrogen-containing heterocyclic ring, said nitrogen-containing heterocyclic ring containing 0 to 2 oxygen atoms, 0 to 2 sulfur atoms and 1 to 3 nitrogen atoms.

3. A compound or a pharmaceutically acceptable salt thereof according to claim 1 or 2, wherein A is a group represented by the formula: -N(R⁵)R⁶ wherein R⁵ and R⁶ are independently a hydrogen atom, a substituted or unsubstituted alkyl group or a substituted or unsubstituted cycloalkyl group, or R⁵ and R⁶, when taken together with the nitrogen atom to which they are bonded, may form a substituted or unsubstituted 5- to 8-membered saturated or unsaturated nitrogen-containing heterocyclic ring, said nitrogen-containing heterocyclic ring containing 0 or 1 oxygen atom, 0 or 1 sulfur atom and 1 or 2 nitrogen atoms.

4. A compound or a pharmaceutically acceptable salt thereof according to claim 1 or 2, wherein A is a substituted or unsubstituted aryl group.

5. A compound or a pharmaceutically acceptable salt thereof according to claim 1 or 2, wherein A is a substituted or unsubstituted heteroaromatic group.

6. A compound or a pharmaceutically acceptable salt thereof according to claim 5, wherein the heteroaromatic group is a substituted or unsubstituted pyridyl group.

7. A compound according to any one of claims 1 to 6, which is represented by the formula (2): 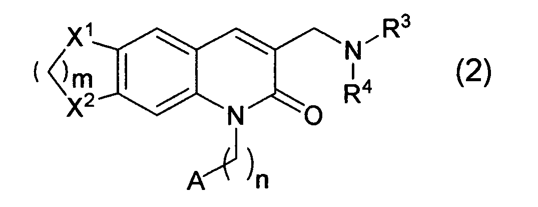 wherein A, n, R³ and R⁴ are as defined in claim 1; m is 1 to 3; and X¹ and X² are independently methylene or an oxygen atom, or a pharmaceutically acceptable salt thereof.

8. A compound according to any one of claims 1 to 6, which is represented by the formula (3): 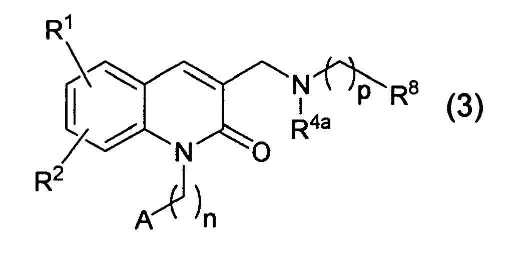 wherein A, n, R¹ and R² are as defined in claim 1; p is 1 to 4; R^{4a} is a hydrogen atom or an alkyl group; and R⁸ is a substituted or unsubstituted aryl group or a substituted or unsubstituted heteroaromatic group, or a pharmaceutically acceptable salt thereof.

9. A compound according to any one of claims 1 to 6, which is represented by the formula (3a): 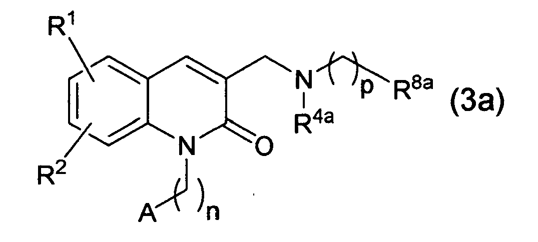 wherein A, n, R¹ and R² are as defined in claim 1; p and R^{4a} are as defined in claim 8; and R^{8a} is a substituted or unsubstituted amino group or a substituted or unsubstituted carbamoyl group, or a pharmaceutically acceptable salt thereof.

10. A compound or a pharmaceutically acceptable salt thereof according to claim 8 or 9, wherein A is a group represented by the formula: -N(R⁵)R⁶ wherein R⁵ and R⁶ are independently a hydrogen atom, a substituted or unsubstituted alkyl group or a substituted or unsubstituted cycloalkyl group, or R⁵ and R⁶, when taken together with the nitrogen atom to which they are bonded, may form a substituted or unsubstituted 5- to 8-membered saturated or unsaturated nitrogen-containing heterocyclic ring, said nitrogen-containing heterocyclic ring containing 0 or 1 oxygen atom, 0 or 1 sulfur atom and 1 or 2 nitrogen atoms.

11. An SNS inhibitor comprising a compound or a pharmaceutically acceptable salt thereof according to any one of claims 1 to 10 as an active ingredient.

12. A pharmaceutical composition for treatment or prophylaxis of neuropathic pain, nociceptive pain, urinary disturbance or multiple sclerosis comprising a compound or a pharmaceutically acceptable salt thereof according to any one of claims 1 to 10 as an active ingredient.
